(19) Europäisches Patentamt
European Patent Office
Office européen des brevets

(11) **EP 4 101 469 A1**

(12) **EUROPEAN PATENT APPLICATION**
published in accordance with Art. 153(4) EPC


(43) Date of publication:
**14.12.2022 Bulletin 2022/50**

(21) Application number: **21751220.1**

(22) Date of filing: **02.02.2021**

(51) International Patent Classification (IPC):
***A61K 47/10*** *(2017.01)* ***A61K 9/08*** *(2006.01)*
***A61K 47/04*** *(2006.01)* ***A61K 47/12*** *(2006.01)*
***A61K 47/18*** *(2017.01)* ***A61K 47/38*** *(2006.01)*
***A61K 47/40*** *(2006.01)* ***A61K 47/46*** *(2006.01)*

(52) Cooperative Patent Classification (CPC):
**A61K 9/08; A61K 47/02; A61K 47/10; A61K 47/12; A61K 47/18; A61K 47/38; A61K 47/40; A61K 47/46**

(86) International application number:
**PCT/JP2021/003724**

(87) International publication number:
**WO 2021/157569 (12.08.2021 Gazette 2021/32)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **03.02.2020 JP 2020016656**



(71) Applicant: **Senju Pharmaceutical Co., Ltd.**
**Osaka-shi,**
**Osaka 541-0048 (JP)**

(72) Inventor: **NISHIDA, Noriaki**
**Osaka-shi, Osaka 541-0048 (JP)**

(74) Representative: **Engelhard, Markus**
**Boehmert & Boehmert**
**Anwaltspartnerschaft mbB**
**Pettenkoferstrasse 22**
**80336 München (DE)**


(54) **USE OF POLYETHER COMPOUND**


(57) The present disclosure provides the use of a polyether compound. In one embodiment, the present disclosure relates to improvement of the stability of a polyether compound that uses a polyether compound and improvement of the solubility of a sparingly soluble compound. In one embodiment, the present disclosure relates to a composition that includes a chelating compound, the composition being for stabilizing a polyether compound. In one embodiment, the present disclosure relates to a composition for improving the solubility of a sparingly soluble compound, the composition including at least one of a hydrophobic polyoxyaliphatic moiety-containing polyether compound and a hydrating compound, by combining a hydrophobic polyoxyaliphatic moiety-containing polyether compound and a hydrating compound. In one embodiment, regarding the present disclosure, regarding the polyether compound, the polyether compound includes a poloxamer and polyethylene glycol.


Mebendazole **Fig 1**

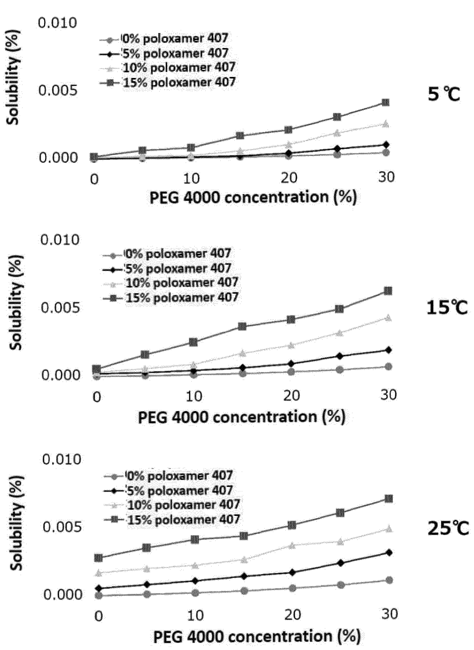





Processed by Luminess, 75001 PARIS (FR)

## Description

[Technical Field]

The present disclosure relates to use of a polyether compound. More specifically, the present disclosure relates to improvement in the stability of a polyether compound and improvement in the solubility of a poorly soluble compound.

[Background Art]

Polyether compounds such as poloxamer and polyethylene glycol are often used in formulation of a pharmaceutical product, etc. (Patent Literature 1). Polyether compounds can be oxidatively degraded by an oxygen radical or light (UV etc.) in an environment such as an aqueous solution. Degradation of a polyether compound may produce an acidic compound such as acetic acid or formic acid, which can result in a decreased pH or viscosity. For this reason, improvement in the stability of polyether compounds is desired.

Drugs include many compounds with low solubility to water. For example, solubility to water is a critical factor in the preparation of an eye drop, etc. It can be difficult to develop an aqueous formulation of a drug with low solubility. If a drug dissolves into oil, an emulsion may be selected as the dosage form, but a drug is often prepared as a suspension. In comparison to aqueous formulations, suspensions are more difficult to formulate and use in industrial settings, and development thereof is more time-intensive. Since intraocular migration of a drug in a suspension is often lower compared to an aqueous formulation, a suspension requires a higher drug concentration. Thus, an aqueous formulation is desired from the viewpoint of safety. For this reason, improvement in the solubility of poorly soluble compounds is desired for the preparation of an aqueous formulation.

[Citation List]

[Non Patent Literature]

[NPL 1] Japanese National Phase PCT Laid-open Publication No. 9-510712

[Summary of Invention]

[Solution to Problem]

The inventors have found that polyether compounds are stabilized by combining a polyether compound with a chelate compound. The inventors have also found that the solubility of a poorly soluble compound can be improved by using a polyether compound. The present disclosure provides a formulation for use in stabilizing a polyether compound, a formulation of a polyether compound for use in improving the solubility of poorly soluble compounds, as well as the applications thereof.

Therefore, the present invention provides the following.

(Item 1)

A composition for use in stabilizing a polyether compound, comprising a chelate compound.

(Item 2)

A composition comprising a polyether compound and a chelate compound.

(Item 3)

The composition of any of the preceding items, wherein the polyether compound comprises poloxamer and polyethylene glycol.

(Item 4)

The composition of any of the preceding items, wherein the chelate compound is selected from the group consisting of thiosulfate, ethylenediaminetetraacetic acid (EDTA) or a salt or ion thereof, and citric acid or a salt or ion thereof.

(Item 5)

The composition of any of the preceding items, wherein the chelate compound comprises thiosulfate.

(Item 6)

The composition of any of the preceding items, wherein the composition further comprises at least one of mannitol and dibutylhydroxytoluene (BHT).

(Item 7)

The composition of any of the preceding items, wherein the poloxamer has an average molecular weight of about 1000 to 15000.

(Item 8)

The composition of any of the preceding items, wherein the poloxamer comprises 10 to 90 wt% of polyoxyethylene per molecule.

(Item 9)

The composition of any of the preceding items, wherein the poloxamer has polyoxypropylene at an average molecular weight of about 900 to 4000 per molecule.

(Item 10)

The composition of any of the preceding items, wherein the polyethylene glycol has an average molecular weight of about 200 to 50000.

(Item 11)

The composition of any of the preceding items, wherein a change in pH from before storage to after storage is 0.5 or less when prepared at a pH of about 7.5 and stored for 4 weeks at 60°C at 1 atm.

(Item 12)

The composition of any of the preceding items, wherein a decrease in viscosity measured with a rotational viscometer under conditions of 25°C and 100 rpm from before storage to after storage is 20% or less when prepared at a pH of about 7.5 and stored for 4 weeks at 60°C at 1 atm.

(Item 13)

A composition for use in improving solubility of a poorly soluble compound by a combination of a hydrophobic polyoxy aliphatic moiety-containing polyether compound and a hydratable compound, comprising at least one of a hydrophobic polyoxy aliphatic moiety-containing polyether compound and a hydratable compound.

(Item 14)

A composition comprising a poorly soluble compound, a hydrophobic polyoxy aliphatic moiety-containing polyether compound, and a hydratable compound.

(Item 15)

The composition of any of the preceding items, wherein the hydrophobic polyoxy aliphatic moiety-containing polyether compound is a polyoxypropylene-containing polyether compound.

(Item 16)

The composition of any of the preceding items, wherein the hydrophobic polyoxy aliphatic moiety-containing polyether compound comprises poloxamer.

(Item 17)

The composition of any of the preceding items, wherein the poloxamer has an average molecular weight of about 1000 to 15000.

(Item 18)

The composition of any of the preceding items, wherein the poloxamer comprises 10 to 90 wt% of polyoxyethylene per molecule.

(Item 19)

The composition of any of the preceding items, wherein the poloxamer has polyoxypropylene at an average molecular weight of about 900 to 4000 per molecule.

(Item 20)

The composition of any of the preceding items, wherein the hydratable compound comprises polyethylene glycol.

(Item 21)

The composition of any of the preceding items, wherein the polyethylene glycol has an average molecular weight of about 200 to 50000.

(Item 22)

The composition of any of the preceding items, wherein the poorly soluble compound has LogP of 0.5 to 8.

(Item 23)

The composition of any of the preceding items, wherein the poorly soluble compound has LogP of 0.5 to 8 at a pH of the composition.

(Item 24)

The composition of any of the preceding items, wherein improvement in solubility of the poorly soluble compound by adding the hydratable compound to the hydrophobic polyoxy aliphatic moiety-containing polyether compound under conditions of an atmospheric pressure of 1 atm at 25°C is 1.1-fold or greater.

(Item 25)

The composition of any of the preceding items, wherein improvement in solubility of the poorly soluble compound by adding the hydratable compound to the hydrophobic polyoxy aliphatic moiety-containing polyether compound under conditions of an atmospheric pressure of 1 atm at 15°C is 1.5-fold or greater.

(Item 26)

The composition of any of the preceding items, wherein improvement in solubility of the poorly soluble compound by adding the hydratable compound to the hydrophobic polyoxy aliphatic moiety-containing polyether compound under conditions of an atmospheric pressure of 1 atm at 5°C is 2-fold or greater.

(Item 27)

The composition of any of the preceding items, comprising cyclodextrin or a water-soluble macromolecule.

(Item 28)

The composition of any of the preceding items, comprising at least one of sulfobutylether-β-cyclodextrin and carboxymethyl cellulose.

(Item 29)

The composition of any of the preceding items, which is a composition of any one of items 1 to 10.

(Item 30)

The composition of any of the preceding items, comprising a buffer.

(Item 30A)

The composition of any of the preceding items, which is an orally administered agent, a topical agent, or an injection agent.

(Item 30B)

The composition of any of the preceding items, which is for intravenous administration, intramuscular administration, subcutaneous administration, or intravitreal administration.

(Item 30C)

The composition of any of the preceding items, which is for administration selected from the group consisting of dermal administration, intranasal administration, eye ball administration, mucosal administration, rectal administration, and inhalation administration.

(Item 31)

The composition of any of the preceding items for application to an eye.

(Item 32)

The composition of any of the preceding items, further comprising a pharmaceutically acceptable excipient.

(Item 33)

The composition of any of the preceding items, which is a pharmaceutical composition.

(Item 34)

An eye drop comprising the composition of any of the preceding items.

(Item 35)

A pharmaceutical composition comprising the composition of any of the preceding items and an active ingredient.

(Item 36)

An ophthalmic pharmaceutical composition comprising the composition of any of the preceding items and an active ingredient.

(Item 37)

A composition for use in improving solubility of a medicament comprising a hydratable compound and a poorly soluble compound, comprising a hydrophobic polyoxy aliphatic moiety-containing polyether compound.

(Item 38)

A composition for use in improving solubility of a medicament comprising and a hydrophobic polyoxy aliphatic moiety-containing polyether compound and a poorly soluble compound, comprising a hydratable compound.

(Item 39)

A chelate compound for use in stabilizing a polyether compound.

(Item 40)

The chelate compound of any of the preceding items, wherein the polyether compound comprises poloxamer and polyethylene glycol.

(Item 41)

The chelate compound of any of the preceding items, selected from the group consisting of thiosulfate, ethylenediaminetetraacetic acid (EDTA) or a salt or ion thereof, and citric acid or a salt or ion thereof.

(Item 42)

The chelate compound of any of the preceding items, comprising thiosulfate.

(Item 43)

The chelate compound of any of the preceding items for use in combination with at least one of mannitol and dibutylhydroxytoluene (BHT).

(Item 44)

The chelate compound of any of the preceding items, wherein the poloxamer has an average molecular weight of about 1000 to 15000.

(Item 45)

The chelate compound of any of the preceding items, wherein the poloxamer comprises 10 to 90 wt% of polyoxyethylene per molecule.

(Item 46)

The chelate compound of any of the preceding items, wherein the poloxamer has polyoxypropylene at an average molecular weight of about 900 to 4000 per molecule.

(Item 47)

The chelate composition of any of the preceding items, wherein the polyethylene glycol has an average molecular weight of about 200 to 50000.

(Item 48)

The chelate compound of any of the preceding items for use in preparing a composition, which has a change in pH from before storage to after storage of 0.5 or less when prepared at a pH of about 7.5 and stored for 4 weeks at 60°C at 1 atm.

(Item 49)

The chelate compound of any of the preceding item for use in preparing a composition, which has a decrease in viscosity measured with a rotational viscometer under conditions of 25°C and 100 rpm from before storage to after storage of 20% or less when prepared at a pH of about 7.5 and stored for 4 weeks at 60°C at 1 atm.

(Item 50)

A hydrophobic polyoxy aliphatic moiety-containing polyether compound or hydratable compound for use in improving solubility of a poorly soluble compound by a combination of a hydrophobic polyoxy aliphatic moiety-containing polyether compound and a hydratable compound.

(Item 51)

The hydrophobic polyoxy aliphatic moiety-containing polyether compound or hydratable compound of any of the preceding items, wherein the hydrophobic polyoxy aliphatic moiety-containing polyether compound is a polyoxypropylene-containing polyether compound.

(Item 52)

The hydrophobic polyoxy aliphatic moiety-containing polyether compound or hydratable compound of any of the preceding items, wherein the hydrophobic polyoxy aliphatic moiety-containing polyether compound comprises poloxamer.

(Item 53)

The hydrophobic polyoxy aliphatic moiety-containing polyether compound or hydratable compound of any of the preceding items, wherein the poloxamer has an average molecular weight of about 1000 to 15000.

(Item 54)

The hydrophobic polyoxy aliphatic moiety-containing polyether compound or hydratable compound of any of the preceding items, wherein the poloxamer comprises 10 to 90 wt% of polyoxyethylene per molecule.

(Item 55)

The hydrophobic polyoxy aliphatic moiety-containing polyether compound or hydratable compound of any of the preceding items, wherein the poloxamer has polyoxypropylene at an average molecular weight of about 900 to 4000 per molecule.

(Item 56)

The hydrophobic polyoxy aliphatic moiety-containing polyether compound or hydratable compound of any of the preceding items, wherein the hydratable compound comprises polyethylene glycol.

(Item 57)

The hydrophobic polyoxy aliphatic moiety-containing polyether compound or hydratable compound of any of the preceding items, wherein the polyethylene glycol has an average molecular weight of about 200 to 50000.

(Item 58)

The hydrophobic polyoxy aliphatic moiety-containing polyether compound or hydratable compound of any of the preceding items, wherein the poorly soluble compound has LogP of 0.5 to 8.

(Item 59)

The hydrophobic polyoxy aliphatic moiety-containing polyether compound or hydratable compound of any of

the preceding items for use in preparing a composition in which the poorly soluble compound has LogP of 0.5 to 8 at a pH of the composition.

(Item 60)

The hydrophobic polyoxy aliphatic moiety-containing polyether compound or hydratable compound of any of the preceding items for use in preparing a composition in which improvement in solubility of the poorly soluble compound by adding the hydratable compound to the hydrophobic polyoxy aliphatic moiety-containing polyether compound under conditions of an atmospheric pressure of 1 atm at 25°C is 1.1-fold or greater.

(Item 61)

The hydrophobic polyoxy aliphatic moiety-containing polyether compound or hydratable compound of any of the preceding items for use in preparing a composition in which improvement in solubility of the poorly soluble compound by adding the hydratable compound to the hydrophobic polyoxy aliphatic moiety-containing polyether compound under conditions of an atmospheric pressure of 1 atm at 15°C is 1.5-fold or greater.

(Item 62)

The hydrophobic polyoxy aliphatic moiety-containing polyether compound or hydratable compound of any of the preceding items for use in preparing a composition in which improvement in solubility of the poorly soluble compound by adding the hydratable compound to the hydrophobic polyoxy aliphatic moiety-containing polyether compound under conditions of an atmospheric pressure of 1 atm at 5°C is 2-fold or greater.

(Item 63)

The hydrophobic polyoxy aliphatic moiety-containing polyether compound or hydratable compound of any of the preceding items, comprising cyclodextrin or a water-soluble macromolecule.

(Item 64)

The hydrophobic polyoxy aliphatic moiety-containing polyether compound or hydratable compound of any of the preceding items for use in combination with at least one of sulfobutylether-β-cyclodextrin and carboxymethyl cellulose.

(Item 65)

The chelate compound, hydrophobic polyoxy aliphatic moiety-containing polyether compound, or hydratable compound of any of the preceding items for use in combination with a buffer.

(Item 65A)

The chelate compound, hydrophobic polyoxy aliphatic moiety-containing polyether compound, or hydratable compound of any of the preceding items for use in an orally administered agent, a topical agent, or an injection agent.

(Item 65B)

The chelate compound, hydrophobic polyoxy aliphatic moiety-containing polyether compound, or hydratable compound of any of the preceding items, which is for use in intravenous administration, intramuscular administration, subcutaneous administration, or intravitreal administration.

(Item 65C)

The chelate compound, hydrophobic polyoxy aliphatic moiety-containing polyether compound, or hydratable compound of any of the preceding items, which is for use in administration selected from the group consisting of dermal administration, intranasal administration, eye ball administration, mucosal administration, rectal administration, and inhalation administration.

(Item 66)

The chelate compound, hydrophobic polyoxy aliphatic moiety-containing polyether compound, or hydratable compound of any of the preceding items for use in application to an eye.

(Item 67)

The chelate compound, hydrophobic polyoxy aliphatic moiety-containing polyether compound, or hydratable compound of any of the preceding items for use in an eye drop.

(Item 68)

A method for treating a subject in need thereof, comprising administering a composition comprising a therapeutically effective amount of an active ingredient, a chelate compound, and a polyether compound to the subject.

(Item 69)

The method of any of the preceding items, wherein the polyether compound comprises poloxamer and polyethylene glycol.

(Item 70)

The method of any of the preceding items, wherein the chelate compound is selected from the group consisting of thiosulfate, ethylenediaminetetraacetic acid (EDTA) or a salt or ion thereof, and citric acid or a salt or ion thereof.

(Item 71)

The method of any of the preceding items, wherein the chelate compound comprises thiosulfate.

(Item 72)

The method of any of the preceding items, wherein the composition comprises at least one of mannitol and dibutylhydroxytoluene (BHT).

(Item 73)

The method of any of the preceding items, wherein the poloxamer has an average molecular weight of about 1000 to 15000.

(Item 74)

The method of any of the preceding items, wherein the poloxamer comprises 10 to 90 wt% of polyoxyethylene per molecule.

(Item 75)

The method of any of the preceding items, wherein the poloxamer has polyoxypropylene at an average molecular weight of about 900 to 4000 per molecule.

(Item 76)

The method of any of the preceding items, wherein the polyethylene glycol has an average molecular weight of about 200 to 50000.

(Item 77)

The method of any of the preceding items, wherein the composition has a change in pH from before storage to after storage of 0.5 or less when prepared at a pH of about 7.5 and stored for 4 weeks at 60°C at 1 atm.

(Item 78)

The method of any of the preceding items, wherein the composition has a decrease in viscosity measured with a rotational viscometer under conditions of 25°C and 100 rpm from before storage to after storage of 20% or less when prepared at a pH of about 7.5 and stored for 4 weeks at 60°C at 1 atm.

(Item 79)

A method for treating a subject in need thereof, comprising administering an effective amount of a composition comprising a poorly soluble compound, a hydrophobic polyoxy aliphatic moiety-containing polyether compound, and a hydratable compound to the subject.

(Item 80)

The method of any of the preceding items, wherein the hydrophobic polyoxy aliphatic moiety-containing polyether compound is a polyoxypropylene-containing polyether compound.

(Item 81)

The method of any of the preceding items, wherein the hydrophobic polyoxy aliphatic moiety-containing polyether compound comprises poloxamer.

(Item 82)

The method of any of the preceding items, wherein the poloxamer has an average molecular weight of about 1000 to 15000.

(Item 83)

The method of any of the preceding items, wherein the poloxamer comprises 10 to 90 wt% of polyoxyethylene per molecule.

(Item 84)

The method of any of the preceding items, wherein the poloxamer has polyoxypropylene at an average molecular weight of about 900 to 4000 per molecule.

(Item 85)

The method of any of the preceding items, wherein the hydratable compound comprises polyethylene glycol.

(Item 86)

The method of any of the preceding items, wherein the polyethylene glycol has an average molecular weight of about 200 to 50000.

(Item 87)

The method of any of the preceding items, wherein the poorly soluble compound has LogP of 0.5 to 8.

(Item 88)

The method of any of the preceding items, wherein the poorly soluble compound has LogP of 0.5 to 8 at a pH of the composition.

(Item 89)

The method of any of the preceding items, wherein the composition has an improvement in solubility of the

poorly soluble compound by adding the hydratable compound to the hydrophobic polyoxy aliphatic moiety-containing polyether compound under conditions of an atmospheric pressure of 1 atm at 25°C of 1.1-fold or greater.

(Item 90)

The method of any of the preceding items, wherein the composition has an improvement in solubility of the poorly soluble compound by adding the hydratable compound to the hydrophobic polyoxy aliphatic moiety-containing polyether compound under conditions of an atmospheric pressure of 1 atm at 15°C of 1.5-fold or greater.

(Item 91)

The method of any of the preceding items, wherein the composition has an improvement in solubility of the poorly soluble compound by adding the hydratable compound to the hydrophobic polyoxy aliphatic moiety-containing polyether compound under conditions of an atmospheric pressure of 1 atm at 5°C of 2-fold or greater.

(Item 92)

The method of any of the preceding items, wherein the composition comprises cyclodextrin or a water-soluble macromolecule.

(Item 93)

The method of any of the preceding items, wherein the composition comprises at least one of sulfobutylether-β-cyclodextrin and carboxymethyl cellulose.

(Item 94)

The method of any of the preceding items, wherein the composition comprises a buffer.

(Item 94A)

The method of any of the preceding items, wherein the administering comprises intravenous administration, intramuscular administration, subcutaneous administration, or intravitreal administration to the subject.

(Item 94B)

The method of any of the preceding items, wherein the administering comprises dermal administration, intranasal administration, eye ball administration, mucosal administration, rectal administration, and inhalation administration to the subject.

(Item 95)

The method of any of the preceding items, wherein the administering comprises administering to an eye of the subject.

(Item 96)

Use of a chelate compound that stabilizes a polyether compound in the manufacture of a medicament for administering treatment using an active ingredient to a subject in need thereof.

(Item 97)

The use of any of the preceding items, wherein the polyether compound comprises poloxamer and polyethylene glycol.

(Item 98)

The use of any of the preceding items, wherein the chelate compound is selected from the group consisting of

thiosulfate, ethylenediaminetetraacetic acid (EDTA) or a salt or ion thereof, and citric acid or a salt or ion thereof.

(Item 99)

The use of any of the preceding items, wherein the chelate compound comprises thiosulfate.

(Item 100)

The use of any of the preceding items, wherein at least one of mannitol and dibutylhydroxytoluene (BHT) is further used.

(Item 101)

The use of any of the preceding items, wherein the poloxamer has an average molecular weight of about 1000 to 15000.

(Item 102)

The use of any of the preceding items, wherein the poloxamer comprises 10 to 90 wt% of polyoxyethylene per molecule.

(Item 103)

The use of any of the preceding items, wherein the poloxamer has polyoxypropylene at an average molecular weight of about 900 to 4000 per molecule.

(Item 104)

The use of any of the preceding items, wherein the polyethylene glycol has an average molecular weight of about 200 to 50000.

(Item 105)

The use of any of the preceding items for preparing a medicament, which has a change in pH from before storage to after storage of 0.5 or less when prepared at a pH of about 7.5 and stored for 4 weeks at 60°C at 1 atm.

(Item 106)

The use of any of the preceding items for preparing a medicament, which has a decrease in viscosity measured with a rotational viscometer under conditions of 25°C and 100 rpm from before storage to after storage of 20% or less when prepared at a pH of about 7.5 and stored for 4 weeks at 60°C at 1 atm.

(Item 106)

Use of at least one of a hydrophobic polyoxy aliphatic moiety-containing polyether compound and a hydratable compound that improves the solubility of a poorly soluble compound by a combination of a hydrophobic polyoxy aliphatic moiety-containing polyether compound and a hydratable compound in the manufacture of a medicament for administering treatment using a poorly soluble compound to a subject in need thereof.

(Item 107)

The use of any of the preceding items, wherein the hydrophobic polyoxy aliphatic moiety-containing polyether compound is a polyoxypropylene-containing polyether compound.

(Item 108)

The use of any of the preceding items, wherein the hydrophobic polyoxy aliphatic moiety-containing polyether compound comprises poloxamer.

(Item 109)

The use of any of the preceding items, wherein the poloxamer has an average molecular weight of about 1000 to 15000.

(Item 110)

The use of any of the preceding items, wherein the poloxamer comprises 10 to 90 wt% of polyoxyethylene per molecule.

(Item 111)

The use of any of the preceding items, wherein the poloxamer has polyoxypropylene at an average molecular weight of about 900 to 4000 per molecule.

(Item 112)

The use of any of the preceding items, wherein the hydratable compound comprises polyethylene glycol.

(Item 113)

The use of any of the preceding items, wherein the polyethylene glycol has an average molecular weight of about 200 to 50000.

(Item 114)

The use of any of the preceding items, wherein the poorly soluble compound has LogP of 0.5 to 8.

(Item 115)

The use of any of the preceding items for the preparation of a composition in which the poorly soluble compound has LogP of 0.5 to 8 at a pH of the composition.

(Item 116)

The use of any of the preceding items for the preparation of a composition in which improvement in solubility of the poorly soluble compound by adding the hydratable compound to the hydrophobic polyoxy aliphatic moiety-containing polyether compound under conditions of an atmospheric pressure of 1 atm at 25°C is 1.1-fold or greater.

(Item 117)

The use of any of the preceding items for the preparation of a medicament in which improvement in solubility of the poorly soluble compound by adding the hydratable compound to the hydrophobic polyoxy aliphatic moiety-containing polyether compound under conditions of an atmospheric pressure of 1 atm at 15°C is 1.5-fold or greater.

(Item 118)

The use of any of the preceding items for the preparation of a medicament in which improvement in solubility of the poorly soluble compound by adding the hydratable compound to the hydrophobic polyoxy aliphatic moiety-containing polyether compound under conditions of an atmospheric pressure of 1 atm at 5°C is 2-fold or greater.

(Item 119)

The use of any of the preceding items, wherein cyclodextrin or a water-soluble macromolecule is further used.

(Item 120)

The use of any of the preceding items, wherein at least one of sulfobutylether-β-cyclodextrin and carboxymethyl

cellulose is further used.

(Item 121)

The use of any of the preceding items, wherein a buffer is further used.

(Item 121A)

The use of any of the preceding items, wherein the medicament is an orally administered agent, a topical agent, or an injection agent.

(Item 121B)

The use of any of the preceding items, wherein the medicament is a medicament for intravenous administration, intramuscular administration, subcutaneous administration, or intravitreal administration.

(Item 121C)

The use of any of the preceding items, wherein the medicament is a medicament for dermal administration, intranasal administration, eye ball administration, mucosal administration, rectal administration, or inhalation administration.

(Item 122)

The use of any of the preceding items, wherein the medicament is a medicament for application to an eye.

(Item 123)

The use of any of the preceding items, wherein the medicament is an eye drop.
The present disclosure is intended so that one or more of the features described above can be provided not only as the explicitly disclosed combinations, but also as other combinations thereof. Additional embodiments and advantages of the present disclosure are recognized by those skilled in the art by reading and understanding the following detailed description as needed.

[Advantageous Effects of Invention]

The present disclosure provides various formulations that can facilitate and improve handling of an agent such as a drug.

[Brief Description of Drawings]

[Figure 1] Figure **1** is a diagram that shows the solubility of mebendazole at 5°C, 15°C, or 25°C under each additive condition. The top panel shows results at 5°C, the middle panel shows results at 15°C, and the bottom panel shows results at 25°C. The vertical axis indicates the mebendazole solubility (%). The horizontal axis indicates the concentration of the polyethylene glycol used (%). The concentration of poloxamer used (%) (in the graph) and corresponding results are indicated by the same marker.
[Figure 2] Figure **2** is a diagram that shows the solubility of dexamethasone at 5°C, 15°C, or 25°C under each additive condition. The top panel shows results at 5°C, the middle panel shows results at 15°C, and the bottom panel shows results at 25°C. The vertical axis indicates the dexamethasone solubility (%). The horizontal axis indicates the concentration of the polyethylene glycol used (%). The concentration of poloxamer used (%) (in the graph) and corresponding results are indicated by the same marker.
[Figure 3] Figure **3** is a diagram that shows the solubility of triamcinolone acetonide at 5°C, 15°C, or 25°C under each additive condition. The top panel shows results at 5°C, the middle panel shows results at 15°C, and the bottom panel shows results at 25°C. The vertical axis indicates the triamcinolone acetonide solubility (%). The horizontal axis indicates the concentration of the polyethylene glycol used (%). The concentration of poloxamer used (%) (in the graph) and corresponding results are indicated by the same marker.

[Figure 4] Figure **4** is a diagram that shows the solubility of fluocinolone acetonide at 5°C, 15°C, or 25°C under each additive condition. The top panel shows results at 5°C, the middle panel shows results at 15°C, and the bottom panel shows results at 25°C. The vertical axis indicates the fluocinolone acetonide solubility (%). The horizontal axis indicates the concentration of the polyethylene glycol used (%). The concentration of poloxamer used (%) (in the graph) and corresponding results are indicated by the same marker.

[Figure 5] Figure **5** is a diagram that shows the solubility of desonide at 5°C, 15°C, or 25°C under each additive condition. The top panel shows results at 5°C, the middle panel shows results at 15°C, and the bottom panel shows results at 25°C. The vertical axis indicates the desonide solubility (%). The horizontal axis indicates the concentration of the polyethylene glycol used (%). The concentration of poloxamer used (%) (in the graph) and corresponding results are indicated by the same marker.

[Figure 6] Figure **6** is a diagram that shows the solubility of flubendazole at 5°C, 15°C, or 25°C under each additive condition. The top panel shows results at 5°C, the middle panel shows results at 15°C, and the bottom panel shows results at 25°C. The vertical axis indicates the flubendazole solubility (%). The horizontal axis indicates the concentration of the polyethylene glycol used (%). The concentration of poloxamer used (%) (in the graph) and corresponding results are indicated by the same marker.

[Figure 7] Figure **7** is a diagram that shows the solubility of cilostazol at 5°C, 15°C, or 25°C under each additive condition. The top panel shows results at 5°C, the middle panel shows results at 15°C, and the bottom panel shows results at 25°C. The vertical axis indicates the cilostazol solubility (%). The horizontal axis indicates the concentration of the polyethylene glycol used (%). The concentration of poloxamer used (%) (in the graph) and corresponding results are indicated by the same marker.

[Figure 8] Figure **8** is a diagram that shows the solubility of itraconazole at 5°C, 15°C, or 25°C under each additive condition. The top panel shows results at 5°C, the middle panel shows results at 15°C, and the bottom panel shows results at 25°C. The vertical axis indicates the itraconazole solubility (%). The horizontal axis indicates the concentration of the polyethylene glycol used (%). The concentration of poloxamer used (%) (in the graph) and corresponding results are indicated by the same marker.

[Figure 9] Figure **9** is a diagram that shows the solubility of sorafenib at 5°C, 15°C, or 25°C under each additive condition. The top panel shows results at 5°C, the middle panel shows results at 15°C, and the bottom panel shows results at 25°C. The vertical axis indicates the sorafenib solubility (%). The horizontal axis indicates the concentration of the polyethylene glycol used (%). The concentration of poloxamer used (%) (in the graph) and corresponding results are indicated by the same marker.

[Figure 10] Figure **10** is a diagram that shows the solubility of regorafenib at 5°C, 15°C, or 25°C under each additive condition. The top panel shows results at 5°C, the middle panel shows results at 15°C, and the bottom panel shows results at 25°C. The vertical axis indicates the regorafenib solubility (%). The horizontal axis indicates the concentration of the polyethylene glycol used (%). The concentration of poloxamer used (%) (in the graph) and corresponding results are indicated by the same marker.

[Figure 11] Figure **11** is a diagram that shows the solubility of telmisartan at 5°C, 15°C, or 25°C under each additive condition. The top panel shows results at 5°C, the middle panel shows results at 15°C, and the bottom panel shows results at 25°C. The vertical axis indicates the telmisartan solubility (%). The horizontal axis indicates the concentration of the polyethylene glycol used (%). The concentration of poloxamer used (%) (in the graph) and corresponding results are indicated by the same marker.

[Figure 12] Figure **12** is a diagram that shows the solubility of cabozantinib at 5°C, 15°C, or 25°C under each additive condition. The top panel shows results at 5°C, the middle panel shows results at 15°C, and the bottom panel shows results at 25°C. The vertical axis indicates the cabozantinib solubility (%). The horizontal axis indicates the concentration of the polyethylene glycol used (%). The concentration of poloxamer used (%) (in the graph) and corresponding results are indicated by the same marker.

[Figure 13] Figure **13** is a diagram that shows the solubility of nilotinib at 5°C, 15°C, or 25°C under each additive condition. The top panel shows results at 5°C, the middle panel shows results at 15°C, and the bottom panel shows results at 25°C. The vertical axis indicates the nilotinib solubility (%). The horizontal axis indicates the concentration of the polyethylene glycol used (%). The concentration of poloxamer used (%) (in the graph) and corresponding results are indicated by the same marker.

[Figure 14] Figure **14** is a diagram that shows the solubility of aprepitant at 5°C, 15°C, or 25°C under each additive condition. The top panel shows results at 5°C, the middle panel shows results at 15°C, and the bottom panel shows results at 25°C. The vertical axis indicates the aprepitant solubility (%). The horizontal axis indicates the concentration of the polyethylene glycol used (%). The concentration of poloxamer used (%) (in the graph) and corresponding results are indicated by the same marker.

[Figure 15] Figure **15** is a diagram that shows the solubility of rotenone at 5°C, 15°C, or 25°C under each additive condition. The top panel shows results at 5°C, the middle panel shows results at 15°C, and the bottom panel shows results at 25°C. The vertical axis indicates the rotenone solubility (%). The horizontal axis indicates the concentration

of the polyethylene glycol used (%). The concentration of poloxamer used (%) (in the graph) and corresponding results are indicated by the same marker.

[Figure 16] Figure **16** is a diagram that shows the solubility of griseofulvin at 5°C, 15°C, or 25°C under each additive condition. The top panel shows results at 5°C, the middle panel shows results at 15°C, and the bottom panel shows results at 25°C. The vertical axis indicates the griseofulvin solubility (%). The horizontal axis indicates the concentration of the polyethylene glycol used (%). The concentration of poloxamer used (%) (in the graph) and corresponding results are indicated by the same marker.

[Figure 17] Figure **17** is a diagram that shows the solubility of osthole at 5°C, 15°C, or 25°C under each additive condition. The top panel shows results at 5°C, the middle panel shows results at 15°C, and the bottom panel shows results at 25°C. The vertical axis indicates the osthole solubility (%). The horizontal axis indicates the concentration of the polyethylene glycol used (%). The concentration of poloxamer used (%) (in the graph) and corresponding results are indicated by the same marker.

[Figure 18] Figure **18** is a diagram that shows the solubility of 4-bromodibenzofuran at 5°C, 15°C, or 25°C under each additive condition. The top panel shows results at 5°C, the middle panel shows results at 15°C, and the bottom panel shows results at 25°C. The vertical axis indicates the 4-bromodibenzofuran solubility (%). The horizontal axis indicates the concentration of the polyethylene glycol used (%). The concentration of poloxamer used (%) (in the graph) and corresponding results are indicated by the same marker.

[Figure 19] Figure **19** is a diagram that shows the solubility of simvastatin at 5°C, 15°C, or 25°C under each additive condition. The top panel shows results at 5°C, the middle panel shows results at 15°C, and the bottom panel shows results at 25°C. The vertical axis indicates the simvastatin solubility (%). The horizontal axis indicates the concentration of the polyethylene glycol used (%). The concentration of poloxamer used (%) (in the graph) and corresponding results are indicated by the same marker.

[Figure 20] Figure **20** is a diagram that shows the solubility of efavirenz at 5°C, 15°C, or 25°C under each additive condition. The top panel shows results at 5°C, the middle panel shows results at 15°C, and the bottom panel shows results at 25°C. The vertical axis indicates the efavirenz solubility (%). The horizontal axis indicates the concentration of the polyethylene glycol used (%). The concentration of poloxamer used (%) (in the graph) and corresponding results are indicated by the same marker.

[Figure 21] Figure **21** is a diagram that shows the solubility of rebamipide at 5°C, 15°C, or 25°C under each additive condition. The top panel shows results at 5°C, the middle panel shows results at 15°C, and the bottom panel shows results at 25°C. The vertical axis indicates the rebamipide solubility (%). The horizontal axis indicates the concentration of the polyethylene glycol used (%). The concentration of poloxamer used (%) (in the graph) and corresponding results are indicated by the same marker.

[Figure 22] Figure **22** is a diagram that shows the solubility of celecoxib at 5°C, 15°C, or 25°C under each additive condition. The top panel shows results at 5°C, the middle panel shows results at 15°C, and the bottom panel shows results at 25°C. The vertical axis indicates the celecoxib solubility (%). The horizontal axis indicates the concentration of the polyethylene glycol used (%). The concentration of poloxamer used (%) (in the graph) and corresponding results are indicated by the same marker.

[Figure 23] Figure **23** is a diagram that shows the solubility of celecoxib in the presence of 0.5% CMC at 5°C, 15°C, or 25°C under each additive condition. The top panel shows results at 5°C, the middle panel shows results at 15°C, and the bottom panel shows results at 25°C. The vertical axis indicates the solubility of celecoxib in the presence of 0.5% CMC (%). The horizontal axis indicates the concentration of the polyethylene glycol used (%). The concentration of poloxamer used (%) (in the graph) and corresponding results are indicated by the same marker.

[Figure 24] Figure **24** is a diagram that shows the solubility of celecoxib in the presence of 5% SBE-β-CD at 5°C, 15°C, or 25°C under each additive condition. The top panel shows results at 5°C, the middle panel shows results at 15°C, and the bottom panel shows results at 25°C. The vertical axis indicates the solubility of celecoxib in the presence of 5% SBE-β-CD (%). The horizontal axis indicates the concentration of the polyethylene glycol used (%). The concentration of poloxamer used (%) (in the graph) and corresponding results are indicated by the same marker.

[Figure 25] Figure **25** is a diagram that shows the solubility of celecoxib in the presence of 5% HP-β-CD at 5°C, 15°C, or 25°C under each additive condition. The top panel shows results at 5°C, the middle panel shows results at 15°C, and the bottom panel shows results at 25°C. The vertical axis indicates the solubility of celecoxib in the presence of 5% HP-β-CD (%). The horizontal axis indicates the concentration of the polyethylene glycol used (%). The concentration of poloxamer used (%) (in the graph) and corresponding results are indicated by the same marker.

[Description of Embodiments]

The present disclosure is described hereinafter while showing the best mode thereof. Throughout the entire specification, a singular expression should be understood as encompassing the concept thereof in the plural form, unless specifically noted otherwise. Thus, singular articles (e.g., "a", "an", "the", and the like in the case of English) should also

be understood as encompassing the concept thereof in the plural form, unless specifically noted otherwise. Further, the terms used herein should be understood as being used in the meaning that is commonly used in the art, unless specifically noted otherwise. Therefore, unless defined otherwise, all terminologies and scientific technical terms that are used herein have the same meaning as the general understanding of those skilled in the art to which the present disclosure pertains. In case of a contradiction, the present specification (including the definitions) takes precedence.

The definitions of the terms and/or details of the basic technologies that are especially used herein are explained hereinafter as appropriate.

As used herein, "polyether compound" refers to a compound comprising of a partial structure of repeats having an ether bond such as a polyoxypropylene chain ($-CH_2-CH(CH_3)-O-$) or polyoxyethylene chain ($-CH_2-CH_2-O-$). A polyether compound can encompass poloxamer, polyethylene glycol, polypropylene glycol, polybutylene glycol, etc.

As used herein, "hydrophobic polyoxy aliphatic moiety" refers to a moiety that is a repeat of -O-(aliphatic group with three or more carbon atoms) in a compound, and "hydrophobic polyoxy aliphatic moiety-containing polyether compound" refers to a polyether compound comprising a hydrophobic polyoxy aliphatic moiety.

As used herein, "poloxamer" is one type of polyether compound and refers to a block copolymer comprising a polyoxypropylene chain (POP) and polyoxyethylene chains (POE) flanking the same. Poloxamer can be characterized by the molecular weight (average molecular weight), wt% of polyoxyethylene, molecular weight of polyoxypropylene per molecule, or presence and/or type of a modification. The poloxamers herein include molecules having the structure of formula I:

[Chemical Formula 1]

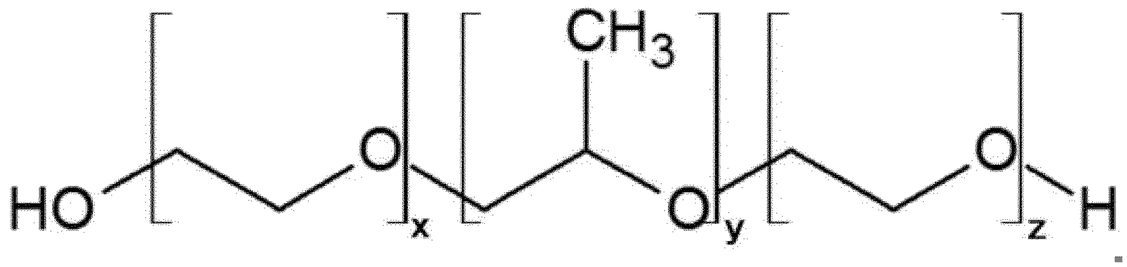

(formula I)

wherein x, y, and z are each independently selected integer that is 0 or greater, and molecules modified therefrom are also included. For example, the poloxamers herein include compounds wherein the total molecular weight of partial structures represented by repeat units of $-CH_2-CH_2-O-$ and $-CH_2-CH(CH_3)-O-$ is 70% or greater, 75% or greater, 80% or greater, 85% or greater, 90% or greater, 95% or greater, 98% or greater, or 99% or greater with respect to the molecular weight of the entire compound. Examples of modifications of poloxamer include, but are not limited to, the following modifications in the structure of formula I:

*a branching modification which replaces one or more hydrogen atoms in a molecule with -(repeat unit of $-CH_2-CH_2-O-$ and/or $-CH_2-CH(CH_3)-O-$)-H;
*a modification which replaces one or more repeat units of $-CH_2-CH_2-O-$ or $-CH_2-CH(CH_3)-O-$ independently with a group selected from the group consisting of $-R^2-O-$, $-R^2-S-$, $-R^2-$, and $-R^2-N(R^1)-$;
*a modification which replaces one or more -OH groups independently with a group selected from the group consisting of $-NR^1_2$, $-OR^1$, $-SR^1$, and $-R^1$;
*a modification which replaces one or more hydrogen atoms in a molecule with a halogen or a methyl group;
*any combination of the modifications described above; etc. wherein

$R^1$ is an optionally substituted $C_{1-10}$ aliphatic group, or a group wherein any of the carbon atoms in the $C_{1-10}$ aliphatic group is replaced with an optionally substituted 5- to 7-membered cyclic group, and
$R^2$ is an optionally substituted $C_{1-10}$ aliphatic group, or a group wherein any of the carbon atoms in the $C_{1-10}$ aliphatic group is replaced with an optionally substituted 5- to 7-membered cyclic group.

Poloxamer having a molecular weight of a polyoxypropylene moiety of about 4000 and a percentage of polyoxyethylene chain of about 70% is generally referred to as poloxamer 407, and poloxamer having a molecular weight of a polyoxypropylene moiety of about 1800 and a percentage of polyoxyethylene chain of about 40% is generally referred to as poloxamer 184, etc. The relationship between the general name of poloxamer and its structure is generally understood by those skilled in the art.

As used herein, "polyethylene glycol" or "PEG" is one type of polyether compound and refers to a copolymer comprising a polyoxyethylene chain (POE). Polyethylene glycol can be characterized by the molecular weight (average molecular weight) or presence and/or type of a modification. The polyethylene glycols herein typically include molecules having the structure of formula II:

$HO\text{-}(CH_2\text{-}CH_2\text{-}O)_n\text{-}H$ (formula II)

wherein n is an integer that is 0 or greater, but molecules modified therefrom are also included. For example, the polyethylene glycol herein can refer to compounds wherein the total molecular weight of partial structures represented by repeat units of $-CH_2-CH_2-O-$ is 70% or greater, 75% or greater, 80% or greater, 85% or greater, 90% or greater, 95% or greater, 98% or greater, or 99% or greater with respect to the molecular weight of the entire molecule. Examples of modifications include, but are not limited to, the following modifications in the structure of formula II:

*a branching modification which replaces one or more hydrogen atoms in a molecule with -(repeat unit of $-CH_2-CH_2-O-$)-H;
*a modification which replaces one or more repeat units of $-CH_2-CH_2-O-$ independently with a group selected from the group consisting of $-R^2-O-$, $-R^2-S-$, $-R^2-$, and $-R^2-N(R^1)-$;
*a modification which replaces one or more -OH groups independently with a group selected from the group consisting of $-NR^1_2$, $-OR^1$, $-SR^1$, and $-R^1$;
*a modification which replaces one or more hydrogen atoms in a molecule with a halogen or a methyl group;
*any combination of the modifications described above; etc. wherein

$R^1$ is an optionally substituted $C_{1-10}$ aliphatic group, or a group wherein any of the carbon atoms in the $C_{1-10}$ aliphatic group is replaced with an optionally substituted 5- to 7-membered cyclic group, and
$R^2$ is an optionally substituted $C_{1-10}$ aliphatic group, or a group wherein any of the carbon atoms in the $C_{1-10}$ aliphatic group is replacedwith an optionally substituted 5- to 7-membered cyclic group.

For example, those skilled in the art understand that PEG 400 has an average molecular weight of 380 to 420, PEG 2000 has an average molecular weight of 1800 to 2200, PEG 4000 has an average molecular weight of 2600 to 3800, PEG 6000 has an average molecular weight of 7300 to 9300, PEG 20000 has an average molecular weight of 15000 to 25000, etc. Those skilled in the art also understand that the number in PEG denoted with a small number, such as PEG-4, represents the number of repeat units of polyoxyethylene in a molecule. Those skilled in the art clearly understand the structure/composition of polyethylene glycol with a specific name (PEG 2000, etc.)

As used herein, "polypropylene glycol" refers to a copolymer comprising a polyoxypropylene chain, and "polybutylene glycol" refers to a copolymer comprising a polyoxybutylene chain (POE), which are both a type of polyether compound. Polypropylene glycol and polybutylene glycol can be characterized by the molecular weight (average molecular weight), or presence and/or type of a modification. The polypropylene glycol and polybutylene glycol herein typically include molecules having the structures of formula III and formula IV, respectively:

$HO\text{-}(CH_2\text{-}CH(CH_3)\text{-}O)_n\text{-}H$ (formula III)

$HO\text{-}(CH_2\text{-}CH_2\text{-}CH(CH_3)\text{-}O)_n\text{-}H$ (formula IV)

wherein n is an integer that is 0 or greater, but molecules modified therefrom are also included. For example, polypropylene glycol and polybutylene glycol herein can respectively refer to compounds wherein the total molecular weight of partial structures represented by repeat units of $-CH_2-CH(CH_3)-O-$ and $-CH_2-CH_2-CH(CH_3)-O-$ is 70% or greater, 75% or greater, 80% or greater, 85% or greater, 90% or greater, 95% or greater, 98% or greater, or 99% or greater with respect to the molecular weight of the entire molecule. Examples of modifications include, but are not limited to, the following modifications in the structure of formula III or formula IV:

*a branching modification which replaces one or more hydrogen atoms in a molecule with -(repeat unit of $-CH_2-CH(CH_3)-O-$ or $-CH_2-CH_2-CH(CH_3)-O-$)-H;
*a modification which replaces one or more repeat units of $-CH_2-CH(CH_3)-O-$ or $-CH_2-CH_2-CH(CH_3)-O-$ independently with a group selected from the group consisting of $-R^2-O-$, $-R^2-S-$, $-R^2-$, and $-R^2-N(R^1)-$;
*a modification which replaces one or more -OH groups independently with a group selected from the group consisting of $-NR^1_2$, $-OR^1$, $-SR^1$, and $-R^1$;
*a modification which replaces one or more hydrogen atoms in a molecule with a halogen or a methyl group;

*any combination of the modifications described above; etc. wherein

$R^1$ is an optionally substituted $C_{1-10}$ aliphatic group, or a group wherein any of the carbon atoms in the $C_{1-10}$ aliphatic group is with an optionally substituted 5- to 7-membered cyclic group, and
$R^2$ is an optionally substituted $C_{1-10}$ aliphatic group, or a group wherein any of the carbon atoms in the $C_{1-10}$ aliphatic group is with an optionally substituted 5- to 7-membered cyclic group.

As used herein, "molecular weight" used for a macromolecular substance (poloxamer, polyethylene glycol, etc.) refers to the average molecular weight unless specifically noted otherwise, and refers to an experimentally determined average molecular weight unless specifically noted otherwise.
As used herein, "average molecular weight" refers to "number average molecular weight" unless noted otherwise. However, when specifically noted, "weight average molecular weight", "viscosity average molecular weight", or other experimentally determined average molecular weight, etc. can be used. However, it should be noted that the specification such as the Examples is described so that the average molecular weight of PEG (e.g., PEG 4000) typically refers to number average molecular weight, and the average molecular weight of poloxamer (e.g., poloxamer 407) typically refers to weight average molecular weight. Experimentally measured average molecular weight can be determined by those skilled in the art by, for example, measuring the osmotic pressure, increase in boiling point, or decrease in freezing point of a solution with a polymer of which average molecular weight is to be measured dissolved therein optionally after diluting the solution. This is used as weight average molecular weight, number average molecular weight, etc. depending on the method.
As used herein, "hydratable compound" refers to a compound with a strong tendency to hydrate in a solution.
As used herein, "poorly soluble compound" generally refers to a compound that is poorly soluble to an aqueous solvent. While poorly soluble compounds are described in more detail elsewhere herein, poorly soluble compounds can be evaluated by the value of LogP or by the ratio of solubilities in an aqueous solvent and non-aqueous solvent, or by a structural feature of a compound.
As used herein, "LogP" of a compound refers to Log (Co/Cw) of the compound at an atmospheric pressure of 1 atm at 25°C (wherein Co: concentration of compound in n-octanol, and Cw: concentration of compound in water). However, LogP of a compound at a specific pH herein refers to the partition coefficient when an acid or base (e.g., hydrochloric acid or sodium hydroxide) that does not degrade a test compound is added to a mixture of octanol, water, and the test compound as of the experiment for obtaining the octanol/water partition coefficient in a minimum amount to adjust the pH of an aqueous layer of the mixture to the specific pH.
As used herein, "substituent" refers to an atom or a functional group that has replaced a certain chemical group with another in a chemical structure of a compound.
If a certain group, moiety, or compound is "substituted", the group, moiety, or compound has at least one hydrogen therein substituted with a group (substituent) other than hydrogen. The number of such substituents is not particularly limited, if substitutable, and is one or more. Except for cases where specifically noted, the description for each group is also applicable when the group is a part of, or a substituent of, another group. If, for example, a $C_{1-6}$ alkyl group is substituted with a certain substituent, the number of carbon atoms of the substituent is not included in the number of carbon atoms of the alkyl group. The same applies to other groups.
"$C_{1-6}$" means that the number of carbon atoms is 1 to 6. The same applies to other numbers. For example, "$C_{1-4}$" means that the number of carbon atoms is 1 to 4. Thus, "$C_{1-6}$ alkyl group" refers to a linear or branched saturated hydrocarbon group with 1 to 6 carbon atoms.
As used herein, "optionally substituted" means that a certain group may or may not be substituted with a substituent.
As used herein, "alkyl group" refers to a linear or branched saturated hydrocarbyl group with a carbon atom. Examples of $C_{1-3}$ alkyl group include a methyl group, ethyl group, propyl group, and isopropyl group. Examples of $C_{1-4}$ alkyl group include the aforementioned $C_{1-3}$ alkyl groups as well as a butyl group, isobutyl group, sec-butyl group, and tert-butyl group. Examples of $C_{1-6}$ alkyl group include the aforementioned $C_{1-4}$ alkyl groups, as well as a pentyl group, isopentyl group, neopentyl group, hexyl group, etc.
As used herein, "alkylene group" is a divalent group generated by further removing one hydrogen from an "alkyl group". Specific examples of alkylene group include, but are not limited to, $-CH_2-$, $-CH_2CH_2-$, $-(CH_2)_3-$, $-CH_2CH(CH_3)-$, $-(CH_2)_4-$, $-CH_2CH_2CH(CH_3)-$, $-CH_2CH(CH_3)CH_2-$, etc.
As used herein, "alkenyl group" refers to a linear or branched hydrocarbyl group having a carbon atom and one or more carbon-carbon double bonds. One or more carbon-carbon double bonds may be inside (e.g., double bond in 2-butenyl) or at a terminus (e.g., double bond in 1-butenyl). Examples of $C_{2-4}$ alkenyl group include ethenyl groups (vinyl groups), 1-propenyl groups, 2-propenyl groups, 1-butenyl groups, 2-butenyl groups, butadienyl groups, etc. Examples of $C_{2-6}$ alkenyl group include the aforementioned $C_{2-4}$ alkenyl groups, as well as pentenyl groups, pentadienyl groups, hexenyl groups, etc.

As used herein, "alkenylene group" is a divalent group generated by further removing one hydrogen from "alkenyl group". Specific examples of alkenylene group include, but are not limited to, -CH=CH-, $-CH=CH-CH_2-$, $-CH=CH-(CH_2)_2-$, - $CH_2-CH=CH-CH_2-$, $-CH=C(CH_3)-CH_2-$, -CH=CH-CH=CH-, $-CH=CH-(CH_2)_3-$, etc.

As used herein, "alkoxyl group" is a monovalent group of -O-alkyl. Examples of alkoxyl group include $C_{1-6}$ alkoxyl groups (i.e., $C_{1-6}$ alkyl-O-), $C_{1-4}$ alkoxyl groups (i.e., $C_{1-4}$ alkyl-O-), etc.

As used herein, "aliphatic group" refers to an alkyl group, alkylene group, alkenyl group, alkenylene group, alkynyl group, and alkynylene group, and does not include cyclic hydrocarbon groups. Specific examples of $C_{1-5}$ aliphatic group include, but are not limited to, $-CH_3$,$-CH_2-$, $-CH_2CH_2-$, $-CH_2CH_3$, -CH=CH-, $-CH=CH_2$, -C≡CH, -C≡C-,$-CH_2CH_2CH_3$, $-CH_2CH_2CH_2-$, $-CH=CHCH_3$, $-C≡CCH_3$, $-CH_2CH=CH_2$, $-CH_2C$ ≡CH, $-CH_2CH(CH_3)-$, $-CH_2CH(CH_3)CH_3$, etc.

As used herein, "halo" or "halogen" refers to fluorine (fluoro), chlorine (chloro), bromine (bromo), or iodine (iodo).

As used herein, the term "aryl group" refers to a single aromatic ring or a fused polycyclic system wherein at least one of the rings is aromatic and all atoms in the ring are carbon. An aryl group comprises a phenyl group. An aryl group also includes a fused polycyclic system (e.g., cyclic system comprising 2, 3, or 4 rings), wherein at least one ring is aromatic, but other rings may or may not be aromatic. Rings in a fused polycyclic system can be connected to one another via fusion, spiro, or crosslinking bond if permitted by the valency requirement. Typical examples of aryl group include, but are not limited to, phenyl groups, indenyl groups, naphthyl groups, 1,2,3,4-tetrahydronaphthyl groups, anthryl groups, pyrenyl groups, etc.

As used herein, the term "heteroaryl group" refers to a single aromatic ring or fused polycyclic system having at least one heteroatom in a ring. The heteroatom is selected from the group consisting of oxygen, nitrogen, and sulfur. A heteroaryl group encompasses a single aromatic ring having about 1 to 6 carbon atoms and about 1 to 4 heteroatoms selected from the group consisting of oxygen, nitrogen, and sulfur in a ring. Examples of such a ring include, but are not limited to, pyridyl groups, pyrimidinyl groups, pyradinyl groups, oxazolyl groups, furyl groups, etc. Sulfur and nitrogen atoms can also be in an oxidated form if a ring is aromatic. A heteroaryl group also encompasses fused polycyclic systems (e.g., cyclic systems comprising 2, 3, or 4 rings) in which a previously defined heteroaryl group can form a fused polycyclic system by fusing with one or more rings selected from heteroaryl (e.g., forming naphthyridinyl such as 1,8-naphthyridinyl), heterocycle (e.g., forming 1,2,3,4-tetrahydronaphthyridinyl such as 1,2,3,4-tetrahydro-1,8-naphthyridinyl), carbocycle (e.g., forming 5,6,7,8-tetrahydroquinolyl), and aryl (e.g., forming indazolyl). It is understood that a position of a bond in the fused polycyclic system described above can be at any position of the fused polycyclic system including the heteroaryl, heterocycle, aryl, or carbocyclic moiety of the fused polycyclic system, and any suitable atom of the fused polycyclic system including a carbon atom and heteroatom (e.g., nitrogen). Examples of heteroaryl group include, but are not limited to, quinolyl groups, benzothiazolyl groups, pyridyl groups, pyrrolyl groups, pyradinyl groups, pyrimidinyl groups, pyridazinyl groups, pyrazolyl groups, thienyl groups, indolyl groups, imidazolyl groups, oxazolyl groups, isooxazolyl groups, thiazolyl groups, furyl groups, oxadiazolyl groups, thiadiazolyl groups, isoquinolyl groups, benzooxazolyl groups, indazolyl groups, quinoxalyl groups, quinazolyl groups, 5,6,7,8-tetrahydroisoquinolinyl benzofuranyl groups, benzoimidazolyl groups, quinazolinyl-4(3H)-one groups, triazolyl groups, 4,5,6,7-tetrahydro-1H-indazole groups, 3b,4,4a,5-tetrahydro-1H-cyclopropa[3,4]cyclopenta[1,2-c]pyrazole groups, etc.

As used herein, "carbocycle" or "carbocyclic group", alone or as a part of another group, refers to a monocyclic, bicyclic, or tricyclic hydrocarbon group, or polycyclic hydrocarbon group with more rings, which is completely saturated or comprises one or more unsaturated units that is not aromatic. In one embodiment, a carbocyclic group can be a monocyclic $C_{3-9}$ hydrocarbon group, bicyclic $C_{8-12}$ hydrocarbon group, or tricyclic $C_{10-16}$ hydrocarbon group. Any individual ring in the carbocyclic group described above can have 3 to 7 ring atoms. Examples of carbocyclic group include, but are not limited to, cycloalkyl groups such as cyclopropyl groups, cyclobutyl groups, cyclopentyl groups, cyclohexyl groups, cycloheptyl groups, cyclooctyl groups, and cyclononyl groups, cycloalkenyl groups such as cyclopropenyl groups, cyclobutenyl groups, cyclopentenyl groups, cyclohexenyl groups, cycloheptenyl groups, cyclooctenyl groups, and cyclononenyl groups, cycloalkynyl groups such as cyclopropynyl groups, cyclobutynyl groups, cyclopentynyl groups, cyclohexynyl groups, cycloheptynyl groups, cyclooctynyl groups, and cyclononyl groups, adamantyl groups, etc. In a carbocyclic group, one of the atoms in the ring may be bound to the rest of the portions of the molecule if possible.

As used herein, "heterocycle" or "heterocyclic group", alone or as a part of another group, refers to a monocyclic, bicyclic, or tricyclic system, or polycyclic system with more rings, which is completely saturated or comprises one or more unsaturated units that is not aromatic, wherein at least one ring in the cyclic system comprises one or more same or different heteroatoms. In some embodiments, a "heterocycle" or "heterocyclic group" has 3 to 14 atoms in a ring, wherein one or more atoms in a ring is a heteroatom independently selected from oxygen, sulfur, nitrogen, or phosphorous, and each ring in the cyclic system comprises 3 to 8 atoms.

Examples of heterocyclic group include, but are not limited to, monocycles such as 2-tetrahydrofuranyl groups, 3-tetrahydrofuranyl groups, 2-tetrahydrothiophenyl groups, 3-tetrahydrothiophenyl groups, 2-morphlino groups, 3-morpholino groups, 4-morpholino groups, 2-thiomorpholino groups, 3-thiomorpholino groups, 4-thiomorpholino groups, 1-pyrrolidinyl groups, 2-pyrrolidinyl groups, 3-pyrrolidinyl groups, 1-tetrahydropiperazinyl groups, 2-tetrahydropiperazinyl groups, 3-tetrahydropiperazinyl groups, 1-piperidinyl groups, 2-piperidinyl groups, 3-piperidinyl groups, 1-pyrazolinyl

groups, 3-pyrazolinyl groups, 4-pyrazolinyl groups, 5-pyrazolinyl groups, 1-piperidinyl groups, 2-piperidinyl groups, 3-piperidinyl groups, 4-piperidinyl groups, 2-thiazolidinyl groups, 3-thiazolidinyl groups, 4-thiazolidinyl groups, 1-imidazolidinyl groups, 2-imidazolidinyl groups, 4-imidazolidinyl groups, and 5-imidazolidinyl groups, and bicycles such as 3-1H-benzoimidazol-2-one groups, 3-(1-alkyl)-benzoimidazol-2-one groups, indolinyl groups, tetrahydroquinolinyl groups, tetrahydroisoquinolinyl groups, benzothiolane groups, benzodithian groups, and 1,3-dihydro-imidazol-2-one groups. In a heterocyclic group, one of the atoms in the ring may be bound to the rest of the portions of the molecule if possible.

As used herein, the term "unsaturated" means that a certain portion has one or more unsaturated units.

As used herein, "cyclic group" refers to an aryl group, arylene group, monovalent or divalent carbocyclic group, monovalent or divalent heterocyclic group, heteroaryl group, and heteroarylene group. Specific examples of 5- to 7-membered cyclic group include, but are not limited to, phenyl, phenylene, cyclopentyl group, cyclohexyl group, cycloheptyl group, cyclohexenyl group, cyclohexynyl group, adamantyl group, quinolyl group, benzothiazolyl group, pyridyl group, pyrrolyl group, pyrazinyl group, pyrimidinyl group, pyridazinyl group, pyrazolyl group, thienyl group, imidazolyl group, tetrahydrofuranyl group, morpholino group, pyrrolidinyl group, piperidinyl group, thiazolidinyl group, etc.

A "heterocycle", "heterocyclic group", or "heteroaryl group", when substituted, may have a substituent on a heteroatom if substitutable.

As used herein, "means" refers to anything which can be a tool for attaining a certain objective (e.g., detection or therapy). As used herein, "means for selective recognition (detection)" especially refers to means which can recognize (detect) a certain subject differently from others.

As used herein, "drug component" refers to any component that can be a constituent of a drug. Examples thereof include an active ingredient (component itself exhibiting efficacy), additive (component that is not expected to have efficacy in itself, but is expected to serve a certain role (e.g., excipient, lubricating agent, surfactant, etc.) when contained as a drug), adjuvant (enhances the efficacy of the active ingredient), etc. A drug component may be an independent substance, or a combination of a plurality of substances or agents. A drug component can also encompass any combination such as a combination of an active ingredient and an additive, and a combination of an adjuvant and an active ingredient.

As used herein, "active ingredient" refers to a component that exerts the intended efficacy. An individual or a plurality of components can fall under an active ingredient.

As used herein, "additive" refers to any component that is not expected to have efficacy, but serves a certain role when contained as a drug. Examples thereof include pharmaceutically acceptable carriers, stabilizing agents, auxiliaries, solubility improving agents, solubilizing agents, diluents, excipients, buffers, binding agents, blasting agents, diluents, flavoring agents, and lubricants.

As used herein, "subject" refers to an entity (including organisms such as a human, and cells, blood, and serum extracted therefrom), which is to be subjected to therapy, etc.

As used herein, an "agent" is used in a broad sense, and may be any substance or other elements (e.g., energy such as light, radiation, heat, and electricity) as long as the intended object can be achieved. Examples of such a substance include, but are not limited to, proteins, polypeptides, oligopeptides, peptides, polynucleotides, oligonucleotides, nucleotides, nucleic acids (e.g., including DNA such as cDNA and genomic DNA, and RNA such as mRNA), polysaccharides, oligosaccharides, lipids, organic small molecules (e.g., hormones, ligands, information transmitting substances, organic small molecules, molecules synthesized by combinatorial chemistry, small molecules which can be utilized as a pharmaceutical product (e.g., a low molecular weight ligand), etc.), and composite molecules thereof.

As used herein, "therapeutic drug (agent)" broadly refers to any agent that can treat a condition of interest. In one embodiment of the present disclosure, "therapeutic drug" may be a pharmaceutical composition comprising an active ingredient and one or more pharmaceutically acceptable carriers. A pharmaceutical composition can be manufactured, for example, by any method that is known in the technical field of pharmaceutical science by mixing an active ingredient with the carrier described above.

As used herein, "prophylactic drug (agent)" broadly refers to any agent capable of preventing a condition of interest.

As used herein, "kit" refers to a unit providing parts to be provided (e.g., detection agent, therapeutic agent, prophylactic agent, user manual, etc.) which are generally separated into two or more segments. Such a kit form is preferred when providing a composition, which should not be provided in a mixed state for stability, etc. and is preferably used by mixing immediately prior to use. Such a kit advantageously comprises an instruction or user manual describing how the provided parts are used or how the reagent should be processed. When a kit is used as a reagent kit herein, an instruction describing the method of use of a detection agent, therapeutic agent, prophylactic agent, etc. is generally included in the kit.

As used herein, "instruction" is a document with an explanation of the method of use of the present disclosure for physicians or other users. The instruction has an instructive description for administration of a drug of the present disclosure, etc. The instruction also has an instructive description for the dosage form. The instruction is prepared in accordance with a format specified by the regulatory agency of the country in which the present disclosure is practiced

(e.g., the Ministry of Health, Labour and Welfare in Japan, Food and Drug Administration (FDA) in the U.S., etc.), with an explicit description showing approval by the regulatory agency. The instruction is a so-called package insert and is typically provided in, but not limited to, paper media. Instructions can also be provided in a form such as electronic media (e.g., web sites provided on the Internet or emails).

The term "about" refers to the indicated value plus or minus 10%. When used in the context of a temperature, "about" refers to the indicated temperature plus or minus 5°C. When used in the context of pH, "about" refers to the indicated pH plus or minus 0.5.

(Preferred embodiments)

Preferred embodiments of the present disclosure are described hereinafter. Embodiments provided below are provided to facilitate the understanding of the present disclosure. It is understood that the scope of the present disclosure should not be limited to the following descriptions. Thus, it is apparent that those skilled in the art can make appropriate modifications within the scope of the present disclosure by referring to the descriptions herein. It is understood that the following embodiments can be used alone or in combination.

In one aspect, the present disclosure provides improvement in the stability of a polyether compound and/or solubility (e.g., water solubility) of a poorly soluble compound. Any means for achieving this is intended to be within the scope of the present disclosure. For example, even without an explicit description, a description of a method using a certain component is also intended as embodiments reflecting other means such as a composition comprising the same component, use of the same component, or the same component for use in the same method. While the present specification describes the present disclosure primarily in embodiments of a composition, a description on a composition for a certain use comprising a certain component is also intended as embodiments reflecting other means such as a method for the same use of the same component or the same use of the same component.

(Stability improving composition)

In one embodiment, the present disclosure provides a composition for stabilizing a polyether compound, comprising one or more types of chelate compounds. In one embodiment, the present disclosure provides a composition comprising a polyether compound utilizing such an effect.

In one embodiment, a polyether compound to be stabilized can comprise a repeat unit of -$R^2$-O-, wherein $R^2$ is an optionally substituted $C_{1-10}$ aliphatic group, or a group wherein any of the carbon atoms in the $C_{1-10}$ aliphatic group is replaced with an optionally substituted 5- to 7-membered cyclic group, wherein "optionally substituted" means that one or more hydrogen atoms is, each independently, optionally replaced with a monovalent $C_{1-5}$ aliphatic group, halogen, -OH, -O-(monovalent $C_{1-5}$ aliphatic group), -COOH, -CO-(monovalent $C_{1-5}$ aliphatic group), -CO-$NH_2$, -CO-NH- (monovalent $C_{1-5}$ aliphatic group), -COH, -SH, - S-(monovalent $C_{1-5}$ aliphatic group), -$NH_2$, -NH-(monovalent $C_{1-5}$ aliphatic group), -N-(monovalent $C_{1-5}$ aliphatic group)$_2$, -$NO_2$, -$PO_3H$, -$SO_3H$, -CN, or monovalent 5- to 7-membered cyclic group. In one embodiment, a polyether compound to be stabilized can have an average molecular weight of about 200 to 50000, about 500 to 20000, about 1000 to 15000, about 2000 to 10000, about 500 to 20000, about 500 to 10000, about 500 to 5000, about 1000 to 50000, about 1000 to 20000, about 1000 to 10000, about 1500 to 20000, or about 1500 to 15000. In one embodiment, a polyether compound to be stabilized can comprise at least one of poloxamer, polyethylene glycol, polypropylene glycol, and polybutylene glycol. In one embodiment, a polyether compound to be stabilized can comprise poloxamer and polyethylene glycol.

In one embodiment, poloxamer to be stabilized can have an average molecular weight of about 500 to 20000, about 1000 to 15000, about 2000 to 10000, about 500 to 20000, about 500 to 10000, about 500 to 5000, about 1000 to 50000, about 1000 to 20000, about 1000 to 10000, about 1500 to 20000, or about 1500 to 15000. In one embodiment, the stabilizing composition of the present disclosure can comprise poloxamer at about 1 to 50 wt%, about 1 to 40 wt%, about 2 to 30 wt%, about 5 to 25 wt%, about 10 to 30 wt%, about 1 to 20 wt%, about 2 to 20 wt%, about 5 to 20 wt%, about 15 to 30 wt%, or about 15 to 40 wt%. In one embodiment, poloxamer to be stabilized can comprise poloxamer 101, poloxamer 105, poloxamer 108, poloxamer 122, poloxamer 123, poloxamer 124, poloxamer 181, poloxamer 182, poloxamer 183, poloxamer 184, poloxamer 185, poloxamer 188, poloxamer 212, poloxamer 215, poloxamer 217, poloxamer 231, poloxamer 234, poloxamer 235, poloxamer 237, poloxamer 238, poloxamer 282, poloxamer 284, poloxamer 288, poloxamer 331, poloxamer 333, poloxamer 334, poloxamer 335, poloxamer 338, poloxamer 401, poloxamer 402, poloxamer 403, or poloxamer 407. In one embodiment, poloxamer to be stabilized can comprise poloxamer 407.

In one embodiment, poloxamer to be stabilized can comprise about 5 to 95 wt%, about 10 to 95 wt%, about 20 to 95 wt%, about 30 to 95 wt%, about 40 to 95 wt%, about 5 to 90 wt%, about 10 to 90 wt%, about 20 to 90 wt%, about 30 to 90 wt%, about 40 to 90 wt%, about 5 to 80 wt%, about 10 to 80 wt%, about 20 to 80 wt%, about 30 to 80 wt%, about 40 to 80 wt%, about 5 to 70 wt%, about 10 to 70 wt%, about 20 to 70 wt%, about 30 to 70 wt%, about 40 to

70 wt%, about 5 to 60 wt%, about 10 to 60 wt%, about 20 to 60 wt%, about 30 to 60 wt%, about 40 to 60 wt%, about 5 to 50 wt%, about 10 to 50 wt%, about 20 to 50 wt%, about 30 to 50 wt%, or about 40 to 50 wt% of polyoxyethylene per molecule.

In one embodiment, poloxamer to be stabilized can have a percentage of the number of oxyethylene units with respect to oxyethylene units + oxypropylene units per molecule of about 5 to 95%, about 10 to 95%, about 15 to 95%, about 20 to 95%, about 30 to 95%, about 40 to 95%, about 50 to 95%, about 5 to 90%, about 10 to 90%, about 15 to 90%, about 20 to 90%, about 30 to 90%, about 40 to 90%, about 50 to 90%, about 5 to 80%, about 10 to 80%, about 15 to 80, about 20 to 80%, about 30 to 80%, about 40 to 80%, about 50 to 80%, about 5 to 70%, about 10 to 70%, about 15 to 70%, about 20 to 70%, about 30 to 70%, about 40 to 70%, about 50 to 70%, about 5 to 65%, about 10 to 65%, about 15 to 65%, about 20 to 65%, about 30 to 65%, about 40 to 65%, about 50 to 65%, about 5 to 60%, about 10 to 60%, about 15 to 60%, about 20 to 60%, about 30 to 60%, about 40 to 60%, or about 50 to 60%. In one preferred embodiment, poloxamer to be stabilized can have a percentage of the number of oxyethylene units with respect to oxyethylene units + oxypropylene units per molecule of about 70% or less, about 65% or less, or about 60% or less.

In one embodiment, poloxamer to be stabilized can have polyoxypropylene at an average molecular weight of about 500 to 15000, about 600 to 10000, about 700 to 7000, about 900 to 10000, about 900 to 7000, about 900 to 4000, about 1000 to 10000, about 1000 to 7000, about 1500 to 7000, about 2000 to 7000, or about 2500 to 7000 per molecule.

In one embodiment, poloxamer to be stabilized can be a molecule having the structure of formula I:

[Chemical Formula 2]

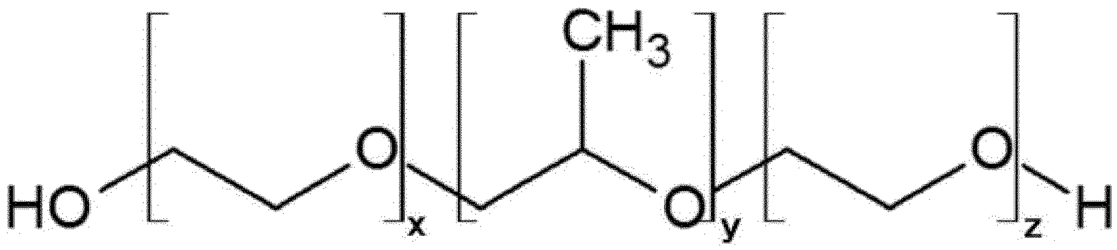

(formula I)

wherein x, y, and z are each independently selected integer that is 0 or greater, or a molecule modified therefrom. In one embodiment, x, y, and z can be x = 1 to 1000, y = 1 to 1000, and z = 1 to 1000. In one embodiment, x, y, and z can be x = 10 to 500, y = 10 to 500, and z = 10 to 500. In one embodiment, x, y, and z can be x = 50 to 200, y = 50 to 200, and z = 50 to 200. In one embodiment, poloxamer to be stabilized can be a compound wherein the total molecular weight of partial structures represented by repeat units of $-CH_2-CH_2-O-$ and $-CH_2-CH(CH_3)-O-$ is 70% or greater, 75% or greater, 80% or greater, 85% or greater, 90% or greater, 95% or greater, 98% or greater, or 99% or greater with respect to the molecular weight of the entire compound.

In one embodiment, poloxamer to be stabilized can be a compound having 1, 2, 3, 4, 5, 6, 7, 8, 9, 10, 11, 12, 13, 14, 15, 16, 17, 18, 19, 20, 21, 22, 23, 24, or 25 modifications, in any combination, selected from the group consisting of the following modifications in the structure of formula I described above:

*a branching modification which replaces one or more hydrogen atoms in a molecule with -(repeat unit of $-CH_2-CH_2-O-$ and/or $-CH_2-CH(CH_3)-O-$)-H;

*a modification which replaces one or more of repeat units of $-CH_2-CH_2-O-$ or $-CH_2-CH(CH_3)-O-$ independently with a group selected from the group consisting of $-R^2-O-$, $-R^2-S-$, $-R^2-$, and $-R^2-N(R^1)-$;

*a modification which replaces one or more -OH groups independently with a group selected from the group consisting of $-NR^1_2$, $-OR^1$, $-SR^1$, and $-R^1$;

*a modification which replaces one or more hydrogen atoms in a molecule with a halogen or a methyl group; and

*any combination of the modifications described above; wherein

$R^1$ is an optionally substituted $C_{1-10}$ aliphatic group, or a group wherein any of the carbon atoms in the $C_{1-10}$ aliphatic group is replaced with an optionally substituted 5- to 7-membered cyclic group, and

$R^2$ is an optionally substituted $C_{1-10}$ aliphatic group, or a group wherein any of the carbon atoms in the $C_{1-10}$ aliphatic group is replaced with an optionally substituted 5- to 7-membered cyclic group,

wherein "optionally substituted" means that one or more hydrogen atoms is, each independently, optionally

replaced with a monovalent $C_{1-5}$ aliphatic group, halogen, -OH, -O-(monovalent $C_{1-5}$ aliphatic group), -COOH, -CO-(monovalent $C_{1-5}$ aliphatic group), -CO-$NH_2$, -CO-NH- (monovalent $C_{1-5}$ aliphatic group), -COH, -SH, -S-(monovalent $C_{1-5}$ aliphatic group), -$NH_2$, -NH-(monovalent $C_{1-5}$ aliphatic group), -N-(monovalent $C_{1-5}$ aliphatic group)$_2$, -$NO_2$, -$PO_3H$, -$SO_3H$, -CN, or monovalent 5- to 7-membered cyclic group.

In one embodiment, poloxamer to be stabilized can be a compound comprising 1, 2, 3, 4, or 5 branching modifications which replace one or more hydrogen atoms in a molecule with -(repeat unit of -$CH_2$-$CH_2$-O- and/or -$CH_2$-$CH(CH_3)$-O-)-H. In one embodiment, poloxamer to be stabilized can be a propylene glycol compound wherein one hydrogen atom of each $CH_3$ of 1, 2 or 3 -$CH_2$-$CH(CH_3)$-O-repeat units is replaced with -OH or -(repeat unit of -$CH_2$-$CH_2$-O- and/or -$CH_2$-$CH(CH_3)$-O-)-H.

In one embodiment, poloxamer to be stabilized can be a compound including a modification which replaces 1 to 20, 1 to 15, 1 to 10, or 1 to 5 such as 1, 2, 3, 4, 5, 6, 7, 8, 9, 10, 11, 12, 13, 14, 15, 16, 17, 18, 19, or 20 of repeat units of -$CH_2$-$CH_2$-O- or -$CH_2$-$CH(CH_3)$-O- independently with a group selected from the group consisting of -$R^2$-O-, -$R^2$-S-, -$R^2$-, and -$R^2$-N($R^1$)-.

In one embodiment, poloxamer to be stabilized can be a compound including a modification which replaces 1 to 20, 1 to 15, 1 to 10, or 1 to 5 such as 1, 2, 3, 4, or 5 -OH groups independently with a group selected from the group consisting of -$NR^1_2$, -$OR^1$, -$SR^1$, and -$R^1$.

In one embodiment, poloxamer to be stabilized can be a compound including a modification which replaces 1 to 100, 1 to 70, 1 to 50, 1 to 30, 1 to 20, 1 to 15, 1 to 10, or 1 to 5 such as 1, 2, 3, 4, 5, 6, 7, 8, 9, 10, 11, 12, 13, 14, 15, 16, 17, 18, 19, or 20 hydrogen atoms in a molecule with a halogen or a methyl group.

In one embodiment, polypropylene glycol or polybutylene glycol to be stabilized can be a compound represented by an average molecular weight and modification range similar to the poloxamer to be stabilized described above.

In one embodiment, polyethylene glycol to be stabilized can have an average molecular weight of about 200 to 50000, about 500 to 20000, about 1000 to 15000, about 2000 to 10000, about 500 to 20000, about 500 to 10000, about 500 to 5000, about 1000 to 50000, about 1000 to 20000, about 1000 to 10000, about 1500 to 20000, or about 1500 to 15000. In one embodiment, the stabilizing compound of the present disclosure can comprise about 1 to 50 wt%, about 1 to 40 wt%, about 2 to 30 wt%, about 5 to 25 wt%, about 10 to 30 wt%, about 1 to 20 wt%, about 2 to 20 wt%, about 5 to 20 wt%, about 15 to 30 wt%, or about 15 to 40 wt% of polyethylene glycol. In one embodiment, polyethylene glycol to be stabilized can comprise PEG 200, PEG 300, PEG 400, PEG 600, PEG 1000, PEG 1500, PEG 2000, PEG 4000, PEG 6000, PEG 8000, PEG 10000, or PEG 20000. In one embodiment, polyethylene glycol to be stabilized can comprise PEG 4000.

In one embodiment, polyethylene glycol to be stabilized can be a molecule having the structure of formula II:

$$HO\text{-}(CH_2\text{-}CH_2\text{-}O)_n\text{-}H \qquad \text{(formula II)}$$

wherein n is an integer that is 0 or greater, or a molecule modified therefrom. In one embodiment, n can be n = 1 to 1000, n = 10 to 500, or n = 50 to 200. In one embodiment, polyethylene glycol to be stabilized can be a compound wherein the total molecular weight of partial structures represented by repeat units of -$CH_2$-$CH_2$-O- is 70% or greater, 75% or greater, 80% or greater, 85% or greater, 90% or greater, 95% or greater, 98% or greater, or 99% or greater with respect to the molecular weight of the entire molecule.

In one embodiment, polyethylene glycol to be stabilized can be a compound having 1, 2, 3, 4, 5, 6, 7, 8, 9, 10, 11, 12, 13, 14, 15, 16, 17, 18, 19, 20, 21, 22, 23, 24, or 25 modifications, in any combination, selected from the group consisting of the following modifications in the structure of formula II described above:

*a branching modification which replaces one or more hydrogen atoms in a molecule with -(repeat unit of -$CH_2$-$CH_2$-O-)-H;
*a modification which replaces one or more of repeat units of -$CH_2$-$CH_2$-O- independently with a group selected from the group consisting of -$R^2$-O-, -$R^2$-S-, -$R^2$-, and -$R^2$-N($R^1$)-;
*a modification which replaces one or more -OH groups independently with a group selected from the group consisting of -$NR^1_2$, -$OR^1$, -$SR^1$, and -$R^1$;
*a modification which replaces one or more hydrogen atoms in a molecule with a halogen or a methyl group; and
*any combination of the modifications described above; wherein

$R^1$ is an optionally substituted $C_{1-10}$ aliphatic group, or a group wherein any of the carbon atoms in the $C_{1-10}$ aliphatic group is replaced with an optionally substituted 5- to 7-membered cyclic group, and
$R^2$ is an optionally substituted $C_{1-10}$ aliphatic group, or a group wherein any of the carbon atoms in the $C_{1-10}$ aliphatic group is replaced with an optionally substituted 5- to 7-membered cyclic group,

wherein "optionally substituted" means that one or more hydrogen atoms is, each independently, optionally replaced with a monovalent $C_{1-5}$ aliphatic group, halogen, -OH, -O-(monovalent $C_{1-5}$ aliphatic group), -COOH, -CO-(monovalent $C_{1-5}$ aliphatic group), -CO-$NH_2$, -CO-NH- (monovalent $C_{1-5}$ aliphatic group), -COH, -SH, -S-(monovalent $C_{1-5}$ aliphatic group), -$NH_2$, -NH-(monovalent $C_{1-5}$ aliphatic group), -N-(monovalent $C_{1-5}$ aliphatic group)$_2$, -$NO_2$, -$PO_3H$, -$SO_3H$, -CN, or monovalent 5- to 7-membered cyclic group.

In one embodiment, polyethylene glycol to be stabilized can be a compound including 1, 2, 3, 4, or 5 branching modifications which replace one or more hydrogen atoms in a molecule with -(repeat unit of -$CH_2$-$CH_2$-O-)-H.

In one embodiment, polyethylene glycol to be stabilized can be a compound including a modification which replaces 1 to 20, 1 to 15, 1 to 10, or 1 to 5 such as 1, 2, 3, 4, 5, 6, 7, 8, 9, 10, 11, 12, 13, 14, 15, 16, 17, 18, 19, or 20 of repeat units of -$CH_2$-$CH_2$-O- independently with a group selected from the group consisting of -$R^2$-O-, -$R^2$-S-, -$R^2$-, and -$R^2$-N($R^1$)-.

In one embodiment, polyethylene glycol to be stabilized can be a compound including a modification which replaces 1 to 20, 1 to 15, 1 to 10, or 1 to 5 such as 1, 2, 3, 4, or 5 -OH groups independently with a group selected from the group consisting of -$NR^1_2$, -$OR^1$, -$SR^1$, and -$R^1$.

In one embodiment, polyethylene glycol to be stabilized can be a compound including a modification which replaces 1 to 100, 1 to 70, 1 to 50, 1 to 30, 1 to 20, 1 to 15, 1 to 10, or 1 to 5 such as 1, 2, 3, 4, 5, 6, 7, 8, 9, 10, 11, 12, 13, 14, 15, 16, 17, 18, 19, or 20 hydrogen atoms in a molecule with a halogen or a methyl group.

In one embodiment, a chelate compound in the stabilizing composition of the present disclosure can have the ability to form a chelate wherein when the chelate compound is added to an aqueous solution with a pH of about 7.0 comprising 0.1 ppm of free metal ion ($Cu^{2+}$, $Fe^{3+}$, $Zn^{2+}$, $Ca^{2+}$, $Al^{3+}$, $Ag^+$, etc.) at the same molar concentration of the free metal ion at 25°C and 1 atm and is left standing for a sufficient period of time, the concentration of the free metal ion would be 0.05 ppm or less, 0.02 ppm or less, 0.01 ppm or less, 0.005 ppm or less, 0.002 ppm or less, or 0.001 ppm or less. Examples of chelate compounds that can be added include ethylenediamine, ethylenediaminetetraacetic acid (EDTA), thiosulfuric acid, citric acid, metaphosphoric acid, pyrophosphoric acid, polyphosphoric acid, malic acid, tartaric acid, phytic acid, gluconic acid, lactic acid, dimercaprol, etc. (and salts thereof). Such chelate compounds can be in a form of a corresponding ion, etc. in a solution.

In one embodiment, the stabilizing composition of the present disclosure comprises a chelate compound selected from the group consisting of thiosulfate, ethylenediaminetetraacetic acid (EDTA) or a salt or ion thereof, and citric acid or a salt or ion thereof. In a preferred embodiment, the stabilizing composition of the present disclosure comprises a chelate compound selected from the group consisting of thiosulfate, and ethylenediaminetetraacetic acid (EDTA) or a salt or ion thereof. In a more preferred embodiment, the stabilizing composition of the present disclosure comprises a chelate compound selected from the group consisting of thiosulfate or a salt or ion thereof. In one embodiment, the stabilizing composition of the present disclosure further comprises at least one additional stabilizing component among mannitol and dibutylhydroxytoluene (BHT) in addition to a chelate compound. The additional stabilizing component does not stabilize a polyether compound alone, but does not obstruct the stabilization of a polyether compound when added to a chelate compound such as thiosulfate or EDTA or can further stabilize a polyether compound. Since a polyether compound such as poloxamer can be stabilized by adding dibutylhydroxytoluene (BHT) alone, the stabilizing composition of the present disclosure may comprise dibutylhydroxytoluene (BHT) without comprising a chelate compound.

Polyether compounds can be generally more unstable and difficult to stabilize compared to a thickener such as a cellulose macromolecule (hydroxypropyl cellulose, etc.) For example, a glycoside bond of a cellulose macromolecule can be more stable than an ether bond of a polyether compound. However, the inventors unexpectedly found that polyether compounds can be stabilized efficiently with (a small amount of) chelate compound. Although not wishing to be bound by any theory, a mechanism is envisioned where sulfuric acid based chelate compounds such as thiosulfuric acid in particular stabilize a polyether compound by indirectly protecting other compounds from oxidation by the compound itself being preferentially oxidized and by directly protecting oxygen of an ether moiety of the polyether compound (which can form a hydrogen bond with a molecule in a solvent such as water) by the chelate compound forming a hydrogen bond therewith.

In one embodiment, the stabilizing composition of the present disclosure can comprise a chelate compound at about 0.001 to 5 wt%, about 0.005 to 2 wt%, about 0.005 to 1 wt%, about 0.005 to 0.5 wt%, about 0.005 to 0.2 wt%, about 0.01 to 5 wt%, about 0.01 to 1 wt%, about 0.01 to 0.5 wt%, about 0.01 to 0.2 wt%, about 0.01 to 0.1 wt%, about 0.02 to 1 wt%, about 0.02 to 0.5 wt%, about 0.02 to 0.2 wt%, about 0.02 to 0.1 wt%, about 0.05 to 2 wt%, about 0.05 to 1 wt%, about 0.05 to 0.5 wt%, about 0.05 to 0.2 wt%, about 0.1 to 5 wt%, about 0.1 to 2 wt%, about 0.1 to 1 wt%, or about 0.1 to 0.5 wt%.

In one embodiment, the stabilizing composition of the present disclosure can comprise about 0.1 to 1000 mol%, about 0.1 to 100 mol%, about 0.1 to 50 mol%, about 0.2 to 1000 mol%, about 0.2 to 100 mol%, about 0.2 to 50 mol%, about 0.5 to 1000 mol%, about 0.5 to 1000 mol%, about 0.5 to 100 mol%, about 0.5 to 50 mol%, about 0.5 to 20 mol%, about 0.5 to 10 mol%, about 1 to 1000 mol%, about 1 to 100 mol%, about 1 to 50 mol%, about 1 to 10 mol%, about 2 to 1000 mol%, about 2 to 100 mol%, about 2 to 50 mol%, about 2 to 200 mol%, about 2 to 100 mol%, about 2 to 50

mol%, about 5 to 1000 mol%, about 10 to 500 mol%, about 20 to 200 mol%, about 10 to 500 mol%, about 10 to 200 mol%, about 10 to 100 mol%, about 20 to 1000 mol%, about 20 to 500 mol%, or about 20 to 200 mol% of chelate compound with respect to a polyether compound.

In one embodiment, the stabilizing composition of the present disclosure can comprise at least one stabilizing component among mannitol and dibutylhydroxytoluene (BHT) at about 0.001 to 5 wt%, about 0.005 to 2 wt%, about 0.01 to 1 wt%, about 0.02 to 0.5 wt%, about 0.05 to 0.2 wt%, about 0.01 to 5 wt%, about 0.01 to 0.5 wt%, about 0.01 to 0.2 wt%, about 0.01 to 0.1 wt%, about 0.02 to 1 wt%, about 0.02 to 0.5 wt%, about 0.02 to 0.2 wt%, about 0.05 to 2 wt%, about 0.05 to 1 wt%, about 0.05 to 0.5 wt%, about 0.1 to 5 wt%, about 0.1 to 2 wt%, or about 0.1 to 1 wt%.

In one embodiment, the stabilizing composition of the present disclosure can comprise about 0.01 to 1000 mol%, about 0.01 to 100 mol%, about 0.01 to 10 mol%, about 0.02 to 1000 mol%, about 0.02 to 100 mol%, about 0.02 to 10 mol%, about 0.05 to 1000 mol%, about 0.05 to 100 mol%, about 0.05 to 10 mol%, about 0.1 to 1000 mol%, about 0.1 to 100 mol%, about 0.1 to 50 mol%, about 0.2 to 1000 mol%, about 0.2 to 100 mol%, about 0.2 to 50 mol%, about 0.5 to 1000 mol%, about 0.5 to 1000 mol%, about 0.5 to 100 mol%, about 0.5 to 50 mol%, about 0.5 to 20 mol%, about 0.5 to 10 mol%, about 1 to 1000 mol%, about 1 to 100 mol%, about 1 to 50 mol%, about 1 to 10 mol%, about 2 to 1000 mol%, about 2 to 100 mol%, about 2 to 50 mol%, about 2 to 200 mol%, about 2 to 100 mol%, about 2 to 50 mol%, about 5 to 1000 mol%, about 10 to 500 mol%, about 20 to 200 mol%, about 10 to 500 mol%, about 10 to 200 mol%, about 10 to 100 mol%, about 20 to 1000 mol%, about 20 to 500 mol%, or about 20 to 200 mol% of stabilizing component with respect to a polyether compound.

In one embodiment, the stabilizing composition of the present disclosure can have stability where the change in pH compared to the predetermined initial pH (e.g., about 4.0, about 5.0, about 5.5, about 6.0, about 6.5, about 7.0, about 7.5, or about 8.0) is 0.5 or less, 1.0 or less, or 1.5 or less when the composition is adjusted to the initial pH is transferred to a storage container, then stored for 4 weeks under the environment of 60°C and 1 atm. In one embodiment, the stabilizing composition of the present disclosure can have stability where the change in pH compared to the pH immediately after transfer to a storage container is 0.5 or less, 1.0 or less, or 1.5 or less when the composition is adjusted to a predetermined initial pH (e.g., about 4.0, about 5.0, about 5.5, about 6.0, about 6.5, about 7.0, about 7.5, or about 8.0) is transferred to the storage container, then stored for 4 weeks under the environment of 60°C and 1 atm. Since the pH as of the preparation of a liquid agent such as a drug can be different from the pH immediately after transfer to a storage container or the pH upon shipping from a factory as a product, the test described above can be conducted while taking into consideration a change in the pH as of the preparation or the pH immediately after transfer to a storage container with respect to the pH of a liquid agent of a product to be about 0.5. The test described above does not necessarily need to be conducted on a liquid agent immediately after preparation. A test may be conducted by adjusting a product immediately after being shipped from a factory to a predetermined pH as needed. For example, a test can be conducted by transferring a product immediately after being shipped from a factory to a container for pH adjustment and adjusting the pH in the container, then transferring the product to a container for storage.

In one embodiment, the stabilizing composition of the present disclosure can have stability where a decrease in viscosity when measured with a rotational viscometer under conditions of 25°C and 100 rpm due to adjusting the pH to a predetermined initial pH (e.g., about 4.0, about 5.0, about 5.5, about 6.0, about 6.5, about 7.0, about 7.5, or about 8.0) and then storing the composition for 4 weeks under the environment of 60°C and 1 atm is 5% or less, 10% or less, 15% or less, 20% or less, 25% or less, 30% or less, 35% or less, 40% or less, 45% or less, or 50% or less.

In this aspect of the present disclosure, any pharmaceutical component or active ingredient can be used together with no particular limitation. Examples thereof include, but are not limited to, docetaxel, paclitaxel, capecitabine, oxaliplatin, geftinat, doxorubicin, irinotecan, gemcitabine, pemetrexed, temozolomide, imatinib, vinorelbin, letrozole, teniposide, etoposide, podophyllotoxin, camptothecin, topotecan, vinblastine, vincristine, vindesine, vinflunine, vinpocetine, norcantharidin, silybin, propofol, florfenicol, mitiglinide, artemisinin, dihydroartemisinin, tacrolimus, sirolimus, ibuprofen, nitrendipine, nicardipine, nimodipine, gliclazide, propulsid, nifedipine, felodipine, glibenclamide, acyclovir, oleanolic acid, breviscapine, ferulic acid, paracetamol, palmitoyl rhizoxin, penclomedine, vitamin A, tamoxifen, navelbine, valproic acid, cyclosporine A, amphotericin B, ketoconazole, domperidone, sulpiride, fenofibrate, bezafibrate, itraconazole, miconazole, latanoprost, brinzolamide, erythromycin, roxithromycin, rifaximin, cisapride, diclofenamic acid, terphenazine, theophylline, ketoprofen, furosemide, spironolactone, dipyridamole, piroxicam, mefenamic acid, trichlorothiazide, pindolol, acemetacin, indomethacin, diflunisal, naproxen, triamcinolone, triamcinolone acetonide, fluorometholone, dexamethasone, hydrocortisone, prednisolone, cisplatin, enoxacin, norfloxacin, clofibrate, probucol, simvastatin, reserpine, verapamil hydrochloride, atenolol, digitoxin, digoxin, diazepam, nitrazepam, haloperidol, droperidol, triazolam, phenytoin, phenobarbital, cimetidine, famotidine, omeprazole, lansoprazole, oxethazaine, sucralfate, gefarnate, metoclopramide, tranilast, tolbutamide, mebendazole, albendazole, fenbendazole, cilostazol, phenylbutazone, and celecoxib.

(Solubility improving composition of poorly soluble composition)

In another aspect, the present disclosure provides a composition for improving the solubility (e.g., water solubility) of a poorly soluble compound. In one embodiment, the present disclosure provides a poorly soluble compound-containing composition which utilizes this effect. In one embodiment, the solubility improving composition of the present disclosure may comprise any feature of the stability improving composition of the present disclosure.

In one embodiment, the present disclosure provides a composition for improving the solubility of a poorly soluble compound by a combination of a hydrophobic polyoxy aliphatic moiety-containing polyether compound and a hydratable compound, comprising at least one of a hydrophobic polyoxy aliphatic moiety-containing polyether compound and a hydratable compound. It is understood that a hydrophobic polyoxy aliphatic moiety-containing polyether compound functions as a surfactant by having a hydrophobic moiety and a hydrophilic moiety to improve the solubility of a poorly soluble compound (solubilizing capability). A hydrophobic polyoxy aliphatic moiety-containing polyether compound has the surfactant capability reduced by hydration and hydrophilization of the hydrophobic moiety thereof (e.g., by exposure to low temperature condition), which can result in reduced solubilizing capability. Although not wishing to be bound by any theory, it is understood that if a hydrophobic polyoxy aliphatic moiety-containing polyether compound is combined with a hydratable compound, the hydratable compound draws many water molecules, resulting in preventing the water molecules from hydrating the hydrophobic polyoxy aliphatic moiety-containing polyether compound, thus improving the solubilizing capability of the hydrophobic polyoxy aliphatic moiety-containing polyether compound and preventing the decrease thereof.

In one embodiment, a hydrophobic polyoxy aliphatic moiety-containing polyether compound can comprise a portion of repeats of -O- ($C_{3-20}$ aliphatic group), -O- ($C_{3-15}$ aliphatic group), -O- ($C_{3-10}$ aliphatic group), -O- ($C_{3-7}$ aliphatic group)-, O- ($C_{3-5}$ aliphatic group), -O-($C_{3-4}$ aliphatic group), or -O-($C_3$ aliphatic group). In one embodiment, a hydrophobic polyoxy aliphatic moiety-containing polyether compound can have an average molecular weight of about 500 to 20000, about 1000 to 15000, about 2000 to 10000, about 500 to 20000, about 500 to 10000, about 500 to 5000, about 1000 to 50000, about 1000 to 20000, about 1000 to 10000, about 1500 to 20000, or about 1500 to 15000. In one embodiment, a hydrophobic polyoxy aliphatic moiety-containing polyether compound can be poloxamer, polypropylene glycol, or polybutylene glycol. In one embodiment, a hydrophobic polyoxy aliphatic moiety-containing polyether compound can be poloxamer.

In one embodiment, a hydratable compound can be polyethylene glycol, propylene glycol, glycerol, or ionic salt. In one embodiment, an ionic salt can be, but is not limited to, a salt generated from a reaction between a metal element such as sodium, potassium, calcium, or magnesium and a strong acid such as hydrochloric acid, nitric acid, or sulfuric acid (e.g., sodium chloride, potassium sulfate, etc.) In one embodiment, a hydratable compound can be polyethylene glycol.

In one embodiment, the solubility improving composition of the present disclosure can comprise a hydrophobic polyoxy aliphatic moiety-containing polyether compound (e.g., poloxamer) and a hydratable compound (e.g., polyethylene glycol) at a total of 10 wt% or greater, 12 wt% or greater, 15 wt% or greater, 25 wt% or greater, 30 wt% or greater, 35 wt% or greater, 40 wt% or greater, 45 wt% or greater, or 50 wt% or greater. A suitable amount of a hydrophobic polyoxy aliphatic moiety-containing polyether compound (e.g., poloxamer) and hydratable compound (e.g., polyethylene glycol) in the solubility improving composition of the present disclosure can vary depending on the type of poorly soluble compound.

In one embodiment, poloxamer for improving the solubility can have an average molecular weight of about 500 to 20000, about 1000 to 15000, about 2000 to 10000, about 500 to 20000, about 500 to 10000, about 500 to 5000, about 1000 to 50000, about 1000 to 20000, about 1000 to 10000, about 1500 to 20000, or about 1500 to 15000. In one embodiment, the solubility improving composition of the present disclosure can comprise poloxamer at about 1 to 40 wt%, about 1 to 40 wt%, about 2 to 30 wt%, about 5 to 25 wt%, about 10 to 30 wt%, about 1 to 20 wt%, about 2 to 20 wt%, about 5 to 20 wt%, about 15 to 30 wt%, or about 15 to 40 wt%. In one embodiment, poloxamer for improving the solubility can comprise poloxamer 101, poloxamer 105, poloxamer 108, poloxamer 122, poloxamer 123, poloxamer 124, poloxamer 181, poloxamer 182, poloxamer 183, poloxamer 184, poloxamer 185, poloxamer 188, poloxamer 212, poloxamer 215, poloxamer 217, poloxamer 231, poloxamer 234, poloxamer 235, poloxamer 237, poloxamer 238, poloxamer 282, poloxamer 284, poloxamer 288, poloxamer 331, poloxamer 333, poloxamer 334, poloxamer 335, poloxamer 338, poloxamer 401, poloxamer 402, poloxamer 403, or poloxamer 407. In one embodiment, poloxamer for improving the solubility can comprise poloxamer 407.

In one embodiment, poloxamer for improving the solubility can comprise about 5 to 95 wt%, about 10 to 90 wt%, about 20 to 80 wt%, about 30 to 75 wt%, about 30 to 95 wt%, about 30 to 90 wt%, about 30 to 80 wt%, about 30 to 75 wt%, about 30 to 70 wt%, about 30 to 60 wt%, about 50 to 90 wt%, about 50 to 80 wt%, about 50 to 75 wt%, or about 50 to 70 wt% of polyoxyethylene per molecule.

In one embodiment, poloxamer for improving the solubility can have polyoxypropylene with an average molecular weight of about 500 to 15000, about 600 to 10000, about 700 to 7000, about 900 to 10000, about 900 to 7000, about 900 to 4000, about 1000 to 10000, about 1000 to 7000, about 1500 to 7000, about 2000 to 7000, or about 2500 to 7000 per molecule.

In one embodiment, poloxamer for improving the solubility can have Hydrophilic-Lipophilic Balance (HLB) of

about 0 to 30, about 1 to 25, about 1 to 20, about 1 to 15, about 1 to 10, about 5 to 30, about 5 to 25, about 5 to 20, about 5 to 15, about 10 to 30, about 10 to 25, about 10 to 20, about 15 to 30, about 15 to 25, or about 25 or greater. Colloids and Surfaces A: Physicochemical and Engineering Aspects, Volume 96, Issues 1-2, 10 March 1995, Pages 1-46 can be referred for HLB values.

In one embodiment, poloxamer for improving the solubility can be a molecule having the structure of formula I:

[Chemical Formula 3]

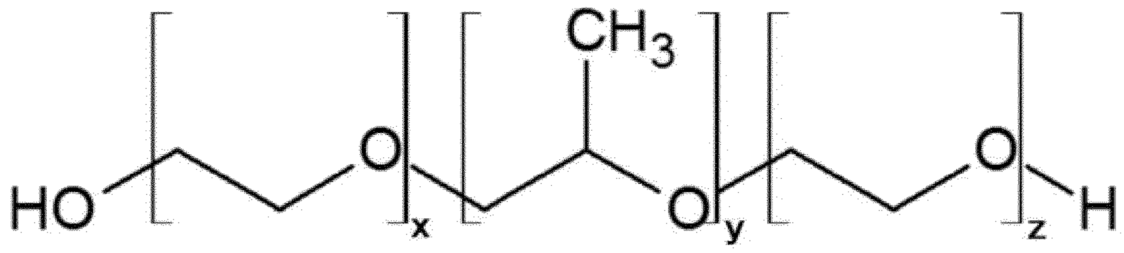

(formula I)

wherein x, y, and z are each independently selected integer that is 0 or greater, or a molecule modified therefrom. In one embodiment, x, y, and z can be x = 1 to 1000, y = 1 to 1000, and z = 1 to 1000. In one embodiment, x, y, and z can be x = 10 to 500, y = 10 to 500, and z = 10 to 500. In one embodiment, x, y, and z can be x = 50 to 200, y = 50 to 200, and z = 50 to 200. In one embodiment, poloxamer for improving the solubility can be a compound wherein the total molecular weight of partial structures represented by repeat units of $-CH_2-CH_2-O-$ and $-CH_2-CH\ (CH_3)\ -O-$ is 70% or greater, 75% or greater, 80% or greater, 85% or greater, 90% or greater, 95% or greater, 98% or greater, or 99% or greater with respect to the molecular weight of the entire compound.

In one embodiment, poloxamer for improving the solubility can be a compound having 1, 2, 3, 4, 5, 6, 7, 8, 9, 10, 11, 12, 13, 14, 15, 16, 17, 18, 19, 20, 21, 22, 23, 24, or 25 modifications, in any combination, selected from the group consisting of the following modifications in the structure of formula I described above:

*a branching modification which replaces one or more hydrogen atoms in a molecule with -(repeat unit of $-CH_2-CH_2-O-$ and/or $-CH_2-CH(CH_3)-O-$)-H;
*a modification which replaces one or more of repeat units of $-CH_2-CH_2-O-$ or $-CH_2-CH(CH_3)-O-$ independently with a group selected from the group consisting of $-R^2-O-$, $-R^2-S-$, $-R^2-$, and $-R^2-N(R^1)-$;
*a modification which replaces one or more -OH groups independently with a group selected from the group consisting of $-NR^1_2$, $-OR^1$, $-SR^1$, and $-R^1$;
*a modification which replaces one or more hydrogen atoms in a molecule with a halogen or a methyl group; and
*any combination of the modifications described above; wherein

$R^1$ is an optionally substituted $C_{1-10}$ aliphatic group, or a group wherein any of the carbon atoms in the $C_{1-10}$ aliphatic group is replaced with an optionally substituted 5- to 7-membered cyclic group, and
$R^2$ is an optionally substituted $C_{1-10}$ aliphatic group, or a group wherein any of the carbon atoms in the $C_{1-10}$ aliphatic group is replaced with an optionally substituted 5- to 7-membered cyclic group,
wherein "optionally substituted" means that one or more hydrogen atoms is, each independently, optionally replaced with a monovalent $C_{1-5}$ aliphatic group, halogen, -OH, -O-(monovalent $C_{1-5}$ aliphatic group), -COOH, -CO-(monovalent $C_{1-5}$ aliphatic group), $-CO-NH_2$, -CO-NH- (monovalent $C_{1-5}$ aliphatic group), -COH, -SH, -S-(monovalent $C_{1-5}$ aliphatic group), $-NH_2$, -NH-(monovalent $C_{1-5}$ aliphatic group), -N-(monovalent $C_{1-5}$ aliphatic group)$_2$, $-NO_2$, $-PO_3H$, $-SO_3H$, -CN, or monovalent 5- to 7-membered cyclic group.

In one embodiment, poloxamer for improving the solubility can be a compound comprising 1, 2, 3, 4, or 5 branching modifications which replace one or more hydrogen atoms in a molecule with -(repeat unit of $-CH_2-CH_2-O-$ and/or $-CH_2-CH(CH_3)-O-$)-H. In one embodiment, poloxamer for improving the solubility can be a propylene glycol compound from replacing one hydrogen atom of each $CH_3$ of 1, 2 or 3 - $CH_2-CH(CH_3)-O-$ repeat units with -OH or -(repeat unit of - $CH_2-CH_2-O-$ and/or $-CH_2-CH(CH_3)-O-$)-H in the structure of formula I described above.

In one embodiment, poloxamer for improving the solubility can be a compound comprising a modification which replaces 1 to 20, 1 to 15, 1 to 10, or 1 to 5, such as 1, 2, 3, 4, 5, 6, 7, 8, 9, 10, 11, 12, 13, 14, 15, 16, 17, 18, 19, or 20 of repeat units of $-CH_2-CH_2-O-$ or $-CH_2-CH(CH_3)-O-$ independently with a group selected from the group consisting of

$-R^2-O-$, $-R^2-S-$, $-R^2-$, and $-R^2-N(R^1)-$.

In one embodiment, poloxamer for improving the solubility can be a compound comprising a modification which replaces 1 to 20, 1 to 15, 1 to 10, or 1 to 5, such as 1, 2, 3, 4, or 5 -OH groups independently with a group selected from the group consisting of $-NR^1_2$, $-OR^1$, $-SR^1$, and $-R^1$.

In one embodiment, poloxamer for improving the solubility can be a compound comprising a modification which replaces 1 to 100, 1 to 70, 1 to 50, 1 to 30, 1 to 20, 1 to 15, 1 to 10, or 1 to 5, such as 1, 2, 3, 4, 5, 6, 7, 8, 9, 10, 11, 12, 13, 14, 15, 16, 17, 18, 19, or 20 hydrogen atoms in a molecule with a halogen or a methyl group.

In one embodiment, polypropylene glycol or polybutylene glycol for improving the solubility can be a compound represented by a similar average molecular weight, modification range, and HLB value as the poloxamer for improving the solubility described above.

In one embodiment, polyethylene glycol as a hydratable compound can have an average molecular weight of about 200 to 50000, about 500 to 20000, about 1000 to 15000, about 2000 to 10000, about 500 to 20000, about 500 to 10000, about 500 to 5000, about 1000 to 50000, about 1000 to 20000, about 1000 to 10000, about 1500 to 20000, or about 1500 to 15000. In one embodiment, the solubility improving composition of the present disclosure can comprise polyethylene glycol at about 1 to 40 wt%, about 1 to 40 wt%, about 2 to 30 wt%, about 5 to 25 wt%, about 10 to 30 wt%, about 1 to 20 wt%, about 2 to 20 wt%, about 5 to 20 wt%, about 15 to 30 wt%, or about 15 to 40 wt%. In one embodiment, polyethylene glycol as a hydratable compound can comprise PEG 200, PEG 300, PEG 400, PEG 600, PEG 1000, PEG 1500, PEG 2000, PEG 4000, PEG 6000, PEG 8000, PEG 10000, or PEG 20000. In one embodiment, polyethylene glycol as a hydratable compound can comprise PEG 4000.

In one embodiment, polyethylene glycol as a hydratable compound can be a molecule having the structure of formula II:

$$HO-(CH_2-CH_2-O)_n-H \qquad \text{(formula II)}$$

wherein n is an integer that is 0 or greater, or a molecule modified therefrom. In one embodiment, n can be n = 1 to 1000, n = 10 to 500, or n = 50 to 200. In one embodiment, polyethylene glycol as a hydratable compound can be a compound wherein the total molecular weight of partial structures represented by repeat units of $-CH_2-CH_2-O-$ is 70% or greater, 75% or greater, 80% or greater, 85% or greater, 90% or greater, 95% or greater, 98% or greater, or 99% or greater with respect to the molecular weight of the entire molecule.

In one embodiment, polyethylene glycol as a hydratable compound can be a compound having 1, 2, 3, 4, 5, 6, 7, 8, 9, 10, 11, 12, 13, 14, 15, 16, 17, 18, 19, 20, 21, 22, 23, 24, or 25 modifications, in any combination, selected from the group consisting of the following modifications in the structure of formula II described above:

*a branching modification which replaces one or more hydrogen atoms in a molecule with -(repeat unit of $-CH_2-CH_2-O-$)-H;
*a modification which replaces one or more of repeat units of $-CH_2-CH_2-O-$ independently with a group selected from the group consisting of $-R^2-O-$, $-R^2-S-$, $-R^2-$, and $-R^2-N(R^1)-$;
*a modification which replaces one or more -OH groups independently with a group selected from the group consisting of $-NR^1_2$, $-OR^1$, $-SR^1$, and $-R^1$;
*a modification which replaces one or more hydrogen atoms in a molecule with a halogen or a methyl group; and
*any combination of the modifications described above; wherein

$R^1$ is an optionally substituted $C_{1-10}$ aliphatic group, or a group wherein any of the carbon atoms in the $C_{1-10}$ aliphatic group is replaced with an optionally substituted 5- to 7-membered cyclic group, and
$R^2$ is an optionally substituted $C_{1-10}$ aliphatic group, or a group wherein any of the carbon atoms in the $C_{1-10}$ aliphatic group is replaced with an optionally substituted 5- to 7-membered cyclic group,

wherein "optionally substituted" means that one or more hydrogen atoms is, each independently, optionally substituted with a monovalent $C_{1-5}$ aliphatic group, halogen, -OH, -O-(monovalent $C_{1-5}$ aliphatic group), -COOH, -CO-(monovalent $C_{1-5}$ aliphatic group), $-CO-NH_2$, -CO-NH-(monovalent $C_{1-5}$ aliphatic group), -COH, -SH, -S-(monovalent $C_{1-5}$ aliphatic group), $-NH_2$, -NH- (monovalent $C_{1-5}$ aliphatic group), -N-(monovalent $C_{1-5}$ aliphatic group)$_2$, $-NO_2$, $-PO_3H$, - $SO_3H$, -CN, or monovalent 5- to 7-membered cyclic group.

In one embodiment, polyethylene glycol as a hydratable compound can be a compound comprising 1, 2, 3, 4, or 5 branching modifications which replace one or more hydrogen atoms in a molecule with -(repeat unit of $-CH_2-CH_2-O-$)-H.

In one embodiment, polyethylene glycol as a hydratable compound can be a compound comprising a modification which replaces 1 to 20, 1 to 15, 1 to 10, or 1 to 5, such as 1, 2, 3, 4, 5, 6, 7, 8, 9, 10, 11, 12, 13, 14, 15, 16, 17,

18, 19, or 20 of repeat units of $-CH_2-CH_2-O-$ independently with a group selected from the group consisting of $-R^2-O-$, $-R^2-S-$, $-R^2-$, and $-R^2-N(R^1)-$.

In one embodiment, polyethylene glycol as a hydratable compound can be a compound comprising a modification which replaces 1 to 20, 1 to 15, 1 to 10, or 1 to 5, such as 1, 2, 3, 4, or 5 -OH groups independently with a group selected from the group consisting of $-NR^1_2$, $-OR^1$, $-SR^1$, and $-R^1$.

In one embodiment, polyethylene glycol as a hydratable compound can be a compound comprising a modification which replaces 1 to 100, 1 to 70, 1 to 50, 1 to 30, 1 to 20, 1 to 15, 1 to 10, or 1 to 5, such as 1, 2, 3, 4, 5, 6, 7, 8, 9, 10, 11, 12, 13, 14, 15, 16, 17, 18, 19, or 20 hydrogen atoms in a molecule with a halogen or a methyl group.

In one embodiment, the solubility improving composition of the present disclosure can have viscosity of about 1 to 1000 mPa·s, about 1 to 500 mPa·s, about 1 to 200 mPa·s, about 1 to 100 mPa·s, about 1 to 50 mPa·s, about 1 to 20 mPa·s, about 1 to 10 mPa·s, about 2 to 1000 mPa·s, about 2 to 500 mPa·s, about 2 to 200 mPa·s, about 2 to 100 mPa·s, about 2 to 50 mPa·s, about 2 to 20 mPa·s, about 5 to 1000 mPa·s, about 5 to 500 mPa·s, about 5 to 200 mPa·s, about 5 to 100 mPa·s, about 5 to 50 mPa·s, about 10 to 1000 mPa·s, about 10 to 500 mPa·s, about 10 to 200 mPa·s, about 10 to 100 mPa·s, about 20 to 1000 mPa·s, about 20 to 500 mPa·s, about 20 to 200 mPa·s, about 50 to 1000 mPa·s, or about 50 to 500 mPa·s when measured with a rotational viscometer under conditions of 25°C and 100 rpm.

In one embodiment, the solubility improving composition of the present disclosure can have an osmotic pressure of about 0.01 to 1000 mOsm/kg, about 0.01 to 500 mOsm/kg, about 0.01 to 200 mOsm/kg, about 0.01 to 100 mOsm/kg, about 0.01 to 50 mOsm/kg, about 0.01 to 20 mOsm/kg, about 0.01 to 10 mOsm/kg, about 0.01 to 5 mOsm/kg, about 0.02 to 1000 mOsm/kg, about 0.02 to 500 mOsm/kg, about 0.02 to 200 mOsm/kg, about 0.02 to 100 mOsm/kg, about 0.02 to 50 mOsm/kg, about 0.02 to 20 mOsm/kg, about 0.02 to 10 mOsm/kg, about 0.05 to 1000 mOsm/kg, about 0.05 to 500 mOsm/kg, about 0.05 to 200 mOsm/kg, about 0.05 to 100 mOsm/kg, about 0.05 to 50 mOsm/kg, about 0.05 to 20 mOsm/kg, about 0.05 to 10 mOsm/kg, about 0.1 to 1000 mOsm/kg, about 0.1 to 500 mOsm/kg, about 0.1 to 200 mOsm/kg, about 0.1 to 100 mOsm/kg, about 0.1 to 50 mOsm/kg, about 0.1 to 20 mOsm/kg, about 0.1 to 10 mOsm/kg, about 0.2 to 1000 mOsm/kg, about 0.2 to 500 mOsm/kg, about 0.2 to 200 mOsm/kg, about 0.2 to 100 mOsm/kg, about 0.2 to 50 mOsm/kg, about 0.5 to 1000 mOsm/kg, about 0.5 to 500 mOsm/kg, about 0.5 to 200 mOsm/kg, about 0.5 to 100 mOsm/kg, about 0.5 to 50 mOsm/kg, about 0.5 to 20 mOsm/kg, about 1 to 1000 mOsm/kg, about 1 to 500 mOsm/kg, about 1 to 200 mOsm/kg, about 1 to 100 mOsm/kg, about 1 to 50 mOsm/kg, about 2 to 1000 mOsm/kg, about 2 to 500 mOsm/kg, about 2 to 200 mOsm/kg, about 2 to 100 mOsm/kg, about 2 to 50 mOsm/kg, about 5 to 1000 mOsm/kg, about 5 to 500 mOsm/kg, about 5 to 200 mOsm/kg, about 5 to 100 mOsm/kg, about 5 to 50 mOsm/kg, about 10 to 1000 mOsm/kg, about 10 to 500 mOsm/kg, about 10 to 200 mOsm/kg, about 10 to 100 mOsm/kg, about 20 to 1000 mOsm/kg, about 20 to 500 mOsm/kg, about 20 to 200 mOsm/kg, about 50 to 1000 mOsm/kg, about 50 to 500 mOsm/kg, or about 100 to 1000 mOsm/kg at 25°C. While it can be difficult to directly measure the osmotic pressure for the composition of the present disclosure that can comprise a polyether compound at a high concentration, the osmotic pressure can be determined by estimating the osmotic pressure of the original composition from results of measuring the osmotic pressure of a diluted composition, etc. When preparing a solution, the osmotic pressure can also be calculated from the amount of each solute.

In one embodiment, a poorly soluble compound can have LogP of about 0.2 to 7, about 0.3 to 4, about 0.4 to 4, about 0.5 to 4, about 0.7 to 4, about 1 to 4, about 1.5 to 4, about 0.5 to 7, about 0.5 to 6, about 0.5 to 5, about 0.5 to 3.5, about 0.5 to 3, or about 0.5 to 2.5. In one embodiment, LogP of a poorly soluble compound at the pH of the solubility improving composition of the present disclosure can be about 0.2 to 7, about 0.3 to 4, about 0.4 to 4, about 0.5 to 4, about 0.7 to 4, about 1 to 4, about 1.5 to 4, about 0.5 to 7, about 0.5 to 6, about 0.5 to 5, about 0.5 to 3.5, about 0.5 to 3, or about 0.5 to 2.5.

Poor solubility of a compound can be evaluated by LogP as described above, but this can also be evaluated by independently measuring the solubility into an aqueous solvent and non-aqueous solvent. In one embodiment, the poorly soluble compound of the present disclosure can be a compound which is poorly soluble in aqueous solvents, but has an improved solubility in non-aqueous solvents (ethanol, PEG 400, propylene glycol, etc.). Although it is preferable to avoid use of a non-aqueous solvent in a composition applied to animals such as humans, the solubility improving composition of the present disclosure that can use an aqueous solvent can also be suitably used for compounds of which solubility improves in a non-aqueous solvent. In one embodiment, a poorly soluble compound that can be suitably used in the solubility improving composition of the present disclosure can be a compound with about 10-fold or greater, about 20-fold or greater, about 50-fold or greater, about 70-fold or greater, about 100-fold or greater, about 150-fold or greater, about 200-fold or greater, about 300-fold or greater, about 400-fold or greater, about 500-fold or greater, about 700-fold or greater, or about 1000-fold or greater improvement in solubility at 25°C in ethanol, PEG 400, and/or propylene glycol compared to solubility at 25°C in buffer adjusted to a pH of 7.0 (e.g., aqueous solution of 0.2% sodium hydrogen phosphate hydrate and 0.3% sodium dihydrogen phosphate hydrate). It is understood that a compound with 100-fold or greater improvement in solubility has an especially large effect.

In one embodiment, a poorly soluble compound can be characterized by one or more structural features among the following features:

(1) is free of a readily ionizable group (acidic group (carboxyl group, tetrazole group, sulfuric acid group, phosphoric acid group, etc.), basic group (amine group, etc.), etc.) or an ionic group thereof;
(2) has fluorine (optionally, free of a hydroxyl group or amino group at a vicinal position of fluorine);
(3) is free of a hydroxyl group; and
(4) comprises 2, 3, 4, 5, 6, 7, 8, 9, 10, or more rings (e.g., aromatic ring) (wherein fused rings or spiro rings are not counted as one ring as a whole, but instead the number of closed rings are tallied).

In one embodiment, a poorly soluble compound has a pyridyl substituted imidazole structure and/or about 5 rings (e.g., aromatic rings).

In this aspect of the present disclosure, any poorly soluble compound may be used, as long as the compound comprises the feature described above. The poorly soluble compound used may comprise any combination of the features described above, such as a combination of the feature of the ratio of solubility into a non-aqueous solvent relative to an aqueous solvent and another feature described above. A poorly soluble compound may be, for example, a pharmaceutical component or an active ingredient, or something else. Examples thereof include, but are not limited to, docetaxel, paclitaxel, capecitabine, oxaliplatin, geftinat, doxorubicin, irinotecan, gemcitabine, pemetrexed, temozolomide, imatinib, vinorelbin, letrozole, teniposide, etoposide, podophyllotoxin, camptothecin, topotecan, vinblastine, vincristine, vindesine, vinflunine, vinpocetine, norcantharidin, silybin, propofol, florfenicol, mitiglinide, artemisinin, dihydroartemisinin, tacrolimus, sirolimus, ibuprofen, nitrendipine, nicardipine, nimodipine, gliclazide, propulsid, nifedipine, felodipine, glibenclamide, acyclovir, oleanolic acid, breviscapine, ferulic acid, paracetamol, palmitoyl rhizoxin, penclomedine, vitamin A, tamoxifen, navelbine, valproic acid, cyclosporine A, amphotericin B, ketoconazole, domperidone, sulpiride, fenofibrate, bezafibrate, itraconazole, miconazole, latanoprost, brinzolamide, erythromycin, roxithromycin, rifaximin, cisapride, diclofenamic acid, terphenazine, theophylline, ketoprofen, furosemide, spironolactone, dipyridamole, piroxicam, mefenamic acid, trichlorothiazide, pindolol, acemetacin, indomethacin, diflunisal, naproxen, triamcinolone, triamcinolone acetonide, fluorometholone, dexamethasone, hydrocortisone, prednisolone, cisplatin, enoxacin, norfloxacin, clofibrate, probucol, simvastatin, reserpine, verapamil hydrochloride, atenolol, digitoxin, digoxin, diazepam, nitrazepam, haloperidol, droperidol, triazolam, phenytoin, phenobarbital, cimetidine, famotidine, omeprazole, lansoprazole, oxethazaine, sucralfate, gefarnate, metoclopramide, tranilast, tolbutamide, mebendazole, albendazole, fenbendazole, cilostazol, phenylbutazone, and celecoxib.

In one embodiment, the solubility improving composition of the present disclosure can comprise cyclodextrin or a water soluble macromolecule. Examples of cyclodextrin include α-cyclodextrin, hydroxypropyl-α-cyclodextrin, β-cyclodextrin, hydroxypropyl-β-cyclodextrin, methyl-β-cyclodextrin, sulfobutylether-β-cyclodextrin, γ-cyclodextrin, hydroxypropyl-γ-cyclodextrin, etc. Examples of water soluble macromolecules include methyl cellulose, ethyl cellulose, hydroxypropyl methylcellulose, hydroxyethyl cellulose, hydroxypropyl methylcellulose acetate succinate, hydroxypropyl methylcellulose phthalate, carboxymethyl cellulose, xanthan gum, carrageenan, gum arabic, locust bean gum, gellan gum, tamarind gum, alginic acid, hyaluronic acid, chondroitin sulfate, carboxyvinyl polymer, polyvinyl alcohol (PVA), polyvinylpyrrolidone (PVP), etc. In one embodiment, the solubility improving composition of the present disclosure can comprise at least one of sulfobutylether-β-cyclodextrin and carboxymethyl cellulose. In one embodiment, addition of such cyclodextrin or a water-soluble macromolecule can further improve the solubility of a poorly soluble compound. In one embodiment, the solubility improving composition of the present disclosure can comprise the cyclodextrin or water-soluble macromolecule at about 0.001 to 20 wt%, about 0.001 to 10 wt%, about 0.005 to 20 wt%, about 0.005 to 10 wt%, about 0.005 to 5 wt%, about 0.01 to 20 wt%, about 0.01 to 10 wt%, about 0.01 to 5 wt%, about 0.02 to 20 wt%, about 0.02 to 10 wt%, about 0.02 to 5 wt%, about 0.05 to 20 wt%, about 0.05 to 10 wt%, about 0.05 to 5 wt%, about 0.001 to 5 wt%, about 0.005 to 2 wt%, about 0.01 to 1 wt%, about 0.02 to 0.5 wt%, about 0.05 to 0.2 wt%, about 0.01 to 5 wt%, about 0.01 to 0.5 wt%, about 0.01 to 0.2 wt%, about 0.01 to 0.1 wt%, about 0.02 to 1 wt%, about 0.02 to 0.5 wt%, about 0.02 to 0.2 wt%, about 0.05 to 2 wt%, about 0.05 to 1 wt%, about 0.05 to 0.5 wt%, about 0.1 to 5 wt%, about 0.1 to 2 wt%, or about 0.1 to 1 wt%.

In one embodiment, the improvement in solubility of a poorly soluble compound by adding a hydratable compound (e.g., 20 wt%) to a hydrophobic polyoxy aliphatic moiety-containing polyether compound (e.g., 10 wt%) under conditions of an atmospheric pressure of 1 atm at a predetermined temperature (e.g., 25°C, 20°C, 15°C, 10°C, 5°C, etc.) in the solubility improving composition of the present disclosure can be 1.05-fold or greater, 1.1-fold or greater, 1.2-fold or greater, 1.5-fold or greater, 1.7-fold or greater, 2-fold or greater, 3-fold or greater, 4-fold or greater, 5-fold or greater, 7-fold or greater, 10-fold or greater, 15-fold or greater, 20-fold or greater, 30-fold or greater, 40-fold or greater, 50-fold or greater, 70-fold or greater, or 100-fold or greater. As used herein, "improvement in solubility of a poorly soluble compound by adding a hydratable compound to a hydrophobic polyoxy aliphatic moiety-containing polyether compound" of a composition refers to a ratio comparing a value computed by the solubility of a composition comprising a hydratable compound and a hydrophobic polyoxy aliphatic moiety-containing polyether compound minus the solubility of the same composition other than not comprising the hydrophobic polyoxy aliphatic moiety-containing polyether compound, i.e., improvement in solubility by combining a hydratable compound with a hydrophobic polyoxy aliphatic moiety-containing

polyether compound ($S_{A+B}$ - $S_B$), with a value computed by the solubility of the same composition other than not comprising the hydratable compound minus the solubility of a composition that comprises neither a hydrophobic polyoxy aliphatic moiety-containing polyether compound nor a hydratable compound, i.e., solubility due to a hydrophobic polyoxy aliphatic moiety-containing polyether compound. This is calculated by the following formula ($S_A$ -So) :

$$\{S_{A+B} - S_B\}/\{S_A - S_0\}$$

wherein

$S_{A+B}$ = solubility of a poorly soluble compound in a composition comprising a hydrophobic polyoxy aliphatic moiety-containing polyether compound and a hydratable compound
$S_B$ = solubility of a poorly soluble compound in a composition comprising a hydratable compound, but not a hydrophobic polyoxy aliphatic moiety-containing polyether compound
$S_A$ = solubility of a poorly soluble compound in a composition comprising a hydrophobic polyoxy aliphatic moiety-containing polyether compound, but not a hydratable compound
$S_0$ = solubility of a poorly soluble compound in a composition that comprises neither a hydrophobic polyoxy aliphatic moiety-containing polyether compound nor a hydratable compound,

wherein each composition comprises the same components other than a hydrophobic polyoxy aliphatic moiety-containing polyether compound and a hydratable compound at the same concentration, and all measurement conditions other than the components of the compositions (temperature, pressure, etc.) are the same.

In one embodiment, improvement in solubility of a poorly soluble compound by adding a hydratable compound (e.g., 20 wt%) to a hydrophobic polyoxy aliphatic moiety-containing polyether compound (e.g., 10 wt%) under conditions of an atmospheric pressure of 1 atm at 25°C in the solubility improving composition of the present disclosure can be 1.05-fold or greater, 1.1-fold or greater, 1.2-fold or greater, 1.5-fold or greater, 1.7-fold or greater, or 2-fold or greater.

In one embodiment, improvement in solubility of a poorly soluble compound by adding a hydratable compound (e.g., 20 wt%) to a hydrophobic polyoxy aliphatic moiety-containing polyether compound (e.g., 10 wt%) under conditions of an atmospheric pressure of 1 atm at 15°C in the solubility improving composition of the present disclosure can be 1.5-fold or greater, 1.7-fold or greater, 2-fold or greater, 3-fold or greater, 4-fold or greater, 5-fold or greater, 7-fold or greater, 10-fold or greater, 15-fold or greater, or 20-fold or greater.

In one embodiment, the improvement in solubility of a poorly soluble compound by adding a hydratable compound (e.g., 20 wt%) to a hydrophobic polyoxy aliphatic moiety-containing polyether compound (e.g., 10 wt%) under conditions of an atmospheric pressure of 1 atm at 5°C in the solubility improving composition of the present disclosure can be 1.5-fold or greater, 1.7-fold or greater, 2-fold or greater, 3-fold or greater, 4-fold or greater, 5-fold or greater, 7-fold or greater, 10-fold or greater, 15-fold or greater, 20-fold or greater, 30-fold or greater, or 40-fold or greater. In one embodiment, a poorly soluble compound used in combination with the solubility improving composition of the present disclosure is not a compound of which solubility improves significantly in the presence of only either a hydrophobic polyoxy aliphatic moiety-containing polyether compound (e.g., poloxamer) (e.g., 10 wt%) or a hydratable compound (e.g., polyethylene glycol) (e.g., 20 wt%) (e.g., under conditions of an atmospheric pressure of 1 atm at 5°C), e.g., the solubility does not improve 5-fold or greater, 10-fold or greater, 20-fold or greater, 50-fold or greater, etc. compared to the solubility in a composition that comprises neither a hydrophobic polyoxy aliphatic moiety-containing polyether compound nor a hydratable compound.

For example, storage conditions of drugs are specified to be 15°C to 25°C, etc. in some cases. Since the use thereof presumes a temperature within said storage temperature, precipitation and/or deposition of a poorly soluble compound is preferably not produced under a low temperature environment of 15°C, etc. For this reason, the solubility improving composition of the present disclosure, which has a particularly excellent effect of improving the solubility of a poorly soluble compound at low temperatures, can be preferably used in any drugs that can be stored under a low temperature environment. Drugs are generally conditional on guaranteeing the quality, safety, and efficacy in the temperature range specified in the package insert. For quality, a specification is determined in accordance with the regulation. For eye drops, foreign objects are controlled by an insoluble particulate matter test in the US and insoluble particulate matter test and insoluble foreign matter test in Japan. If a drug precipitates due to lack of solubility, it is understood that numerous foreign objects with a size of several $\mu$m to several hundred $\mu$m are produced, which would not be within the specification. If a foreign object is found during storage (within the temperature range of storage conditions) of a drug which is specified as "colorless and clear" from checking the attribute, the drug would no longer be clear, so that the drug would not be within the specification. For efficacy, if a drug precipitates, the dissolved drug concentration would decrease and the amount of intraocular migration would be lower than expected, so that sufficient treatment may not be achieved. For safety, if a foreign object with a size of several hundred $\mu$m is produced, a cornea can be damaged.

In this manner, the solubility improving composition of the present disclosure generally can be beneficial in drugs that can be exposed to various temperature environments including lowing temperatures during storage, etc. The solubility improving composition of the present disclosure can provide excellent quality stability to not only drugs, but also any product that can be exposed to a low temperature environment.

(Other components)

The composition of the present disclosure can be provided in various forms. In one embodiment, the composition of the present disclosure can be an aqueous formulation. In one embodiment, the ratio of water in the composition of the present disclosure can be about 30 wt% or greater, about 35 wt% or greater, about 40 wt% or greater, about 45 wt% or greater, about 50 wt% or greater, about 55 wt% or greater, about 60 wt% or greater, about 65 wt% or greater, about 70 wt% or greater, about 75 wt% or greater, about 80 wt% or greater, about 85 wt% or greater, about 90 wt% or greater, or about 95 wt% or greater. In one embodiment, the composition of the present disclosure may comprise methanol, ethanol, propanol, propylene glycol, ethyl acetate, oil (castor oil, peanut oil, soybean oil, mineral oil, sesame oil, olive oil, rapeseed oil, etc.), etc. as a carrier other than water. The pH of any composition of the present disclosure can be about 3, about 3.5, about 4, about 4.5, about 5, about 5.5, about 6, about 6.5, about 7, about 7.5, about 8, about 8.5, about 9, about 9.5, about 10, about 10.5, about 11, or a range between any two of said values.

Any component of the composition of the present disclosure can be provided as a pharmaceutically acceptable salt. Examples thereof include salts formed with a free carboxyl group derived from hydrochloric acid, phosphoric acid, acetic acid, oxalic acid, tartaric acid, etc., salts formed with a free amine group such as those derived from isopropylamine, triethylamine, 2-ethylaminoethanol, histidine, procaine, etc., and salts formed with sodium, potassium, ammonium, calcium, or ferric hydroxide.

The composition of the present disclosure may contain any additive. Examples of additives include a thickener, solubilizing agent, suspending agent, surfactant, tonicity agent, buffer, analgesic, stabilizer, preservative, antioxidant, pH modulator, diluent, adjuvant, colorant, flavor, etc.

In one embodiment, a thickener can be a polysaccharide, cellulose macromolecule, synthetic macromolecule, etc. In one embodiment, a polysaccharide can be alginic acid, chondroitin sulfate, hyaluronic acid, xanthan gum, etc. In one embodiment, a cellulose macromolecule can be nonionic cellulose, anionic cellulose, etc. Examples of nonionic cellulose include methyl cellulose, ethyl cellulose, hydroxymethyl cellulose, hydroxyethyl cellulose, hydroxypropyl cellulose, hydroxyethyl methyl cellulose, hydroxypropyl methylcellulose, etc. Examples of anionic cellulose include carboxymethyl cellulose, hydroxypropyl methyl cellulose acetate succinate, hydroxypropyl methylcellulose phthalate, carboxymethyl ethyl cellulose, cellulose acetate phthalate, etc. In one embodiment, a synthetic macromolecule can be carboxyvinyl polymer, polyacrylic acid, polyvinyl pyrrolidone, polyvinyl alcohol, etc.

In one embodiment, a surfactant can be a cationic surfactant, anionic surfactant, nonionic surfactant, etc. In one embodiment, a cationic surfactant can be an alkylamine salt, alkylamine polyoxyethylene adduct, fatty acid triethanolamine monoester salt, acylaminoethyl diethylamine salt, fatty acid polyamine condensate, alkylimidazoline, 1-acylaminoethyl-2-alkylimidazoline, 1-hydroxyethyl-2-alkylimidazoline, etc. In one embodiment, an anionic surfactant can be a phospholipid such as lecithin. In one embodiment, a nonionic surfactant can be a polyoxyethylene fatty acid ester such as polyoxyl 40 stearate, polyoxyethylene sorbitan fatty acid ester such as polysorbate 80, polysorbate 60, polysorbate 40, polyoxyethylene sorbitan monolaurate, polyoxyethylene sorbitan trioleate, or polysorbate 65, polyoxyethylene hydrogenated castor oil such as polyoxyethylene (10) hydrogenated castor oil, polyoxyethylene (40) hydrogenated castor oil, polyoxyethylene (50) hydrogenated castor oil, or polyoxyethylene (60) hydrogenated castor oil, polyoxyl castor oil such as polyoxyl 5 castor oil, polyoxyl 9 castor oil, polyoxyl 15 castor oil, polyoxyl 35 castor oil, or polyoxyl 40 castor oil, a sucrose fatty acid ester such as sucrose stearate, tocopherol polyethylene glycol 1000 succinate (vitamin E TPGS), etc.

In one embodiment, a buffer can be boric acid, borax, phosphoric acid, carbonic acid, organic acid, salt thereof, etc. In one embodiment, an organic acid can be citric acid, acetic acid, $\varepsilon$-aminocaproic acid, gluconic acid, fumaric acid, lactic acid, ascorbic acid, succinic acid, maleic acid, malic acid, or amino acid.

In one embodiment, a preservative can be benzalkonium chloride, benzalkonium bromide, benzethonium chloride, chlorhexidine, paraoxybenzoate, parahydroxybenzoate, chlorobutanol, benzylalcohol, phenethyl alcohol, dehydroacetic acid, sorbic acid, sodium chlorite, etc.

In one embodiment, an antioxidant can be sulfite, ascorbic acid, dibutylhydroxytoluene, $\alpha$-tocopherol, etc.

In one embodiment, tonicity agent can be sodium chloride, potassium chloride, calcium chloride, magnesium chloride, glycerol, propylene glycol, sorbitol, mannitol, trehalose, maltose, sucrose, xylitol, etc. In one embodiment, an analgesic can be benzyl alcohol, etc.

In one embodiment, a suspending agent can be a surfactant such as stearyl triethanolamine, sodium lauryl sulfate, laurylaminopropionic acid, lecithin, benzalkonium chloride, benzethonium chloride, or glyceryl monostearate, hydrophilic macromolecule such as polyvinyl alcohol, polyvinylpyrrolidone, carboxymethyl cellulose sodium, methyl cellulose, hydroxymethyl cellulose, hydroxyethyl cellulose, or hydroxypropyl cellulose, etc.

(Use/Application)

The composition of the present disclosure can be used in any application that utilizes the effect of improving the stability of a polyether compound and/or improving the solubility of a poorly soluble compound of the present disclosure. In one embodiment, the composition of the present disclosure can be used to improve the solubility of a drug comprising the hydrophobic polyoxy aliphatic moiety-containing polyether compound and/or hydratable compound of the present disclosure and poorly soluble compound. In one embodiment, the composition of the present disclosure can be administered through intraocular, ocular surface, intravenous, topical, intramuscular, subcutaneous, intradermal, transdermal, rectal, transvaginal, oral mucosal membrane, pulmonary mucosal membrane, transnasal, transocular, or oral (enteric) route, etc.

In one embodiment, the composition of the present disclosure can be a liquid agent or a suspension. In one embodiment, the composition of the present disclosure can be an orally administered agent, a topical agent, or an injection agent. In one embodiment, the composition of the present disclosure can be an eye drop, injection agent, bolus injection, intravenous drip, oral agent, inhalation, or aerosol. Examples of the route of administration, if the composition of the present disclosure is an injection agent, include, but are not limited to, intravenous administration, intramuscular administration, subcutaneous administration, intravitreal administration, etc. Examples of the route of administration, if the composition of the present disclosure is a topical agent, include, but are not limited to, dermal administration, intranasal administration, eye ball administration, mucosal administration, rectal administration, inhalation administration, etc.

In one embodiment, the present disclosure can be useful for evaluating the efficacy of a poorly soluble compound. Evaluation of the efficacy (e.g., clinical efficacy) of a poorly soluble compound can be simplified or expedited by dissolving the poorly soluble compound at a high concentration. In one embodiment, the present disclosure can avoid the use of another solubilizing agent and eliminate the need to consider development of a suspension for a poorly soluble compound that does not dissolve with a normal solubilizing agent.

When the composition of the present disclosure is applied to a subject, the subject is not particularly limited. The subject can be a mammal (e.g., mouse, rat, hamster, rabbit, cat, dog, cow, sheep, pig, monkey, human, etc.), avian, reptile, amphibian, arthropod, fish, etc.

When the composition of the present disclosure is used as a therapeutic agent, prophylactic agent, or detection agent, the amount of the therapeutic agent of the present disclosure that is effective in the treatment of a disorder or condition specific to the active ingredient (poorly soluble compound, etc.) can vary depending on the nature of the disorder or condition, but can be determined by those skilled in the art through standard clinical technologies based on the description herein. In some cases, the composition can assist in identifying the optical range of dosage by using an in vitro assay. Since the accurate dose to be used in a formulation can also vary depending on the route of administration and the severity of a disease or disorder, the dose should be determined in accordance with the judgment of the attending physician or status of each patient. While the dosage is not particularly limited, the dosage may be, for example, 0.00001, 0.0001, 0.001, 1, 5, 10, 15, 100, or 1000 mg/kg body weight per dose or a value within a range of any two of said values. While the dosing interval is not particularly limited, the interval may be, for example, once or twice every 1, 7, 14, 21, or 28 days, or in a range of any two of said values. The dosage, dosing interval, and dosing method may be appropriately selected depending on the patient's agent, body weight, symptom, target organ, etc.

The composition of the present disclosure can be provided as a kit. In one embodiment, the present disclosure provides a drug pack or kit comprising one or more containers filled with one or more components that can be added to the composition of the present disclosure. Optionally, information indicating approval for manufacture, use, or sale for human administration by a government agency regulating the manufacture, use, or sale of drugs or biological products can be displayed on such containers in a form specified by the government agency.

A procedure of formulation of the therapeutic agent, prophylactic agent, etc. of the present disclosure as a drug is known in the art and is described, for example, in the Japanese Pharmacopoeia, U.S. Pharmacopoeia, and other countries' pharmacopoeias. Thus, those skilled in the art can determine the embodiment such as the amount to be used, etc. from the descriptions herein without undue experimentation.

In one embodiment, the kit of the present disclosure comprises an instruction manual for use of the kit of the present disclosure. Examples of a suitable container include a bottle, vial (e.g., dual-chamber vial), syringe (dual-chamber syringe, etc.), test tube, multi-dose container, disposable unit-dose container, and PFMD (Preservative Free Multi Dose) container. Such a container can be made of various materials such as glass or plastic.

As used herein, "or" is used when "at least one or more" of the listed matters in the sentence can be employed. When explicitly described herein as "within the range" of "two values", the range also includes the two values themselves.

Reference literatures such as scientific literatures, patents, and patent applications cited herein are incorporated herein by reference to the same extent that the entirety of each document is specifically described.

As described above, the present disclosure has been described while showing preferred embodiments to facilitate understanding. The present disclosure is described hereinafter based on Examples. The above descriptions and the following Examples are not provided to limit the present disclosure, but for the sole purpose of exemplification.

Thus, the scope of the present disclosure is not limited to the embodiments and Examples specifically described herein and is limited only by the scope of claims.

[Examples]

For reagents, the specific products described in the Examples were used. However, the reagents can be substituted with an equivalent product from another manufacturer (Sigma-Aldrich, Fujifilm Wako Pure Chemical, Nacalai Tesque, R & D Systems, USCN Life Science Inc., etc.)

(Example 1) Stability Test

Polyether compounds such as poloxamer and polyethylene glycol can be degraded by light, an oxygen radical, etc., which can result in a decreased pH or viscosity in a solution. For this reason, a stabilizer that can be suitably combined with such a polyether compound was tested.

An additional component was added to an aqueous solution comprising poloxamer and/or polyethylene glycol, and the change in the viscosity and pH was measured over time under harsh conditions of 60°C. The following were used as the poloxamer, polyethylene glycol, and buffer.

*Poloxamer 407 (Kolliphor P 407, BASF, Germany)
*PEG 4000 (Macrogol 4000, NOF, Tokyo)
*Buffer: 0.9% boric acid, 0.1% borax

Specifically, formulations of interest were prepared by measuring and placing a buffer, PEG, poloxamer, and additive in a preparation vessel, and adding purified water. The pH was adjusted with hydrochloric acid or sodium hydroxide. Each prepared solution was filtered through a filter with a pore size of 0.45 $\mu$m (Merck, Germany) or a filter with a pore size of 5 $\mu$m (GE Healthcare, US). A polyethylene eye drop bottle was filled with 5 mL of each filtrate and stored in a 60°C thermostatic vessel. The pH and viscosity of the test solutions as of preparation and at four weeks after storage were measured. The viscosity was measured using a rotational viscometer (Toki Sangyo (Osaka) Model TVE-25 viscometer type L, 25°C, 100 rpm). The pH as of the preparation was measured in the test solution before transferring to an eye drop bottle for storage.

The following tables show the results of studying the change in pH by adding an additive specified in the following tables to the base composition of 25% PEG 4000 only, 10% poloxamer 407 only, and 25% PEG 4000 + 10% poloxamer 407.

[Table 1-1]

| **Base composition: 25% PEG 4000** | | | | |
|---|---|---|---|---|
| **Additive** | **Amount added** | **Sample No.** | **At preparation** | **After four weeks** |
| | | **A01** | **7.52** | **2.68** |
| **Mannitol** | **0.1%** | **A03** | **7.49** | **2.85** |
| **Sodium sulfite** | **0.1%** | **A04** | **7.48** | **2.63** |
| **Sodium pyrosulfite** | **0.1%** | **A05** | **7.54** | **2.60** |
| **Sodium thiosulfate hydrate** | **0.01%** | **A06** | **7.53** | **7.48** |
| | **0.03%** | **A07** | **7.54** | **7.52** |
| | **0.1%** | **A08** | **7.52** | **7.53** |
| | **0.3%** | **A09** | **7.53** | **7.52** |
| | **1%** | **A10** | **7.54** | **7.48** |

(continued)

| Base composition: 25% PEG 4000 | | | | |
|---|---|---|---|---|
| **Additive** | **Amount added** | **Sample No.** | **At preparation** | **After four weeks** |
| **EDTA** | **0.01%** | **A11** | **7.52** | **2.85** |
| | **0.03%** | **A12** | **7.54** | **3.11** |
| | **0.1%** | **A13** | **7.53** | **6.91** |
| | **0.3%** | **A14** | **7.52** | **7.46** |
| | **1%** | **A15** | **7.54** | **7.53** |
| **Sodium thiosulfate hydrate<br>Mannitol** | **0.01%<br>0.1%** | **A 21** | **7.54** | **7.49** |
| **Sodium thiosulfate hydrate<br>Sodium sulfite** | **0.01%<br>0.1%** | **A22** | **7.50** | **2.55** |

[Table 1-2]

| | | | | |
|---|---|---|---|---|
| **Sodium thiosulfate hydrate<br>Sodium pyrosulfite** | **0.01%<br>0.1%** | **A23** | **7.50** | **2.54** |
| **Sodium thiosulfate hydrate<br>EDTA** | **0.01%<br>0.01%** | **A24** | **7.53** | **7.45** |
| **EDTA<br>Mannitol** | **0.01 %<br>0.1%** | **A29** | **7.53** | **3.10** |
| **EDTA<br>Sodium sulfite** | **0.01%<br>0.1%** | **A30** | **7.51** | **2.61** |
| **EDTA<br>Sodium pyrosulfite** | **0.01%<br>0.1%** | **A31** | **7.53** | **2.59** |

[Table 2-1]

| Base composition: 10% poloxamer 407 | | | | |
|---|---|---|---|---|
| **Additive** | **Amount added** | **Sample No.** | **At preparation** | **After four weeks** |
| | | **B01** | **7.51** | **2.99** |
| **Dibutylhydroxytoluene** | **0.005%** | **B02** | **7.53** | **7.37** |
| **Mannitol** | **0.1%** | **B03** | **7.51** | **3.13** |
| **Sodium sulfite** | **0.1%** | **B04** | **7.54** | **2.76** |
| **Sodium pyrosulfite** | **0.1%** | **B05** | **7.52** | **2.70** |
| **Sodium thiosulfate hydrate** | **0.01 %** | **B06** | **7.53** | **2.86** |
| | **0.03%** | **B07** | **7.52** | **7.40** |
| | **0.1%** | **B08** | **7.53** | **7.34** |
| | **0.3%** | **B09** | **7.52** | **7.33** |
| | **1%** | **B10** | **7.54** | **7.37** |

(continued)

| Base composition: 10% poloxamer 407 | | | | |
|---|---|---|---|---|
| **Additive** | **Amount added** | **Sample No.** | **At preparation** | **After four weeks** |
| **EDTA** | **0.01%** | **B11** | **7.54** | **7.31** |
| | **0.03%** | **B12** | **7.52** | **7.31** |
| | **0.1%** | **B13** | **7.53** | **7.39** |
| | **0.3%** | **B14** | **7.54** | **7.39** |
| | **1%** | **B15** | **7.52** | **7.39** |
| **Dibutylhydroxytoluene** | **0.003%** | **B16** | **7.54** | **7.31** |
| **Sodium thiosulfate hydrate**<br>**Dibutylhydroxytoluene** | **0.01%**<br>**0.003%** | **B19** | **7.53** | **7.36** |
| **Sodium thiosulfate hydrate**<br>**Dibutylhydroxytoluene** | **0.01 %**<br>**0.005%** | **B20** | **7.52** | **7.32** |

[Table 2-2]

| | | | | |
|---|---|---|---|---|
| **Sodium thiosulfate hydrate**<br>**Mannitol** | **0.01%**<br>**0.1%** | **B21** | **7.51** | **7.24** |
| **Sodium thiosulfate hydrate**<br>**Sodium sulfite** | **0,01%**<br>**0.1%** | **B22** | **7.54** | **2.64** |
| **Sodium thiosulfate hydrate**<br>**Sodium pyrosulfite** | **0.01%**<br>**0.1%** | **B23** | **7.52** | **2.62** |
| **Sodium thiosulfate hydrate**<br>**EDTA** | **0.01%**<br>**0.01%** | **B24** | **7.54** | **7.35** |
| **EDTA**<br>**Dibutylhydroxytoluene** | **0.01%**<br>**0.003%** | **B27** | **7.54** | **7.38** |
| **EDTA**<br>**Dibutylhydroxytoluene** | **0.01%**<br>**0.005%** | **B28** | **7.54** | **7.38** |
| **EDTA**<br>**Mannitol** | **0.01%**<br>**0.1%** | **B29** | **7.54** | **7.39** |
| **EDTA**<br>**Sodium sulfite** | **0.01%**<br>**0.1%** | **B30** | **7.53** | **2.71** |
| **EDTA**<br>**Sodium pyrosulfite** | **0.01%**<br>**0.1%** | **B31** | **7.53** | **2.69** |

[Table 3-1]

| **Base composition: 25% PEG 4000 + 10% poloxamer 407** | | | | |
|---|---|---|---|---|
| **Additive** | **Amount added** | **Sample No.** | **At preparation** | **After four weeks** |
| | | **C01** | **7.54** | **3.19** |
| **Dibutylhydroxytoluene** | **0.005%** | **C02** | **7.52** | **4.99** |
| **Mannitol** | **0.1%** | **C03** | **7.51** | **3.29** |
| **Sodium sulfite** | **0.1%** | **C04** | **7.54** | **2.84** |
| **Sodium pyrosulfite** | **0.1%** | **C05** | **7.53** | **2.77** |

(continued)

| Base composition: 25% PEG 4000 + 10% poloxamer 407 | | | | |
|---|---|---|---|---|
| **Additive** | **Amount added** | **Sample No.** | **At preparation** | **After four weeks** |
| Sodium thiosulfate hydrate | 0.01% | C06 | 7.52 | 4.69 |
| | 0.03% | C07 | 7.52 | 7.33 |
| | 0.1% | C08 | 7.54 | 7.46 |
| | 0.3% | C09 | 7.54 | 7.38 |
| | 1% | C10 | 7.53 | 7.31 |
| EDTA | 0.01% | C11 | 7.52 | 4.57 |
| | 0.03% | C12 | 7.54 | 5.54 |
| | 0.1% | C13 | 7.53 | 7.20 |
| | 0.3% | C14 | 7.54 | 7.38 |
| | 1% | C15 | 7.53 | 7.44 |
| Dibutylhydroxytoluene | 0.003% | C16 | 7.50 | 3.59 |
| Sodium thiosulfate hydrate<br>Dibutylhydroxytoluene | 0.01%<br>0.003% | C19 | 7.51 | 7.35 |
| Sodium thiosulfate hydrate<br>Dibutylhydroxytoluene | 0.01%<br>0.005% | C20 | 7.52 | 7.35 |
| Sodium thiosulfate hydrate | 0.01% | C21 | 7.50 | 7.31 |

[Table 3-2]

| | | | | |
|---|---|---|---|---|
| Mannitol | 0.1% | | | |
| Sodium thiosulfate hydrate<br>Sodium sulfite | 0.01%<br>0.1% | C22 | 7.51 | 2.81 |
| Sodium thiosulfate hydrate<br>Sodium pyrosulfite | 0.01 %<br>0.1% | C23 | 7.54 | 2.78 |
| Sodium thiosulfate hydrate<br>EDTA | 0.01%<br>0.01% | C24 | 7.51 | 4.24 |
| EDTA<br>Dibutylhyd roxytoluene | 0.01 %<br>0.003% | C27 | 7.51 | 6.98 |
| EDTA<br>Dibutylhydroxytoluene | 0.01%<br>0.005% | C28 | 7.50 | 7.06 |
| EDTA<br>Mannitol | 0.01%<br>0.1% | C29 | 7.54 | 5.10 |
| EDTA<br>Sodium sulfite | 0.01%<br>0.1% | C30 | 7.54 | 2.89 |
| EDTA<br>Sodium pyrosulfite | 0.01%<br>0.1% | C31 | 7.50 | 2.81 |

Sodium thiosulfate was able to stabilize PEG 4000 even at a low concentration of 0.01%, and stabilize poloxamer 407 at a low concentration of 0.03%. Dibutylhydroxytoluene (BHT) alone was able to stabilize poloxamer 407. When sodium thiosulfate was combined with dibutylhydroxytoluene (BHT), poloxamer 407 (and PEG 4000) was able to be stabilized even with a low concentration of sodium thiosulfate. EDTA was able to stabilize poloxamer 407 even at a low

concentration of 0.01%, but a concentration of 0.1% or greater was required for stabilizing PEG 4000. Mannitol, sodium sulfite, and sodium pyrosulfite independently could not stabilize poloxamer 407 or PEG 4000, but a decrease in stability was not observed by adding mannitol. Similar results from measuring pH were observed in results of measuring viscosity.

Likewise, the following tables show results of studying the change in pH when various amounts of PEG 4000 and poloxamer 407 were added by using 0.1% sodium thiosulfate or 0.1% EDTA as an additive.

[Table 4]

| Amount of PEG 4000 added | Additive | Amount added | Sample No. | At preparation | After four weeks |
|---|---|---|---|---|---|
| 5% | | | D01 | 7.53 | 2.69 |
| 10% | | | D02 | 7.54 | 2.60 |
| 15% | | | D03 | 7.51 | 2.59 |
| 20% | | | D04 | 7.52 | 2.59 |
| 30% | | | D05 | 7.51 | 2.70 |
| 50% | | | D06 | 7.52 | 3.15 |
| 5% | Sodium thiosulfate hydrate | 0.1% | D07 | 7.54 | 7.40 |
| 10% | | 0.1% | D08 | 7.54 | 7.40 |
| 15% | | 0.1% | D09 | 7.52 | 7.40 |
| 20% | | 0.1% | D10 | 7.53 | 7.41 |
| 30% | | 0.1% | D11 | 7.52 | 7.40 |
| 50% | | 0.1% | D12 | 7.51 | 6.82 |
| 5% | EDTA | 0.1% | D13 | 7.53 | 7.50 |
| 10% | | 0.1% | D14 | 7.54 | 7.50 |
| 15% | | 0.1% | D15 | 7.54 | 7.46 |
| 20% | | 0.1% | D16 | 7.54 | 7.38 |
| 30% | | 0.1% | D17 | 7.53 | 4.02 |
| 50% | | 0.1% | D18 | 7.53 | 3.78 |

[Table 5]

| Amount of poloxamer 407 added | Additive | Amount added | Sample No. | At preparation | After four weeks |
|---|---|---|---|---|---|
| 5% | | 0.1% | E01 | 7.54 | 2.81 |
| 15% | | 0.1% | E02 | 7.53 | 5.25 |
| 5% | Sodium thiosulfate hydrate | 0.1% | E03 | 7.53 | 7.36 |
| 15% | | 0.1% | E04 | 7.52 | 7.32 |
| 5% | EDTA | 0.1% | E05 | 7.53 | 7.46 |
| 15% | | 0.1% | E06 | 7.50 | 7.27 |

[Table 6]

| Amount of PEG 4000 added | Amount of poloxamer 407 added | Additive | Amount added | Sample No. | At preparation | After four weeks |
|---|---|---|---|---|---|---|
| 20% | 5% | | | F01 | 7.53 | 2.72 |
| 25% | 5% | | | F02 | 7.52 | 2.79 |
| 30% | 5% | | | F03 | 7.52 | 2,85 |
| 15% | 10% | | | F04 | 7.51 | 3.35 |
| 20% | 10% | | | F05 | 7.53 | 2.94 |
| 30% | 10% | | | F06 | 7.54 | 3.10 |
| 20% | 15% | | | F07 | 7.54 | 3.44 |
| 25% | 15% | | | F08 | 7.51 | 3.44 |
| 30% | 15% | | | F09 | 7.51 | 3.45 |
| 20% | 5% | Sodium thiosulfate hydrate | 0.1% | F10 | 7.54 | 7.33 |
| 25% | 5% | | 0.1% | F11 | 7.52 | 7.29 |
| 30% | 5% | | 0.11% | F12 | 7.50 | 7.37 |
| 15% | 10% | | 0.1% | F13 | 7.52 | 7.35 |
| 20% | 10% | | 0.1% | F14 | 7.54 | 7.43 |
| 30% | 10% | | 0.1% | F15 | 7.54 | 7.44 |
| 20% | 15% | | 0.1% | F16 | 7.53 | 7.42 |
| 25% | 15% | | 0.1% | F17 | 7.53 | 7.46 |
| 30% | 15% | | 0.1% | F18 | 7,52 | 7.49 |

0.1 % sodium thiosulfate was not able to achieve sufficient stabilization when a large amount of PEG 4000, i.e., 50%, was present, but exhibited a high ability to stabilize a smaller amount of PEG 4000 and poloxamer 407. 0.1% EDTA was able to stabilize PEG 4000 up to 20% and exhibited a high ability to stabilize poloxamer 407. Similar results from measuring pH were observed in results of measuring viscosity.

Likewise, the following tables show the results of studying the change in pH when different types of PEG (amount added: 25%) or poloxamer (amount added: 10%) were added by using 0.1% sodium thiosulfate or 0.1% EDTA as an additive.

[Table 7]

| PEG 25% | Additive | Amount added | Sample No. | At preparation | After four weeks |
|---|---|---|---|---|---|
| PEG400 | | | G01 | 7.54 | 2.68 |
| PEG2000 | | | G02 | 7.54 | 2.65 |
| PEG6000 | | | 003 | 7.53 | 2.62 |
| PEG20000 | | | 004 | 7.50 | 2.61 |
| PEG400 | Sodium thiosulfate hydrate | 0.1% | G05 | 7.54 | 7.31 |
| PEG2000 | | 0.1% | G06 | 7.54 | 7.25 |
| PEG6000 | | 0.1% | G07 | 7.53 | 7.27 |
| PEG20000 | | 0.1% | G08 | 7.51 | 7.38 |

[Table 8]

| 10% poloxamer | Additive | Amount added | Sample No. | At preparation | After four weeks |
|---|---|---|---|---|---|
| Poloxamer 188 | | | H01 | 7.53 | 2.76 |
| Poloxamer 188 | Sodium thiosulfate hydrate | 0.1% | H03 | 7.54 | 7.38 |
| Poloxamer 188 | EDTA | 0.1% | H05 | 7.53 | 7.34 |
| Poloxamer 105 | | 0.1% | H06 | 7.52 | 2.86 |
| Poloxamer 184 | | 0.1% | H07 | 7.51 | 4.56 |
| Poloxamer 105 | Sodium thiosulfate hydrate | 0.1% | H08 | 7.53 | 7.36 |
| Poloxamer 184 | | 0.1% | H09 | 7.54 | 7.34 |
| Poloxamer 105 | EDTA | 0.1% | H10 | 7.52 | 7.32 |
| Poloxamer 184 | | 0.1% | H11 | 7.52 | 7.28 |

Sodium thiosulfate exhibited similar stabilization effects on various PEG and poloxamer. EDTA exhibited similar stabilization effects on various poloxamer and exhibited a stabilization effect on low molecular weight PEG 400. Similar results from measuring the pH were observed in the results of measuring the viscosity.

The experiment described above was conducted under a buffer condition of boric acid/borax with a pH of 7.5. Meanwhile, the following table shows the results of studying the change in pH when 0.1% sodium thiosulfate or 0.1% EDTA was used as an additive to the base composition of 25% PEG 4000 + 10% poloxamer 407 by setting the buffer condition as specified in the following Table.

[Table 9]

| Base composition: 25% PEG 4000 + 10% poloxamer 407 | | | | | |
|---|---|---|---|---|---|
| Buffer | Additive | Amount added | Sample No. | At preparation | After four weeks |
| Sodium phosphate pH 5.0 | | | J01 | 4.99 | 3.29 |
| Sodium phosphate pH 5.5 | | | J02 | 5.51 | 3.33 |
| Sodium phosphate pH 6.0 | | | J03 | 5.98 | 3.25 |
| Sodium phosphate pH 7.0 | | | J04 | 7.03 | 3.30 |
| Boric acid/borax pH 8.0 | | | J05 | 8.02 | 3.33 |
| Sodium phosphate pH 5.0 | EDTA | 0.1% | J06 | 4.99 | 4.75 |
| Sodium phosphate pH 5.5 | | 0.1% | J07 | 5.51 | 5.08 |
| Sodium phosphate pH 6.0 | | 0.1% | J08 | 6.03 | 5.69 |
| Sodium phosphate pH 7.0 | | 0.1% | J09 | 7.02 | 5.96 |
| Boric acid/borax pH 8.0 | | 0.1% | J10 | 8.03 | 7.30 |

(continued)

| Base composition: 25% PEG 4000 + 10% poloxamer 407 | | | | | |
|---|---|---|---|---|---|
| **Buffer** | **Additive** | **Amount added** | **Sample No.** | **At preparation** | **After four weeks** |
| **Sodium phosphate pH 7.0** | **Sodium thiosulfate hydrate** | **0.1%** | **J11** | **7.02** | **6.98** |
| **Boric add/borax pH 8.0** | | **0.1%** | **J12** | **8.04** | **7.90** |
| *In the table, "Sodium phosphate" and "Boric acid/borax" have the following composition.<br>*Sodium phosphate: 0.08% sodium hydrogen phosphate hydrate and 0.12% sodium dihydrogen phosphate hydrate<br>*Boric acid/borax: 0.9% boric acid and 0.1% borax | | | | | |

The same results were obtained when the buffer and pH were changed. Similar results from measuring the pH were observed in the results of measuring the viscosity. A formulation comprising EDTA or sodium thiosulfate can be stable at various pHs (e.g., pH of 6).

(Example 2) Solubility Test

There are various advantages in preparing a poorly soluble compound as an aqueous formulation. For this reason, a formulation that can improve the solubility of a poorly soluble compound was tested.

The conditions for additives were changed in an aqueous solution with the following base composition to measure the solubility of a poorly soluble compound.

*0.2% of sodium hydrogen phosphate hydrate
*0.3% of sodium dihydrogen phosphate hydrate
*sodium hydroxide and hydrochloric acid (suitable amount is added to adjust the pH to 7.0)
*sodium hydrogen phosphate hydrate and sodium dihydrogen phosphate hydrate ... Fujifilm Wako Pure Chemical

Specifically, formulations of interest were prepared by measuring and placing sodium hydrogen phosphate hydrate, sodium dihydrogen phosphate hydrate, and an additive in a preparation vessel, and adding purified water. A poorly soluble compound (mebendazole (Toronto Research Chemicals, Canada), dexamethasone (Tokyo Chemical Industry, Japan), or triamcinolone acetonide (Tokyo Chemical Industry, Japan)) was measured out and added to the prepared solution, and the pH was adjusted to 7.0 with hydrochloric acid or sodium hydroxide. Each formulation was stirred in a 5°C, 15°C, or 25°C storage for several days and checked to confirm that the poorly soluble compound was suspended, then filtered through a filter with a pore size of 0.45 $\mu$m (Merck, Germany) in the storage at each temperature. The poorly soluble compound content in the recovered filtrate was measured by liquid chromatography specified below (Shimadzu (Kyoto), high performance liquid chromatograph Prominence).

Measurement of poorly soluble compound

*Each filtrate was appropriately diluted with a diluent (30% acetonitrile solution) to prepare a sample solution.
*A standard product of mebendazole was precisely measured out and dissolved into the diluent (30% acetonitrile solution) to prepare a 0.001% poorly soluble compound standard solution. In this regard, reagents from Fujifilm Wako Pure Chemical (Japan) were used as the standard products of mebendazole and dexamethasone. As the standard product of triamcinolone acetonide, a reagent from the Pharmaceutical and Medical Device Regulatory Science Society of Japan (Japan) was used.
*50 $\mu$L of sample solution and standard solution was measured by liquid chromatography under the following conditions. The peak area of each solution was calculated by automatic integration to compute the poorly soluble compound content in the sample solution.

*Measurement conditions

Detector: UV spectrophotometer (measurement wavelength: 235 nm for mebendazole and 239 nm for dexamethasone

and triamcinolone acetonide)
Column: OSAKA SODA CAPCELL PAK C18 MGII 3 μm, 4.6 × 150 mm
Column temperature: constant temperature of about 40°C
Mobile phase A: 0.1% trifluoroacetic acid/10% acetonitrile solution
Mobile phase B: 0.1% trifluoroacetic acid/90% acetonitrile solution
Delivery of mobile phase: the ratio of mixture of mobile phase A and mobile phase B was changed as follows to control the linear concentration gradient.

[Table 10]

| Time after infusion (min) | Mobile phase A (% volume) | Mobile phase B (% volume) |
|---|---|---|
| 0~30 | 100→0 | 0→100 |
| 30-35 | 0 | 100 |
| 35-35. 01 | 0→100 | 100→0 |
| 35. 01~45 | 100 | 0 |

Detergent: 50% acetonitrile solution
Flow rate: 1.0 mL/min
Temperature within autosampler: 5°C
Area measurement range: 45 minutes
*The following calculation was used for mebendazole and dexamethasone.

Poorly soluble compound content (%) in the standard solution
= $W_F$/fill-up volume (mL) × $Pu_F$/100 × (100 - loss on drying (%))/100 × 1/dilution rate/10

Poorly soluble compound content (%) in the sample solution
= $A_{TF}/A_{SF}$ × poorly soluble compound content (%) in the standard solution × dilution rate

$W_F$: measured amount of poorly soluble compound standard product (mg)
$Pu_F$: purity of poorly soluble compound standard product (%) $A_{SF}$: peak area of poorly soluble compound of a standard solution
$A_{TF}$: peak area of poorly soluble compound of a sample solution
*The following calculation was used for triamcinolone acetonide.

Poorly soluble compound content (%) in the standard solution
= $W_F$/fill-up volume (mL) × 1/dilution rate/10

Poorly soluble compound content (%) in the sample solution
= $A_{TF}/A_{SF}$ × poorly soluble compound content (%) in the standard solution × dilution rate

$W_F$: measured amount of poorly soluble compound standard product (mg)
$A_{SF}$: peak area of poorly soluble compound of a standard solution
$A_{TF}$: peak area of poorly soluble compound of a sample solution

The following conditions were tested as additives to a poorly soluble compound

[Table 11]

| Poloxamer (g/100 mL) | PEG (g/100 mL) | | Poloxamer (g/100 mL) | PEG (g/100 mL) |
|---|---|---|---|---|
| **0** | **0** | | **10** | **0** |
| | **5** | | | **5** |
| | **10** | | | **10** |
| | **15** | | | **15** |
| | **20** | | | **20** |
| | **25** | | | **25** |
| | **30** | | | **30** |
| **5** | **0** | | **15** | **0** |
| | **5** | | | **5** |
| | **10** | | | **10** |
| | **15** | | | **15** |
| | **20** | | | **20** |
| | **25** | | | **25** |
| | **30** | | | **30** |
| *Poloxamer 407 (Kolliphor P 407, BASF, Germany)<br>*PEG 4000 (Macrogol 4000, NOF, Tokyo) | | | | |

The results are shown in Figures 1 to 3. A scale factor (referred to as the solubility scale factor; calculated using the following formulas) computed by comparing a value computed by the solubility of a composition comprising PEG 4000 and poloxamer 407 minus the solubility of the same composition other than not comprising the poloxamer 407, i.e., improvement in the solubility by combining PEG 4000 with poloxamer 407 ($S_{A+B}$ - $S_B$), with a value computed by the solubility of the same composition other than not comprising the PEG 4000 minus the solubility of a composition that comprises neither the poloxamer 407 nor the PEG 4000, i.e., solubility due to poloxamer 407 ($S_A$ - $S_0$), at a temperature of each of 5°C, 15°C, and 25°C, is shown below.

$$\{S_{A+B} - S_B\}/\{S_A - S_0\}$$

wherein

$S_{A+B}$ = solubility of a poorly soluble compound in a composition comprising poloxamer 407 and PEG 4000
$S_B$ = solubility of a poorly soluble compound in a composition comprising PEG 4000, but not poloxamer 407
$S_A$ = solubility of a poorly soluble compound in a composition comprising poloxamer 407, but not PEG 4000
$S_0$ = solubility of a poorly soluble compound in a composition that comprises neither poloxamer 407 nor PEG 4000.

The following Tables 12 to 14 are results from using mebendazole as the poorly soluble compound.

[Table 12]

| Results at 5°C | | | | |
|---|---|---|---|---|
| | | Poloxamer 407 | | |
| | | 5% | 10% | 15% |
| PEG4000 | 0% | 1 | 1 | 1 |
| | 5% | 1.09 | 1.13 | 3.29 |
| | 10% | 1.86 | 1.31 | 4.18 |
| | 15% | 2.57 | 3.42 | 8.75 |
| | 20% | 6.23 | 6.71 | 10.84 |
| | 25% | 13.79 | 12.87 | 15.63 |
| | 30% | 18.31 | 17.11 | 20.86 |

[Table 13]

| Results at 15°C | | | | |
|---|---|---|---|---|
| | | Poloxamer 407 | | |
| | | 5% | 10% | 15% |
| PEG4000 | 0% | 1 | 1 | 1 |
| | 5% | 1.22 | 1.72 | 2.81 |
| | 10% | 1.54 | 2.52 | 4.35 |
| | 15% | 2.18 | 5.01 | 8.33 |
| | 20% | 3.03 | 6.59 | 7.04 |
| | 25% | 5.17 | 9.14 | 8.18 |
| | 30% | 6.24 | 12.23 | 10.19 |

[Table 14]

| Results at 25°C | | | | |
|---|---|---|---|---|
| | | Poloxamer 407 | | |
| | | 5% | 10% | 15% |
| PEG4000 | 0% | 1 | 1 | 1 |
| | 5% | 1.35 | 1.13 | 1.23 |
| | 10% | 1.68 | 1.22 | 1.42 |
| | 15% | 2.03 | 1.37 | 1.46 |
| | 20% | 2.18 | 1.89 | 1.87 |
| | 25% | 3.05 | 1.91 | 1.91 |
| | 30% | 3.84 | 2.26 | 2.16 |

The following Tables 15 to 17 are results from using dexamethasone as the poorly soluble compound.

[Table 15]

| Results at 5°C | | | | |
|---|---|---|---|---|
| | | Poloxamer 407 | | |
| | | 5% | 10% | 15% |
| PEG4000 | 0% | 1 | 1 | 1 |
| | 5% | 1.27 | 1.23 | 1.21 |
| | 10% | 1.87 | 1.79 | 1.65 |
| | 1 5% | 2.03 | 2.07 | 2.16 |
| | 20% | 3.05 | 2.9 | 4.9 |
| | 25% | 4 | 5.79 | 6.12 |
| | 30% | 4.91 | 7.45 | 7.52 |

[Table 16]

| Results at 15°C | | | | |
|---|---|---|---|---|
| | | Poloxamer 407 | | |
| | | 5% | 10% | 15% |
| PEG4000 | 0% | 1 | 1 | 1 |
| | 5% | 1.34 | 1.3 | 1.59 |
| | 1 0% | 1.93 | 1.57 | 2.81 |
| | 15% | 1.82 | 3.32 | 3.31 |
| | 20% | 3.26 | 4.71 | 3.98 |
| | 25% | 5.3 | 5.86 | 4.78 |
| | 30% | 7.52 | 7.1 | 5.79 |

[Table 17]

| Results at 25°C | | | | |
|---|---|---|---|---|
| | | Poloxamer 407 | | |
| | | 5% | 10% | 15% |
| PEG4000 | 0% | 1 | 1 | 1 |
| | 5% | 1.32 | 1.05 | 1.09 |
| | 10% | 1.55 | 1.19 | 1.2 |
| | 15% | 1.55 | 1.26 | 1.31 |
| | 20% | 1.87 | 1.47 | 1.51 |
| | 25% | 2.1 | 1.7 | 1.68 |
| | 30% | 2.12 | 1.85 | 1.97 |

The following Tables 18 to 20 are results from using triamcinolone acetonide as the poorly soluble compound.

[Table 18]

| Results at 5°C | | | | |
|---|---|---|---|---|
| | | Poloxamer 407 | | |
| | | 5% | 10% | 15% |
| PEG4000 | 0% | 1 | 1 | 1 |
| | 5% | 0.67 | 0.97 | 1.12 |
| | 10% | 1.4 | 1.48 | 1.6 |
| | 15% | 1.55 | 2.21 | 2.58 |
| | 20% | 2.74 | 2.58 | 5.35 |
| | 25% | 3.31 | 5.19 | 7.33 |
| | 30% | 3.64 | 7.32 | 7.82 |

[Table 19]

| Results at 15°C | | | | |
|---|---|---|---|---|
| | | Poloxamer 407 | | |
| | | 5% | 10% | 15% |
| PEG4000 | 0% | 1 | 1 | 1 |
| | 5% | 0.91 | 1.13 | 1.57 |
| | 10% | 1.74 | 1.67 | 3.17 |
| | 15% | 1.77 | 3.31 | 4.42 |
| | 20% | 2.8 | 5.07 | 5.12 |
| | 25% | 4.8 | 5.9 | 6.43 |
| | 30% | 6.27 | 7.52 | 7.26 |

[Table 20]

| Results at 25°C | | | | |
|---|---|---|---|---|
| | | Poloxamer 407 | | |
| | | 5% | 10% | 15% |
| PEG4000 | 0% | 1 | 1 | 1 |
| | 5% | 1.29 | 1 | 1.08 |
| | 10% | 1.55 | 1.11 | 1.16 |
| | 15% | 1.62 | 1.23 | 1.28 |
| | 20% | 1.78 | 1.41 | 1.43 |
| | 25% | 2.13 | 1.51 | 1.58 |
| | 30% | 2.49 | 1.69 | 1.82 |

At each of 5°C, 15°C, and 25°C, addition of PEG 4000 improved the solubilization of a poorly soluble compound due to poloxamer 407. Especially at low temperatures of 5°C and 15°C, the solubilization of a poorly soluble compound due to PEG 4000 and poloxamer 407 improved significantly.

The solubility in a composition of PEG and poloxamer was measured in the same manner described above for additional compounds. The following compounds were tested as the additional compounds (measurement wavelength

in UV spectrophotometer in parentheses): fluocinolone acetonide (239 nm), desonide (239 nm), flubendazole (235 nm), cilostazol (254 nm), itraconazole (258 nm), sorafenib (265 nm), regorafenib (260 nm), telmisartan (295 nm), cabozantinib (244 nm), nilotinib (266 nm), aprepitant (215 nm), rotenone (295 nm), griseofulvin (291 nm), osthole (322 nm), 4-bromodibenzofuran (281 nm), simvastatin (238 nm), efavirenz (247 nm), rebamipide (254 nm), and celecoxib (252 nm) .

[Chemical Formula 4]

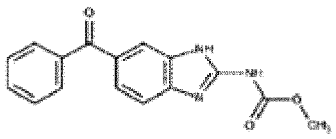

Mebendazole

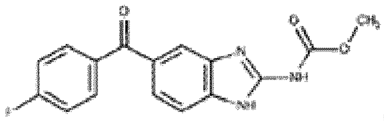

Flubendazole

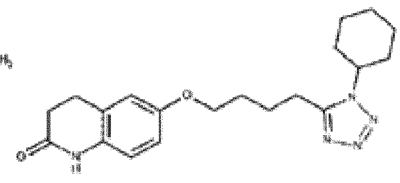

Cilostazol

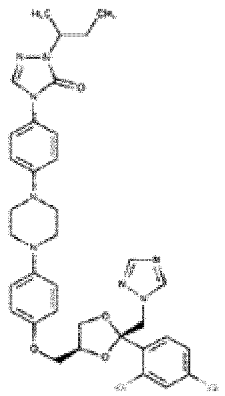

Itraconazole

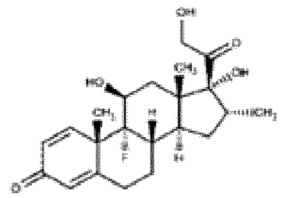

Dexamethasone

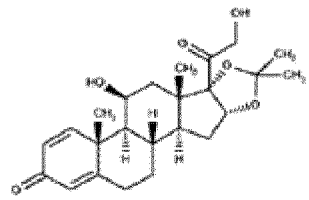

Desonide

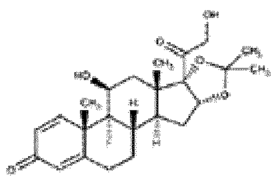

Triamcinolone acetonide

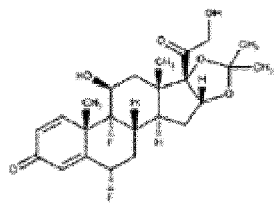

Fluocinolone acetonide

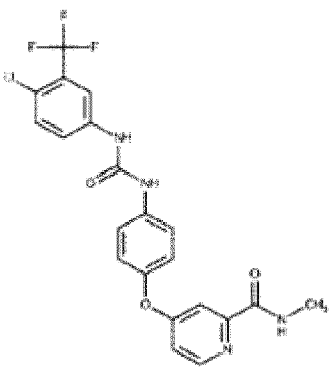

Sorafenib

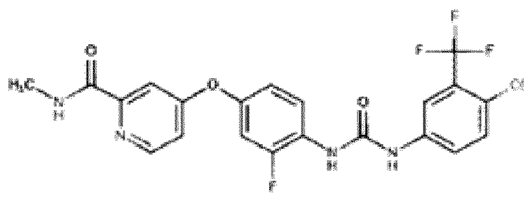

Regorafenib

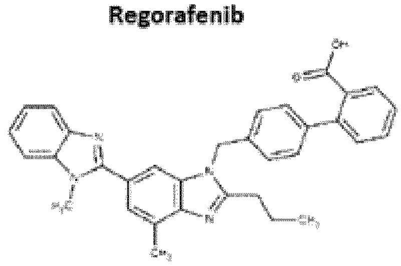

Telmisartan

[Chemical Formula 5]

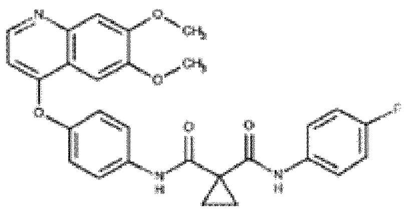

Cabozantinib

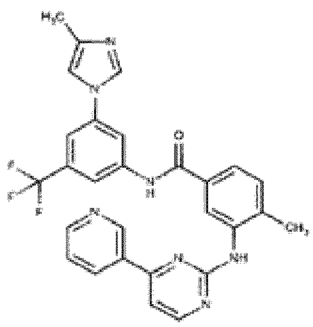

Nilotinib

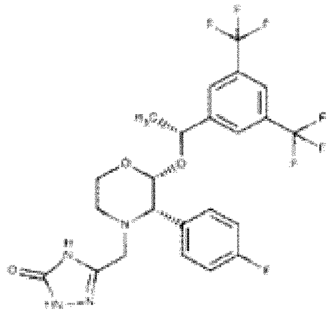

Aprepitant

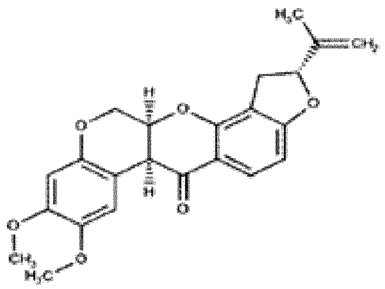

Rotenone

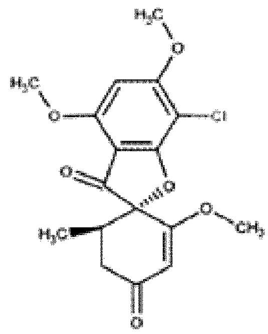

Griseofulvin

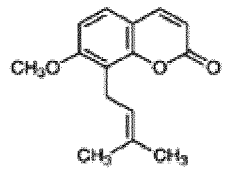

Osthole

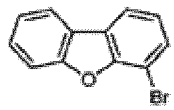

4-bromodibenzofuran

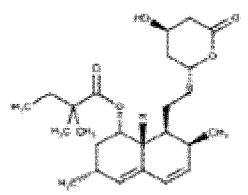

Simvastatin

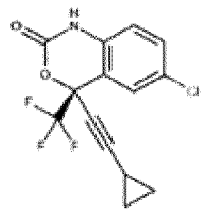

Efavirenz

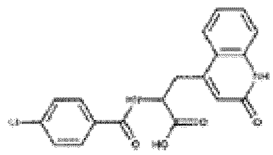

Rebamipide

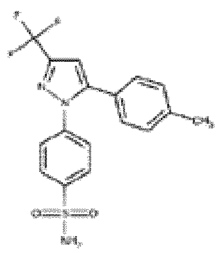

Celecoxib

The additional compounds were obtained from the following sources.

[Table 21]

| Compound name | For preparation of sample solution | Standard product |
|---|---|---|
| | Manufacturer | Manufacturer |
| Itraconazole | Fujifilm Wako Pure Chemical | Fujifilm Wako Pure Chemical |
| Rebamipide | Tokyo Chemical Industry | Tokyo Chemical Industry |
| Telmisartan | Fujifilm Wako Pure Chemical | Fujifilm Wako Pure Chemical |
| Desonide | Tokyo Chemical Industry | Tokyo Chemical Industry |
| Griseofulvin | Tokyo Chemical Industry | Tokyo Chemical Industry |
| Cilostazol | Fujifilm Wako Pure Chemical | Fujifilm Wako Pure Chemical |
| Flubendazole | Tokyo Chemical Industry | Fujifilm Wako Pure Chemical |
| Fluocinolone acetonide | Tokyo Chemical Industry | Pharmaceutical and Medical Device Regulatory Science |
| | | Society of Japan |

(continued)

| Compound name | For preparation of sample solution | Standard product |
|---|---|---|
| | Manufacturer | Manufacturer |
| Osthole | Tokyo Chemical Industry | Tokyo Chemical Industry |
| Rotenone | Tokyo Chemical Industry | Tokyo Chemical Industry |
| Simvastatin | Tokyo Chemical Industry | Tokyo Chemical Industry and Fujifilm Wako Pure Chemical |
| Celecoxib | Tokyo Chemical Industry | Tokyo Chemical Industry |
| Aprepitant | Tokyo Chemical Industry | Tokyo Chemical Industry |
| Efavirenz | Tokyo Chemical Industry | Tokyo Chemical Industry and Fujifilm Wako Pure Chemical |
| 4-bromodibenzofuran | Tokyo Chemical Industry | Tokyo Chemical Industry |
| Regorafenib | Chemscene LLC (regorafenib hydrate) | Chemscene LLC (regorafenib hydrate) |
| Cabozantinib | LC Laboratories | LC Laboratories |
| Nilotinib | Chemscene LLC | Chemscene LLC |
| Sorafenib | Carbosynth Limited | Carbosynth Limited |

For cabozantinib and telmisartan, the pH of the aqueous solution with the base composition described above was changed to 7.4. For rebamipide, the aqueous solution with the base composition was as follow.

*0.05% sodium hydrogen phosphate hydrate
*0.45% sodium dihydrogen phosphate hydrate
*sodium hydroxide and hydrochloric acid (suitable amount was added to adjust the pH to 6.0)

Additional compounds were measured in the same manner described above. As the specific calculation equation, the same formula as dexamethasone described above was applied for itraconazole, telmisartan, cilostazol, and flubendazole, and the same formula for triamcinolone acetonide described above was applied for rebamipide, fluocinolone acetonide, and cabozantinib. Calculations for other compounds were as follows (the specific value for each instance was used for the fill-up value).

*The following calculation was used for desonide, griseofulvin, osthole, rotenone, simvastatin, celecoxib, aprepitant, efavirenz, 4-bromodibenzofuran, and nilotinib.

Poorly soluble compound content in the standard solution (%)
$$= W_F/\text{fill-up volume (mL)} \times Pu_F/100 \times 1/\text{dilution rate}/10$$

Poorly soluble compound content in the sample solution (%)
$$= A_{TF}/A_{SF} \times \text{poorly soluble compound content in the standard solution (\%)} \times \text{dilution rate}$$

$W_F$: measured amount of poorly soluble compound standard product (mg)
$Pu_F$: purity of poorly soluble compound standard product (%) $A_{SF}$: peak area of poorly soluble compound in a standard solution
$A_{TF}$: peak area of poorly soluble compound in a sample solution

*The following calculation was used for regorafenib (hydrate).

Poorly soluble compound content in the standard solution (%)
= $W_F$/fill-up volume (mL) × $Pu_F$/100 × (molecular weight of regorafenib hydrate - (amount of hydrate × molecular weight of water))/molecular weight of regorafenib hydrate × 1/dilution rate/10

Poorly soluble compound content in the sample solution (%)
= $A_{TF}/A_{SF}$ × poorly soluble compound content in the standard solution × dilution rate (%)

$W_F$: measured amount of poorly soluble compound standard product (mg)
$Pu_F$: purity of poorly soluble compound standard product (%) $A_{SF}$: peak area of poorly soluble compound in a standard solution
$A_{TF}$: peak area of poorly soluble compound in a sample solution
*The following calculation was used for sorafenib.

Poorly soluble compound content in the standard solution (%)
= $W_F$/fill-up volume (mL) × (100 - loss on drying (%))/100 × 1/dilution rate/10

Poorly soluble compound content in the sample solution (%)
= $A_{TF}/A_{SF}$ × poorly soluble compound content in the standard solution (%) × dilution rate

$W_F$: measured amount of poorly soluble compound standard product (mg)
$A_{SF}$: peak area of poorly soluble compound in a standard solution
$A_{TF}$: peak area of poorly soluble compound in a sample solution

The results for the solubility of each compound are shown in Figures **4** to **22.** The following Table 22 shows a part of results of calculating the solubility scale factor in the same manner described above. The solubility scale factors under other conditions that are not specifically described in Table 22 are understood based on Figures **4** to **22.** Cabozantinib and telmisartan were tested with a solution adjusted to a pH of 7.4. Rebamipide was tested with a solution adjusted to a pH of 6.0. Other compounds were tested with a solution adjusted to a pH of 7.0. Since efavirenz was converted into gel under the conditions of 15% poloxamer at 15°C and 25°C, and 4-bromodibenzofuran was converted into gel under the conditions of 15% poloxamer at 25°C, measurement results under these conditions may not be accurate (for such cases, an experiment was conducted after cooling for about 5 minutes prior to filtration and dilution to achieve a solution state in order to obtain a reference value).

[Table 22]

| Compound name | 5°C solubility scale factor | | | 15°C solubility scale factor | | |
|---|---|---|---|---|---|---|
| | 30%PEG 4000/ 5% poloxamer 407 | 30%PEG 4000/ 10% poloxamer 407 | 30%PEG 4000/ 15% poloxamer 407 | 30%PEG 4000/ 5% poloxamer 407 | 30%PEG 4000/ 10% poloxamer 407 | 30%PEG 4000/ 15% poloxamer 407 |
| Itraconazole | 29.32 | 15.59 | 8.30 | 31.18 | 12.86 | 11.16 |
| Rebamipide | 3.01 | 1.13 | 1.39 | 1.52 | 1.07 | 0.97 |
| Telmisartan | 4.47 | 2.03 | 1.4 | 2.53 | 1.71 | 1.09 |
| Dexamethasone | 4.91 | 7.45 | 7.52 | 7.52 | 7.1 | 5.79 |
| Desonide | 5.94 | 7.59 | 7.91 | 5.67 | 6.73 | 5.02 |
| Griseofulvin | 14.21 | 15.19 | 12.43 | 12.33 | 14.31 | 10.81 |
| Triamcinolone acetonide | 3.64 | 7.32 | 7.82 | 6.27 | 7.52 | 7.26 |
| Cilostazol | 7.87 | 8.97 | 10.67 | 12.12 | 10.22 | 9.21 |
| Mebendazole | 18.31 | 17.11 | 20.86 | 6.24 | 12.23 | 10.19 |
| Flubendazole | 21.46 | 24.46 | 18.60 | 11.51 | 12.41 | 10.59 |
| Fluocinolone acetonide | 16.45 | 19.75 | 21.60 | 15.76 | 18.27 | 13.97 |
| Osthole | 95.07 | 113.05 | 102.66 | 88.52 | 88.92 | 68.54 |
| Rotenone | 119.31 | 82.15 | 58.13 | 42.10 | 67.76 | 37.30 |
| Simvastatin | 143.32 | 226.30 | 196.85 | 91.19 | 87.92 | 5.25 |
| Celecoxib | 452.95 | 686.99 | 588.66 | 277.34 | 183.33 | 26.18 |
| Aprepitant | 482.38 | 1377.77 | 394.67 | 342.50 | 236.00 | 37.59 |
| Efavirenz | 717.81 | 14.23 | 2.73 | 3.20 | 1.97 | 2.64 |
| 4-bromodibenzofuran | 835.40 | 793.72 | 648.67 | 1.60 | 1.61 | 2.01 |
| Regorafenib | 296.59 | 182.78 | 239.62 | 137.98 | 114.87 | 65.89 |
| Cabozantinib | 208.16 | 288.38 | 304.58 | 65.79 | 74.57 | 46.24 |
| Nilotinib | 169.05 | 144.99 | 83.51 | 55.58 | 31.62 | 27.18 |
| Sorafenib | 899.62 | 2355.97 | 614.39 | 436.87 | 450.47 | 60.19 |

The solubilization of compounds due to poloxamer 407 improved by an addition of PEG 4000 in the same manner for the additional compounds. The extent of improvement in solubilization was lowest for Rebamipide.

The basic solubility to various solvents was tested in order to study the compound properties that suitably achieve improved solubility in the formulation of the present disclosure. The solubility at 25°C was tested for four types of solvents, i.e., ethanol (Fujifilm Wako Pure Chemical), PEG 400 (Fujifilm Wako Pure Chemical), propylene glycol (PG) (Fujifilm Wako Pure Chemical), and buffer solution. In this regard, the buffer solution had the following composition:

*0.2% sodium hydrogen phosphate hydrate
*0.3% sodium dihydrogen phosphate hydrate
*sodium hydroxide and hydrochloric acid (suitable amount was added to adjust the pH to 7.0; however, pH was adjusted to 7.4 for cabozantinib and telmisartan).

However, the following composition was used for rebamipide instead:

*0.05% sodium hydrogen phosphate hydrate
*0.45% sodium dihydrogen phosphate hydrate
*sodium hydroxide and hydrochloric acid (suitable amount was added to adjust the pH to 6.0).

Each compound was measured in the same manner described above. The results of the solubility in each solvent are shown in Table 23. In the Table, the molecular weight and LogP values are reference values (or values calculated from reference values).

[Table 23]

| Compound name | Molecular weight | Log P | Solubility at 25°C | | | |
|---|---|---|---|---|---|---|
| | | | Ethanol | PEG400 | PG | Buffer solution |
| Itraconazole | 705.63 | 5.7[A] | 0.0296716 | 0.2329984 | 0.0168900 | -less than detection limit- |
| Rebamipide | 370.79 | -0.22 | 0.0661531 | 0.1338445 | 0.1156478 | 0.0247321 |
| Telmisartan | 514.62 | 3.2 | 0.0688912 | 0.1032256 | 0.0288046 | 0.0002394 |
| Dexamethasone | 392.46 | 1.83 | 1.6076849 | 1.5064297 | 1.3742763 | 0.0082213 |
| Desonide | 416.5 | 2.7[A] | 1.9409140 | 1.0310041 | 1.2526795 | 0.0071708 |
| Griseofulvin | 352.80 | 2.2[A] | 0.2423147 | 1.3672577 | 0.1568565 | 0.0007834 |
| Triamcinolone acetonide | 434.50 | 2.5[A] | 1.4886196 | 1.2502056 | 0.9700173 | 0.0014264 |
| Cilostazol | 369.46 | 2.76 | 0.2877168 | 0.4341802 | 0.2952282 | 0.0003779 |
| Mebendazole | 295.29 | 3.05 | 0.0070996 | 0.0809826 | 0.0401218 | 0.0000357 |
| Flubendazole | 313.28 | 2.9[A] | 0.0073076 | 0.0576473 | 0.0137315 | 0.0000098 |
| Fluocinolone acetonide | 452.49 | 2.48 | 4.6348494 | 2.8970545 | 3.2238284 | 0.0016061 |
| Osthole | 244.28 | 3.8[A] | 8.8796992 | 11.7346187 | 1.9505386 | 0.0005355 |
| Rotenone | 394.4 | 4.1[A] | 0.2726473 | 3.3140428 | 0.1184340 | 0.0000090 |
| Simvastatin | 418.57 | 4.7[A] | 20.7342871 | 5.2404130 | 3.3980178 | 0.0002165 |
| Celecoxib | 381.37 | 4 or greater | 10.2421911 | 47.1609723 | 3.6146960 | 0.0001268 |
| Aprepitant | 534.43 | 4.8±0.1 | 2.4827211 | 1.1635433 | 0.8447124 | 0.0000192 |
| Efavirenz | 315.67 | 5.40 | 73.9498924 | 70.5246786 | 48.3027223 | 0.0007278 |
| 4-bromodibenzofuran | 247.09 | 4.8[A] | 5.8065947 | 22.8986083 | 1.4486694 | 0.0000136 |
| Regorafenib | 482.8 | 5.2[B] | 0.8366318 | 3.2104848 | 0.4952526 | 0.0000004174 |
| Cabozantinib | 501.5 | 3.92 | 0.1543956 | 2.0541037 | 0.0961013 | 0.0000000792 |
| Nilotinib | 529.5 | 4.9[A] | 0.0922241 | 0.4032993 | 0.0816342 | 0.0000000568 |
| Sorafenib | 464.8 | 5.6[B] | 0.6656119 | 1.0037285 | 0.6227120 | 0.0000001392 |

[A] value calculated from XLogP3

[B] value calculated from CLogP

*pH upon measurement of LogP: rebamipide (pH 7.0), telmisartan (pH 7.4), cilostazol (pH 7.0), mebendazole (pH 6.0), celecoxib (pH 7), aprepitant (pH7), cabozantinib (pH 7.4)

As an indicator of poor solubility of a compound, the ratio of solubility in a non-aqueous solvent (ethanol, PEG 400, PG) as compared to a buffer solution (aqueous solvent) was found. Itraconazole was excluded because the solubility in the buffer solution was below the detection limit.

Such solubility ratios and values extracted from the solubility scale factor for PEG/poloxamer formulation found as above are summarized in the following Table 24.

[Table 24]

| Compound name | Solubility ratio of solvent with respect to buffer solution | | | Solubility scale factor at 5°C | Solubility scale factor at 15°C |
|---|---|---|---|---|---|
| | Ethanol/buffer solution | PEG400/buffer solution | PG/buffer solution | 30% PEG 4000/10% poloxamer 407 | 30% PEG 4000/10% poloxamer 407 |
| Rebamipide | 2.67 | 5.41 | 4.68 | 1.13 | 1.07 |
| Telmisartan | 287.77 | 431.18 | 120.32 | 2.03 | 1.71 |
| Dexamethasone | 195.55 | 183.23 | 167.16 | 7.45 | 7.10 |
| Desonide | 270.67 | 143.78 | 174.69 | 7.59 | 6.73 |
| Griseofulvin | 309.31 | 1745.29 | 200.23 | 15.19 | 14.31 |
| Triamcinolone acetonide | 1043.62 | 876.48 | 680.05 | 7.32 | 7.52 |
| Cilostazol | 761.36 | 1148.93 | 781.23 | 8.97 | 10.22 |
| Mebendazole | 198.87 | 2268.42 | 1123.86 | 17.11 | 12.23 |
| Flubendazole | 745.67 | 5882.38 | 1401.17 | 24.46 | 12.41 |
| Fluocinolone acetonide | 2885.78 | 1803.78 | 2007.24 | 19.75 | 18.27 |
| Osthole | 16582.07 | 21913.39 | 3642.46 | 113.05 | 88.92 |
| Rotenone | 30294.14 | 368226.98 | 13159.33 | 82.15 | 67.76 |
| Simvastatin | 95770.38 | 24205.14 | 15695.23 | 226.30 | 87.92 |
| Celecoxib | 80774.38 | 371931.96 | 28507.07 | 686.99 | 183.33 |
| Aprepitant | 129308.39 | 60601.21 | 43995.44 | 1377.77 | 236.00 |
| Efavirenz | 101607.44 | 96901.18 | 66368.13 | 14.23 | 1.97 |
| 4-bromodibenzofuran | 426955.49 | 1683721.20 | 106519.81 | 793.72 | 1.61 |
| Regorafenib | 2004388.60 | 7691626.26 | 1186517.97 | 182.78 | 114.87 |
| Cabozantinib | 1949439.39 | 25935652.78 | 1213400.25 | 288.38 | 74.57 |
| Nilotinib | 1623663.73 | 7100339.79 | 1437221.83 | 144.99 | 31.62 |
| Sorafenib | 4781694.68 | 7210693.25 | 4473505.75 | 2355.97 | 450.47 |

It was observed that the solubility ratio in a non-aqueous solvent relative to an aqueous solvent is highly correlated with the solubilization improvement level due to the PEG/poloxamer formulation of the present disclosure. It is expected that the solubility ratio and solubility scale factor are similarly low for rebamipide when tested at pH of 7.0. Similarly, it is expected that the solubility ratio and solubility scale factor would not be significantly different from the results described above when cabozantinib and telmisartan are tested at a pH of 7.0.

In view of the structure and property of each compound, it is understood that poorly soluble compounds with the following features tend to especially benefit from solubilization due to the formulation of the present disclosure:

*having high solubility ratio in a non-aqueous solvent relative to an aqueous solvent
*having no readily ionizable group such as a carboxyl group or tetrazole
*having a fluorine atom (excluding cases where a hydroxyl group or -NH group is present at a vicinal position of a fluorine atom)
*having no hydroxyl group
*having a molecular weight of 350 or greater
*having two or more rings.

The solubility of a poorly soluble compound was measured by further adding a water-soluble macromolecule. As a result, sulfobutylether-β-cyclodextrin further improved the dissolution of a poorly soluble compound due to poloxamer 407 and PEG 4000. Carboxymethyl cellulose was observed to have a tendency to further assist dissolution of a poorly soluble compound due to poloxamer 407 and PEG 4000.

More specifically, one of the following modifications was added for measuring the solubility in a PEG/poloxamer

composition similar to those described above for celecoxib:

*add 0.5% carboxymethyl cellulose (CMC) to a PEG/poloxamer composition
*add 5% sulfobutylether-β-cyclodextrin (SBE-β-CD) to a PEG/poloxamer composition
*add 5% hydroxypropyl-β-cyclodextrin (HP-β-CD) to a PEG/poloxamer composition
*CMC ... DKS Co. Ltd.
*SBE-β-CD ... MedChemExpress LLC
*HP-β-CD ... Fujifilm Wako Pure Chemical

The results of the solubility of each compound are shown in Figures **23** to **25.** While the addition of HP-β-CD hardly improved the solubility, the addition of SBE-β-CD or CMC improved solubility overall.

(Note)

As disclosed above, the present disclosure has been exemplified by the use of its preferred embodiments. However, it is understood that the scope of the present disclosure should be interpreted based solely on the Claims. It is also understood that any patent, patent application, and references cited herein should be incorporated herein by reference in the same manner as the contents are specifically described herein. The present application claims priority to Japanese Patent Application No. 2020-16656 filed on February 3, 2020 with the JPO. The entire content thereof is incorporated herein by reference in the same manner as the contents are specifically described herein.

[Industrial Applicability]

The present disclosure can be used to improve the ease of handling of agents (especially drugs).

## Claims

1. A composition for use in stabilizing a polyether compound, comprising a chelate compound.

2. A composition comprising a polyether compound and a chelate compound.

3. The composition of claim 1 or 2, wherein the polyether compound comprises poloxamer and polyethylene glycol.

4. The composition of any one of claims 1 to 3, wherein the chelate compound is selected from the group consisting of thiosulfate, ethylenediaminetetraacetic acid (EDTA) or a salt or ion thereof, and citric acid or a salt or ion thereof.

5. The composition of any one of claims 1 to 4, wherein the chelate compound comprises thiosulfate.

6. The composition of claim 4 or 5, wherein the composition further comprises at least one of mannitol and dibutylhydroxytoluene (BHT).

7. The composition of any one of claims 1 to 6, wherein the poloxamer has an average molecular weight of about 1000 to 15000.

8. The composition of any one of claims 1 to 7, wherein the poloxamer comprises 10 to 90 wt% of polyoxyethylene per molecule.

9. The composition of any one of claims 1 to 8, wherein the poloxamer has polyoxypropylene at an average molecular weight of about 900 to 4000 per molecule.

10. The composition of any one of claims 1 to 9, wherein the polyethylene glycol has an average molecular weight of about 200 to 50000.

11. A composition for use in improving solubility of a poorly soluble compound by a combination of a hydrophobic polyoxy aliphatic moiety-containing polyether compound and a hydratable compound, comprising at least one of a hydrophobic polyoxy aliphatic moiety-containing polyether compound and a hydratable compound.

12. A composition comprising a poorly soluble compound, a hydrophobic polyoxy aliphatic moiety-containing polyether compound, and a hydratable compound.

13. The composition of claim 11 or 12, wherein the hydrophobic polyoxy aliphatic moiety-containing polyether compound is a polyoxypropylene-containing polyether compound.

14. The composition of any one of claims 11 to 13, wherein the hydrophobic polyoxy aliphatic moiety-containing polyether compound comprises poloxamer.

15. The composition of claim 14, wherein the poloxamer has an average molecular weight of about 1000 to 15000.

16. The composition of claim 14 or 15, wherein the poloxamer comprises 10 to 90 wt% of polyoxyethylene per molecule.

17. The composition of any one of claims 14 to 16, wherein the poloxamer has polyoxypropylene at an average molecular weight of about 900 to 4000 per molecule.

18. The composition of any one of claims 11 to 17, wherein the hydratable compound comprises polyethylene glycol.

19. The composition of claim 18, wherein the polyethylene glycol has an average molecular weight of about 200 to 50000.

20. The composition of any one of claims 11 to 19, wherein the poorly soluble compound has LogP of 0.5 to 8.

21. The composition of any one of claims 11 to 20, wherein the poorly soluble compound has LogP of 0.5 to 8 at a pH of the composition.

22. The composition of any one of claims 11 to 21, wherein improvement in solubility of the poorly soluble compound by adding the hydratable compound to the hydrophobic polyoxy aliphatic moiety-containing polyether compound under conditions of an atmospheric pressure of 1 atm at 25°C is 1.1-fold or greater.

23. The composition of any one of claims 11 to 22, wherein improvement in solubility of the poorly soluble compound by adding the hydratable compound to the hydrophobic polyoxy aliphatic moiety-containing polyether compound under conditions of an atmospheric pressure of 1 atm at 15°C is 1.5-fold or greater.

24. The composition of any one of claims 11 to 23, wherein improvement in solubility of the poorly soluble compound by adding the hydratable compound to the hydrophobic polyoxy aliphatic moiety-containing polyether compound under conditions of an atmospheric pressure of 1 atm at 5°C is 2-fold or greater.

25. The composition of any one of claims 11 to 24, comprising cyclodextrin or a water-soluble macromolecule.

26. The composition of any one of claims 11 to 25, comprising at least one of sulfobutylether-β-cyclodextrin and carboxymethyl cellulose.

27. The composition of any one of claims 11 to 26, which is a composition of any one of claims 1 to 10.

28. The composition of any one of claims 1 to 27, comprising a buffer.

29. The composition of any one of claims 1 to 28, which is an orally administered agent, a topical agent, or an injection agent.

30. The composition of any one of claims 1 to 29, which is for intravenous administration, intramuscular administration, subcutaneous administration, or intravitreal administration.

31. The composition of any one of claims 1 to 29, which is for administration selected from the group consisting of dermal administration, intranasal administration, eye ball administration, mucosal administration, rectal administration, and inhalation administration.

32. The composition of any one of claims 1 to 30 for application to an eye.

**33.** The composition of any one of claims 1 to 31, which is an eye drop.

Mebendazole

Fig 1

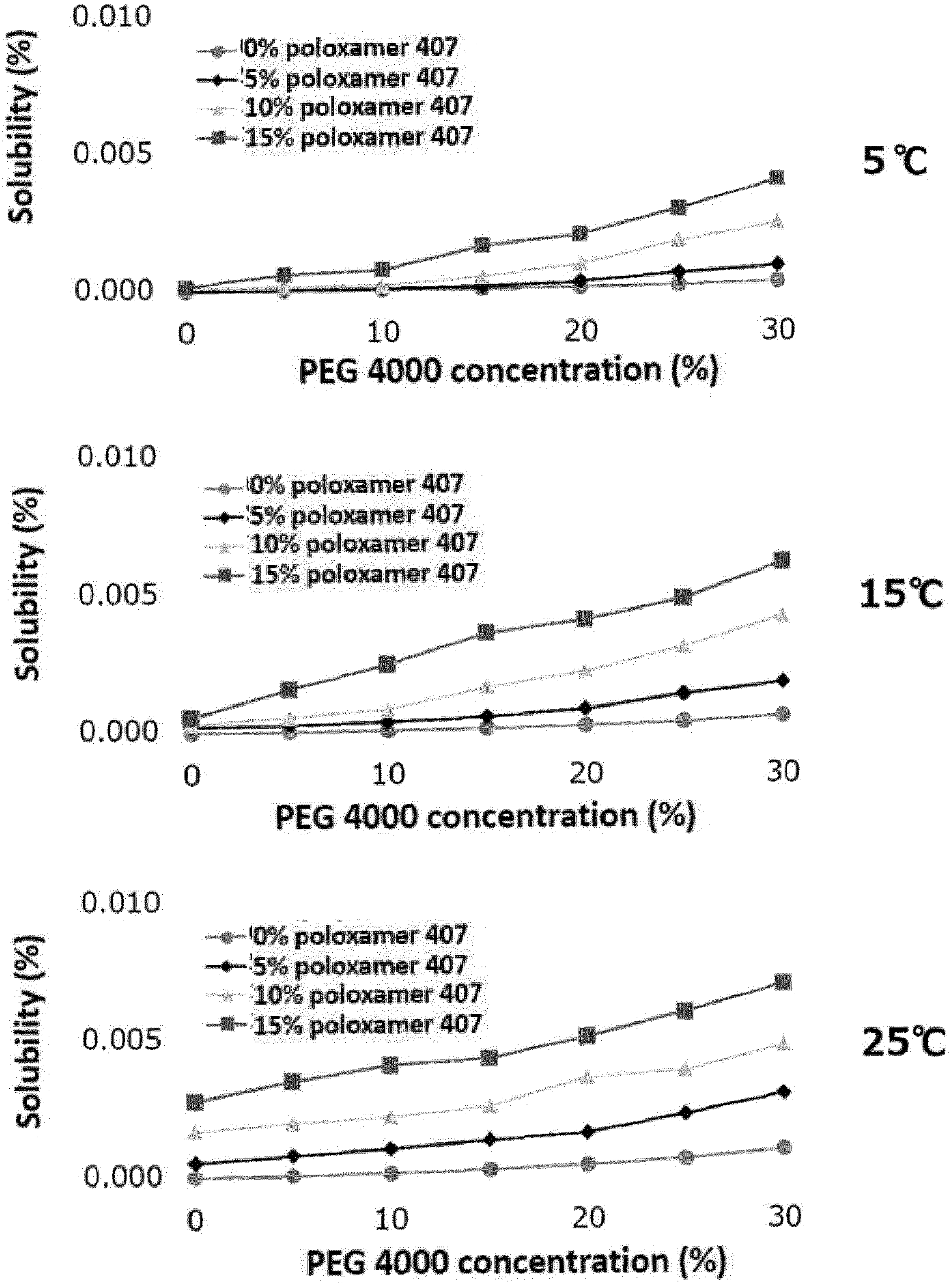

Dexamethasone

Fig 2

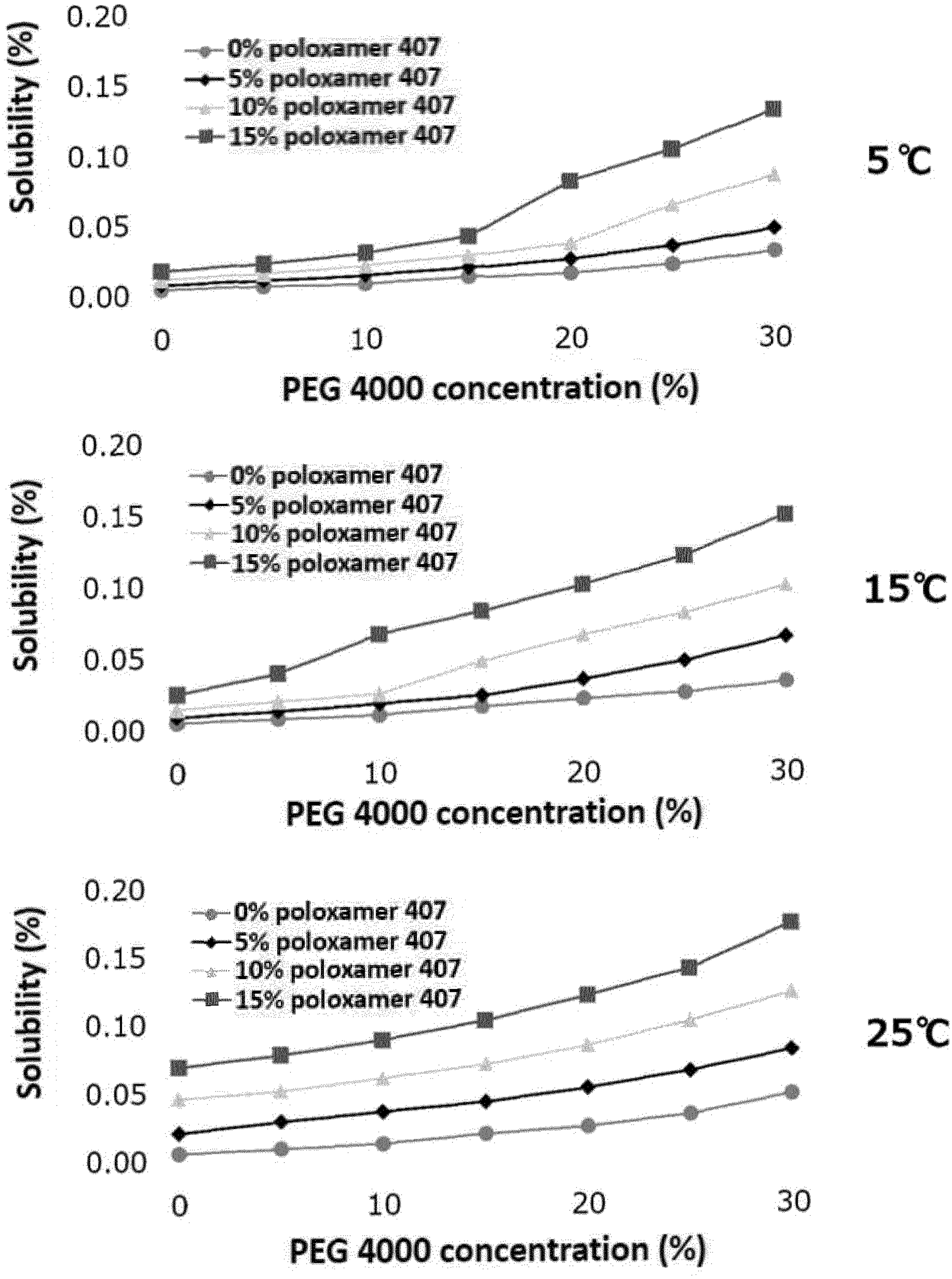

# Triamcinolone acetonide

Fig 3

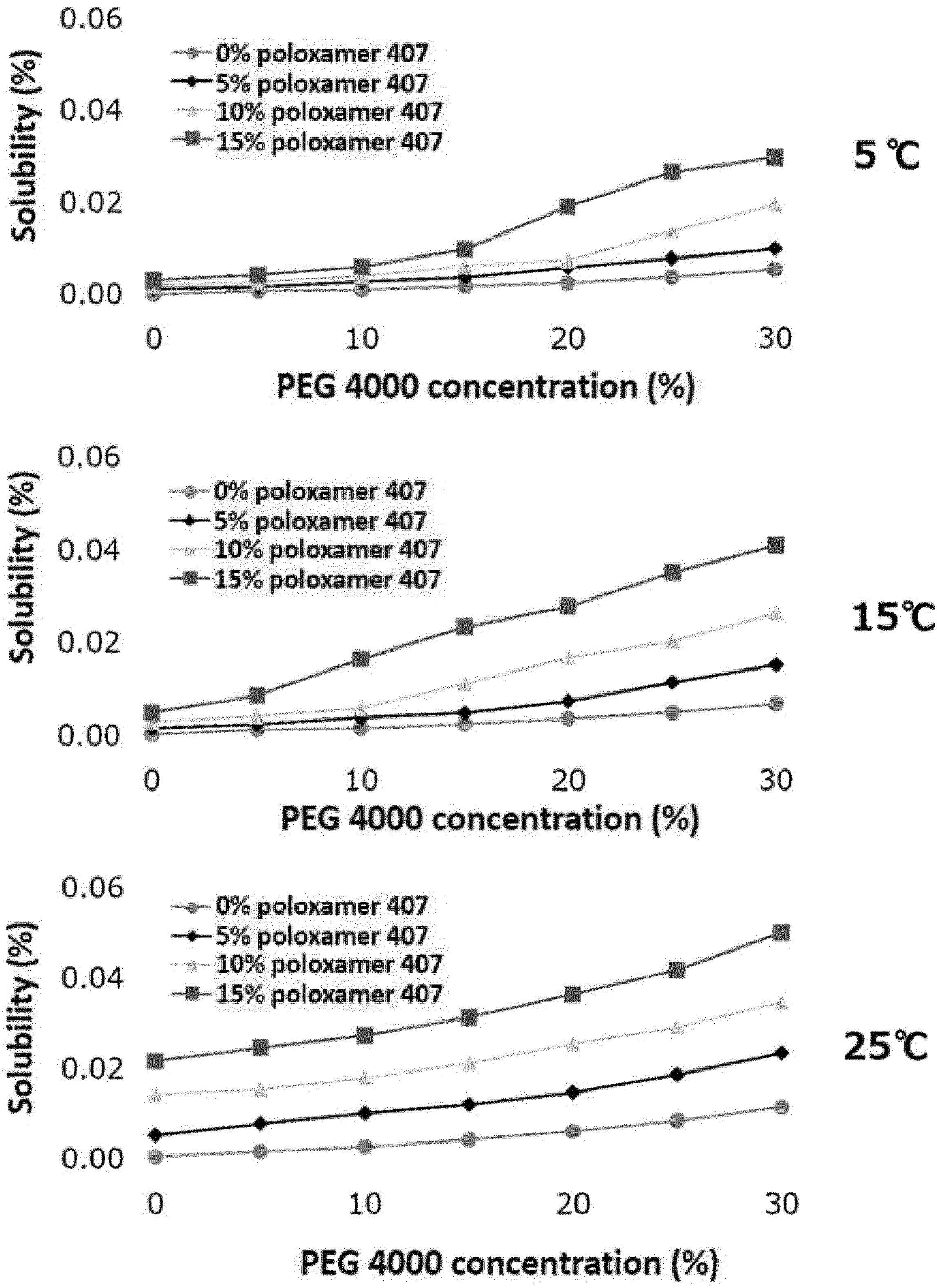

# Fluocinolone acetonide

Fig 4

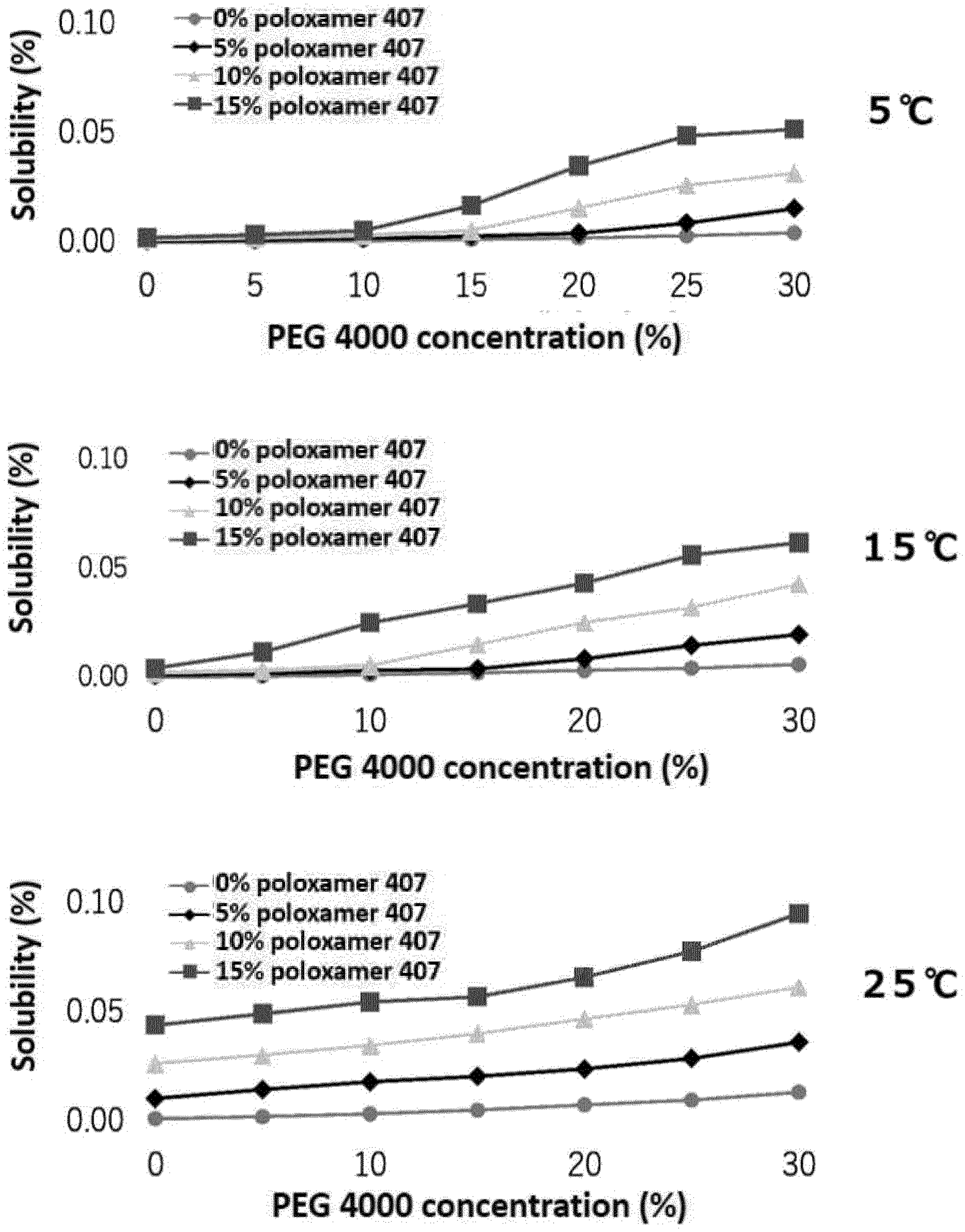

Desonide

Fig 5

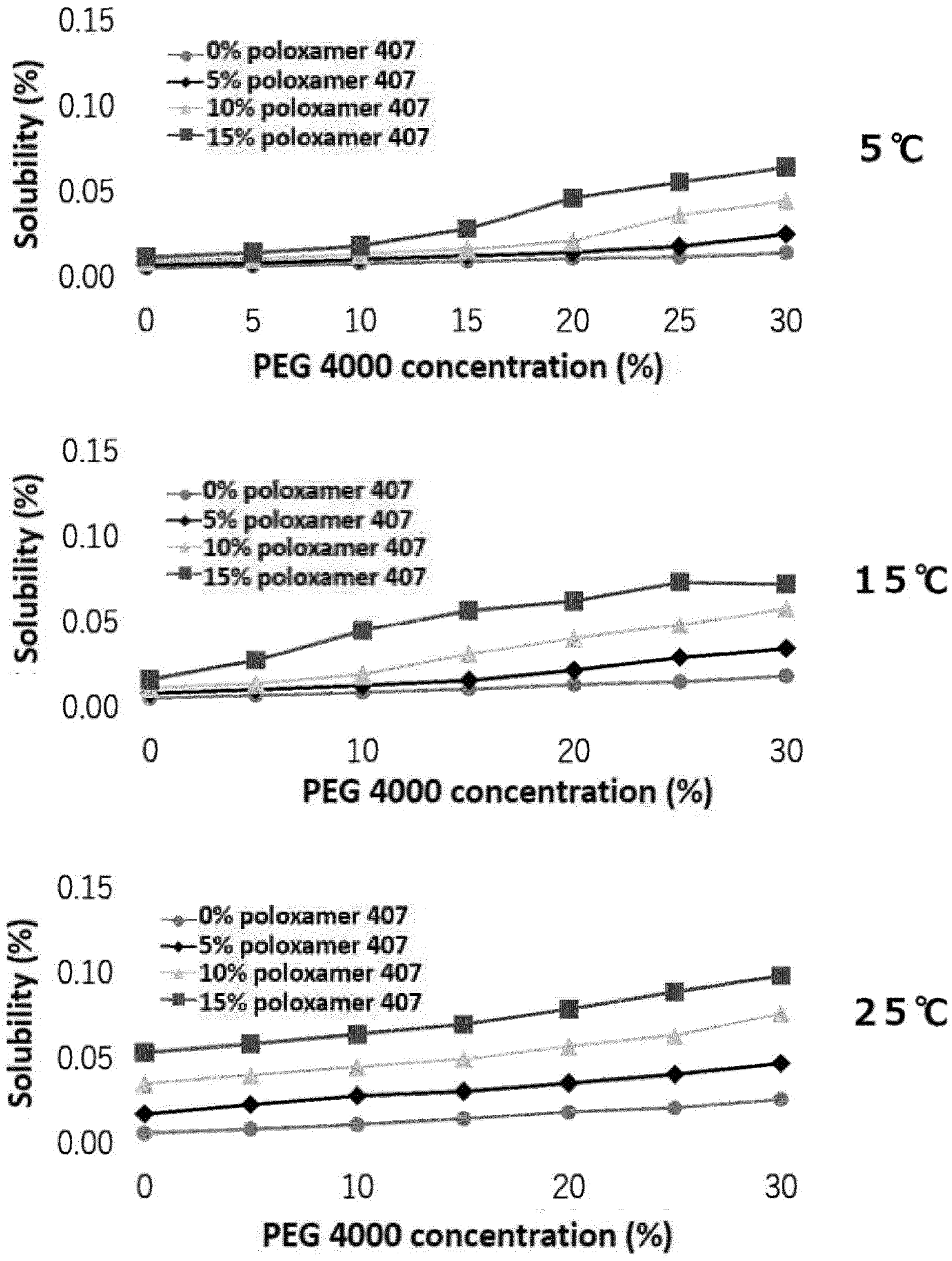

# Flubendazole

Fig 6

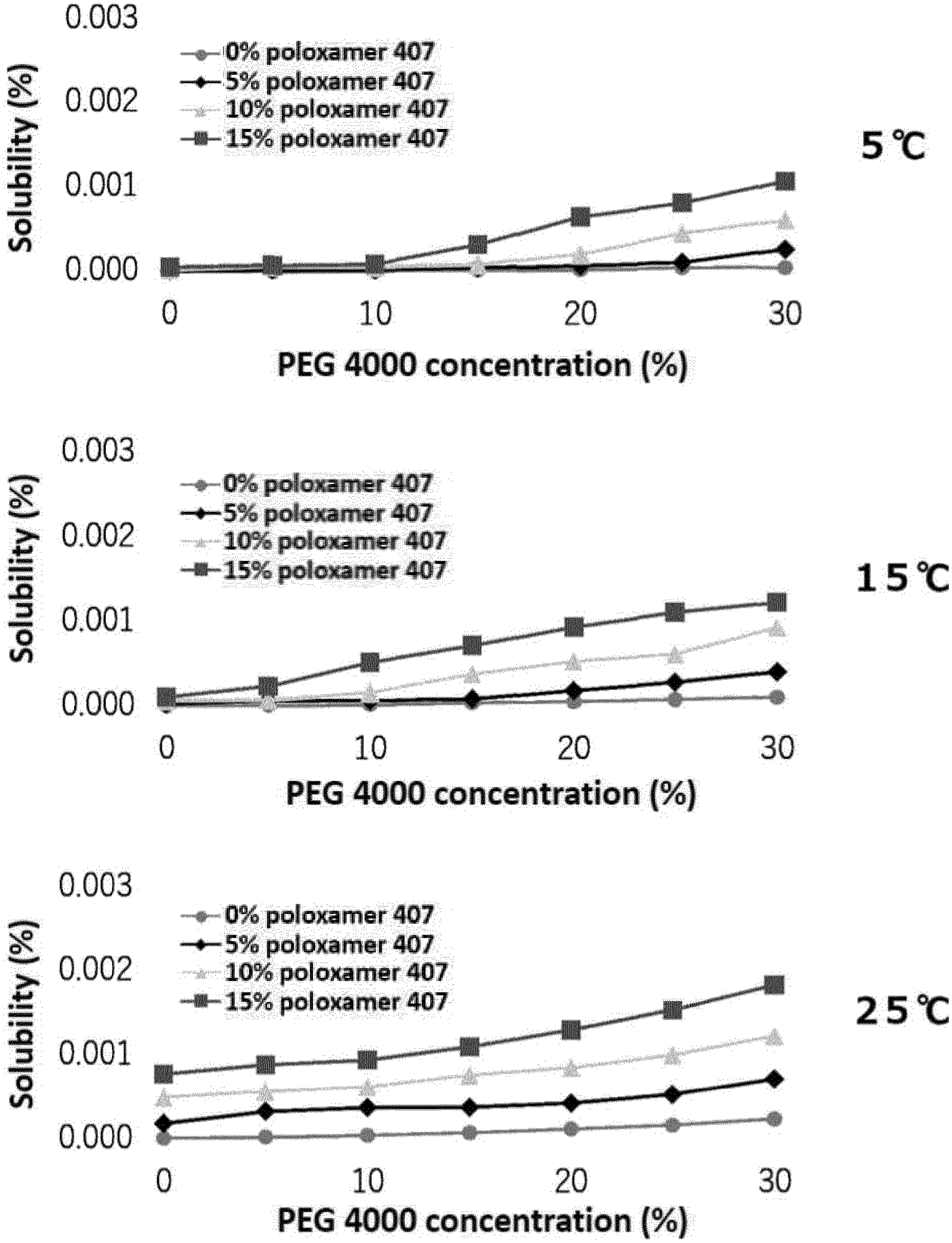

# Cilostazol

Fig 7

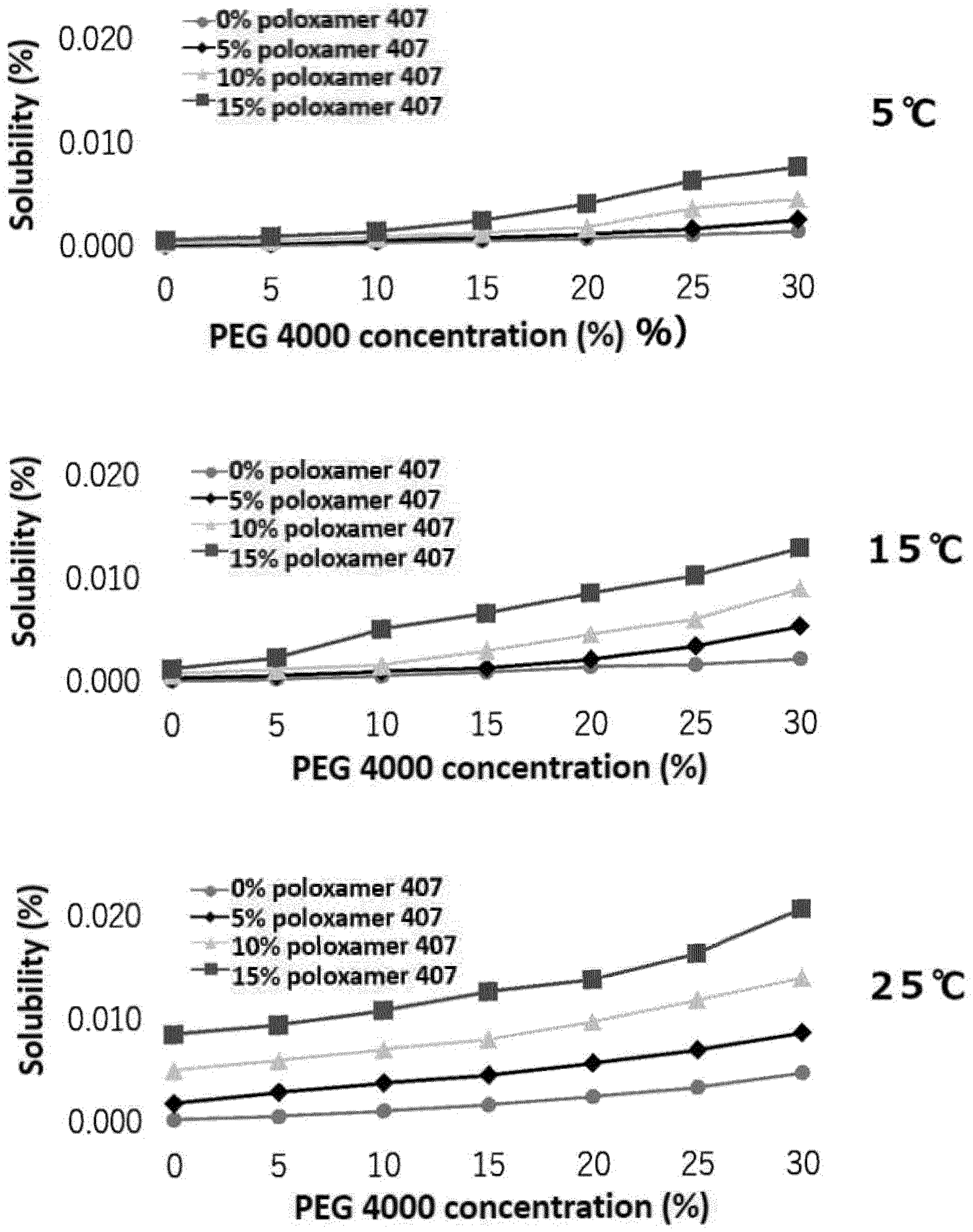

Itraconazole

Fig 8

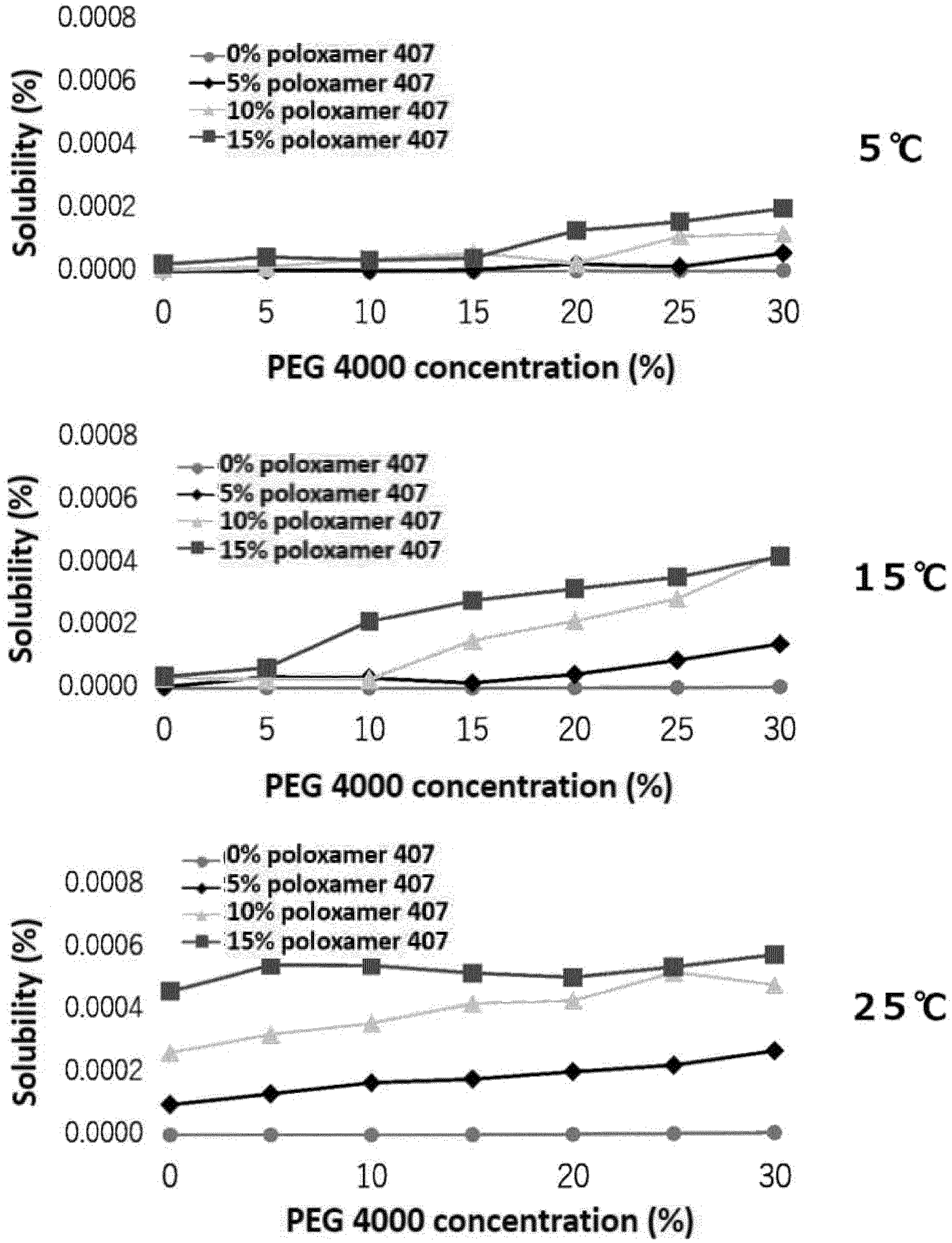

Sorafenib

Fig 9

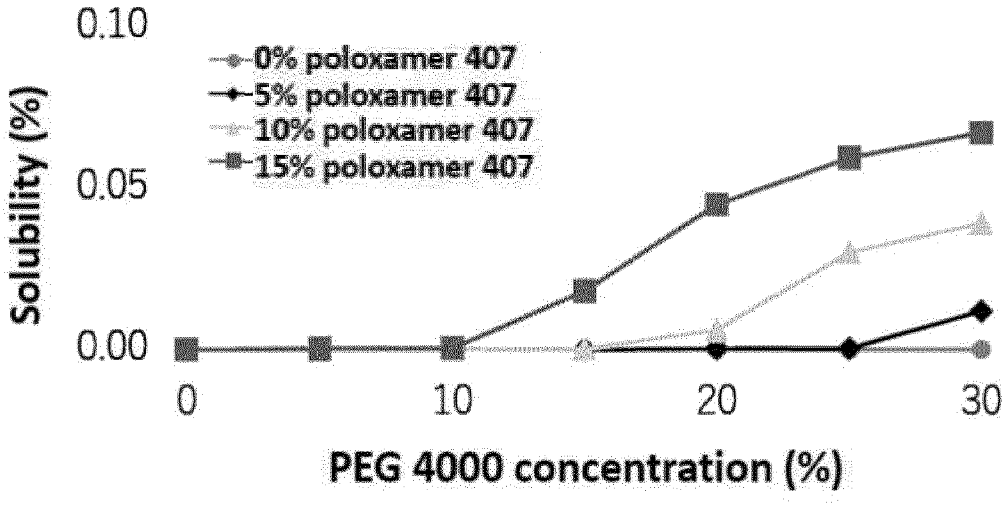


5 ℃

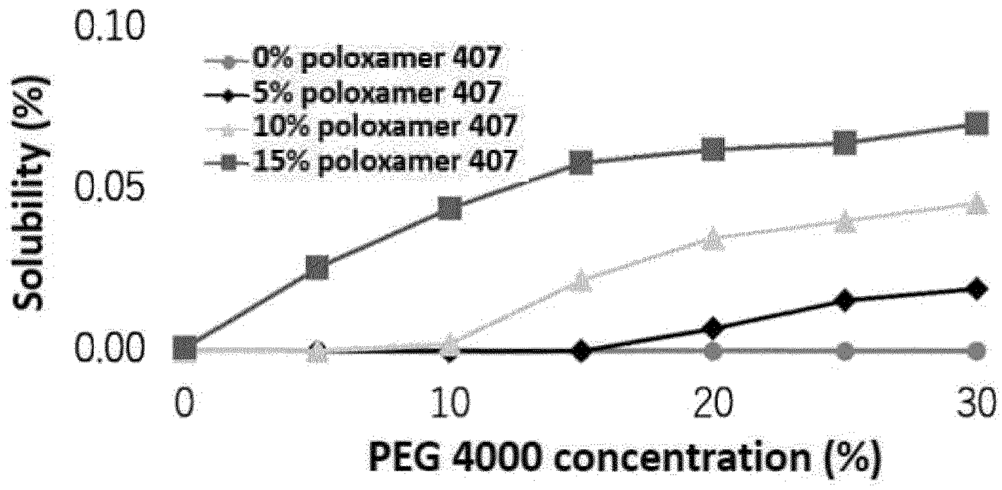


15 ℃

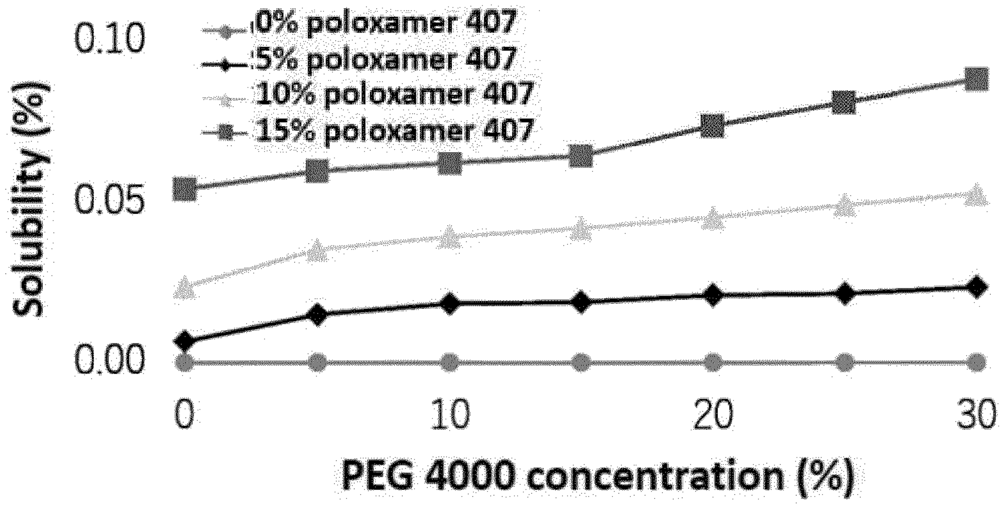


25 ℃

# Regorafenib

Fig 10

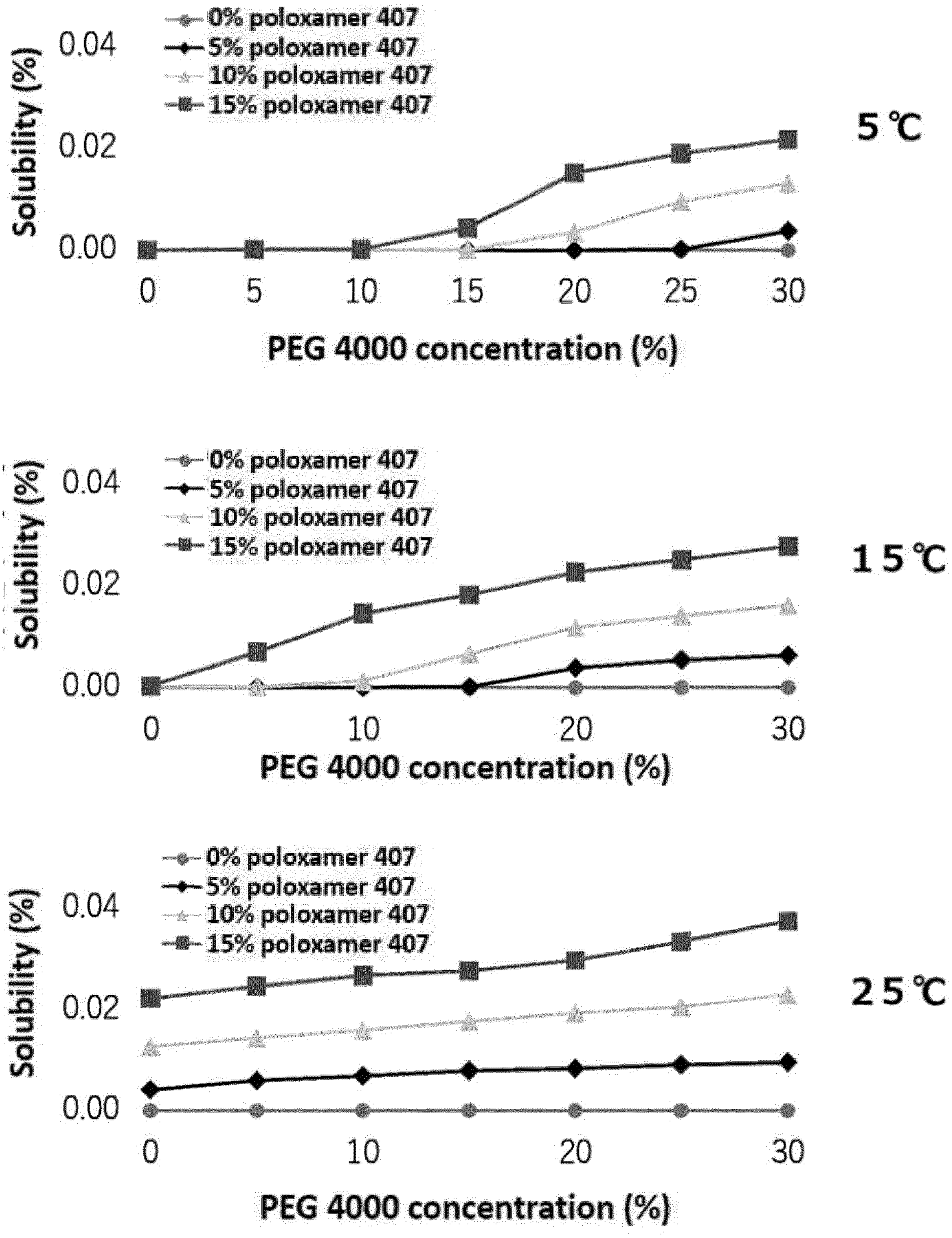

# Telmisartan

Fig 11

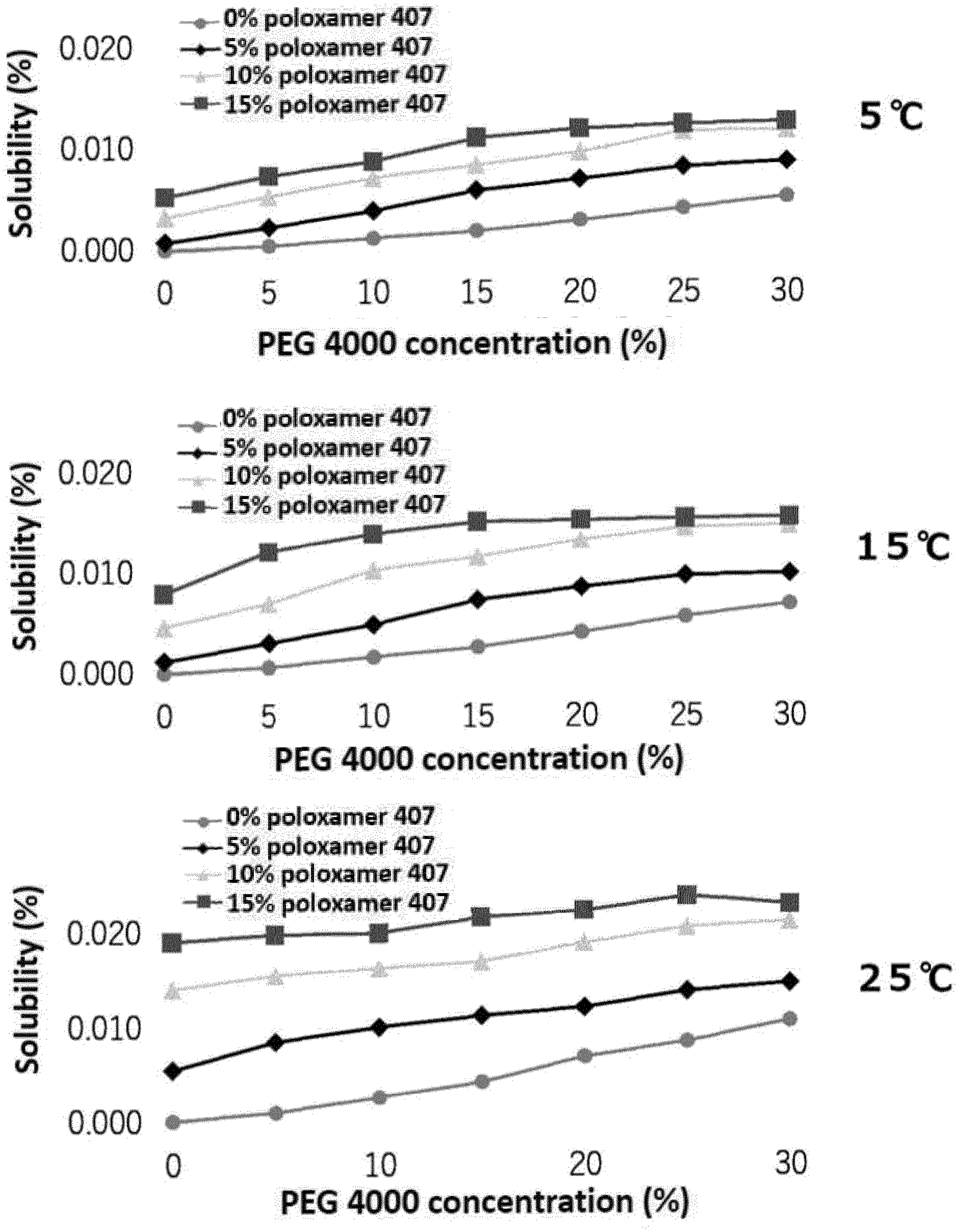

# Cabozantinib

Fig 12

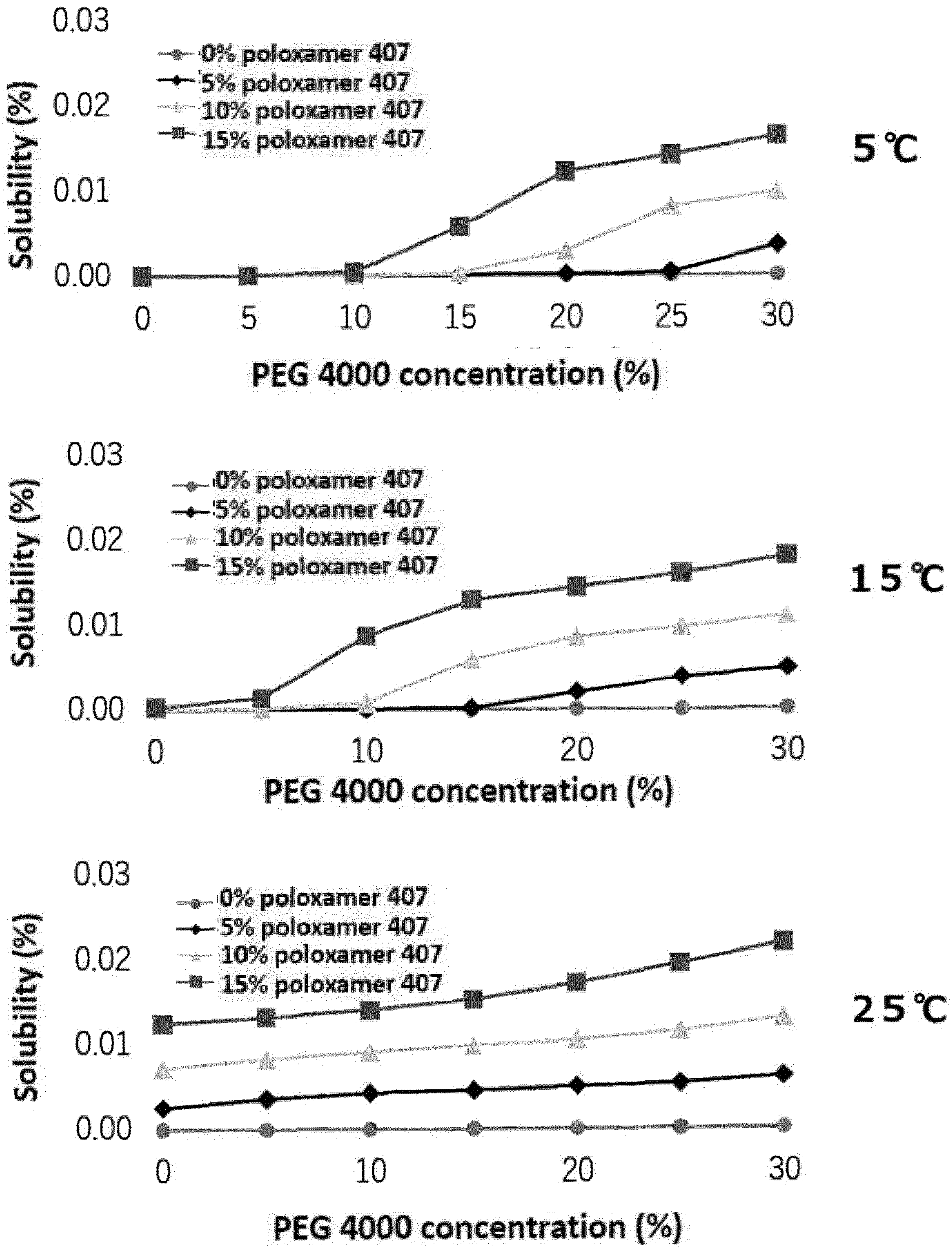

# Nilotinib

Fig 13

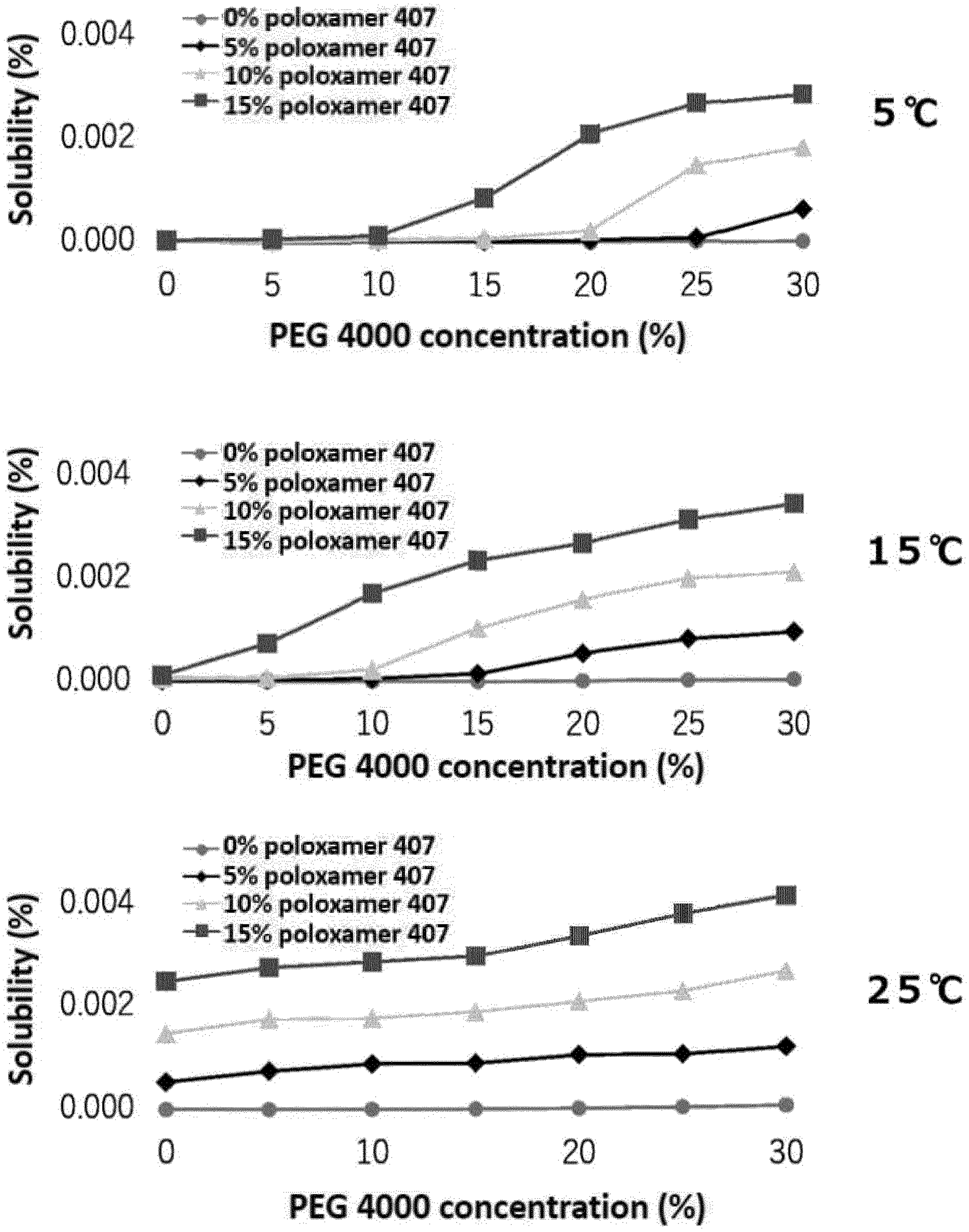

# Aprepitant

Fig 14

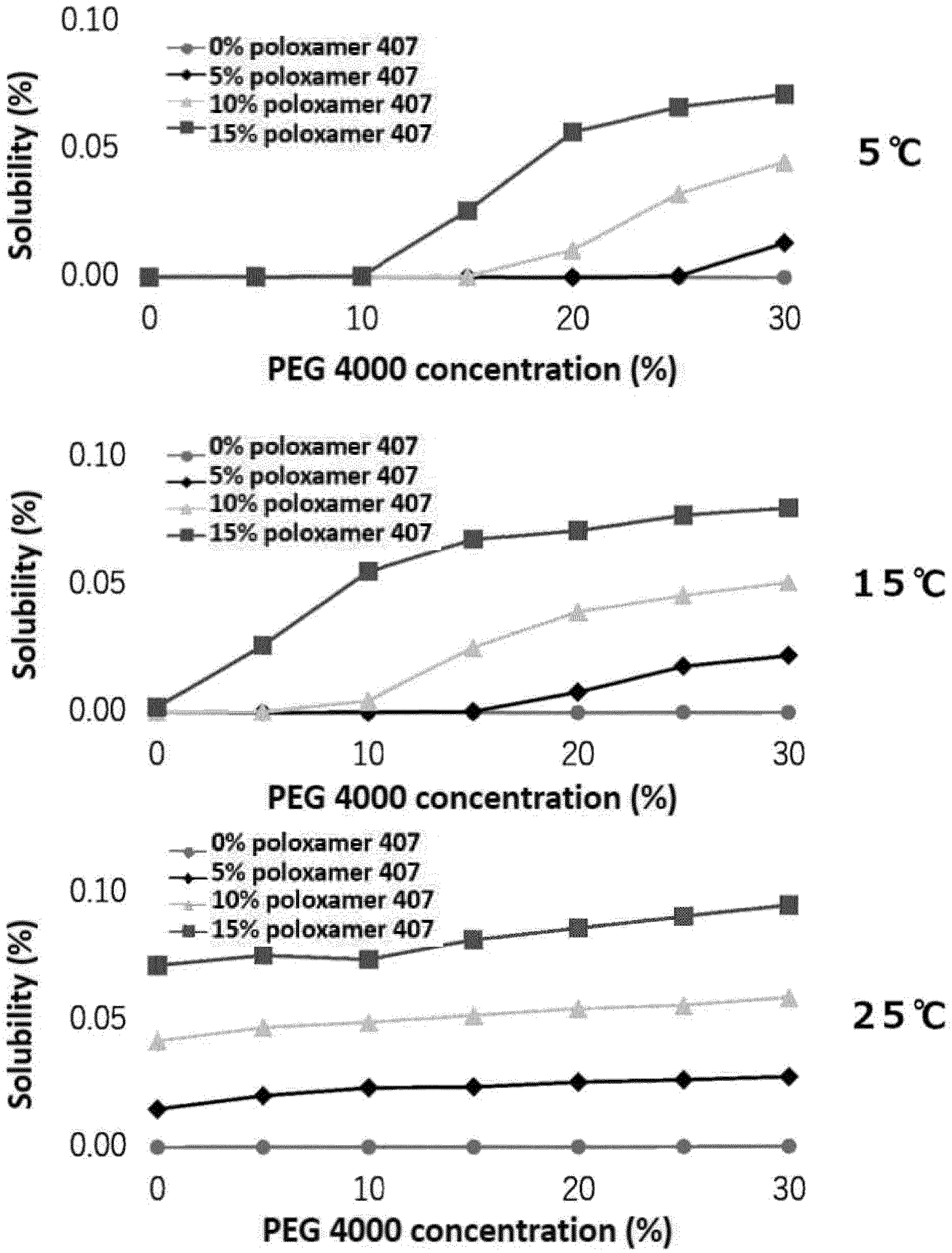

Rotenone

Fig 15

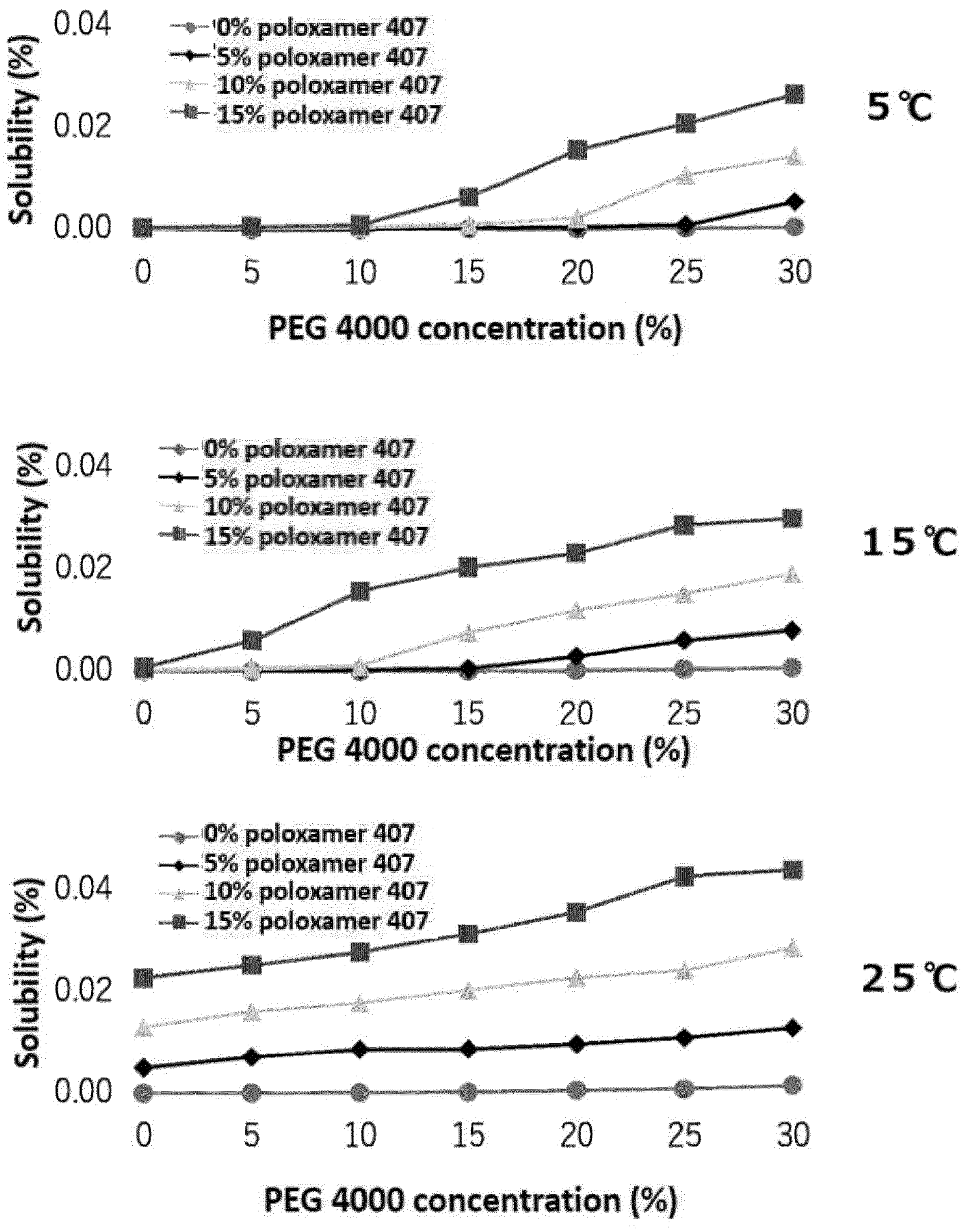

Griseofulvin

Fig 16

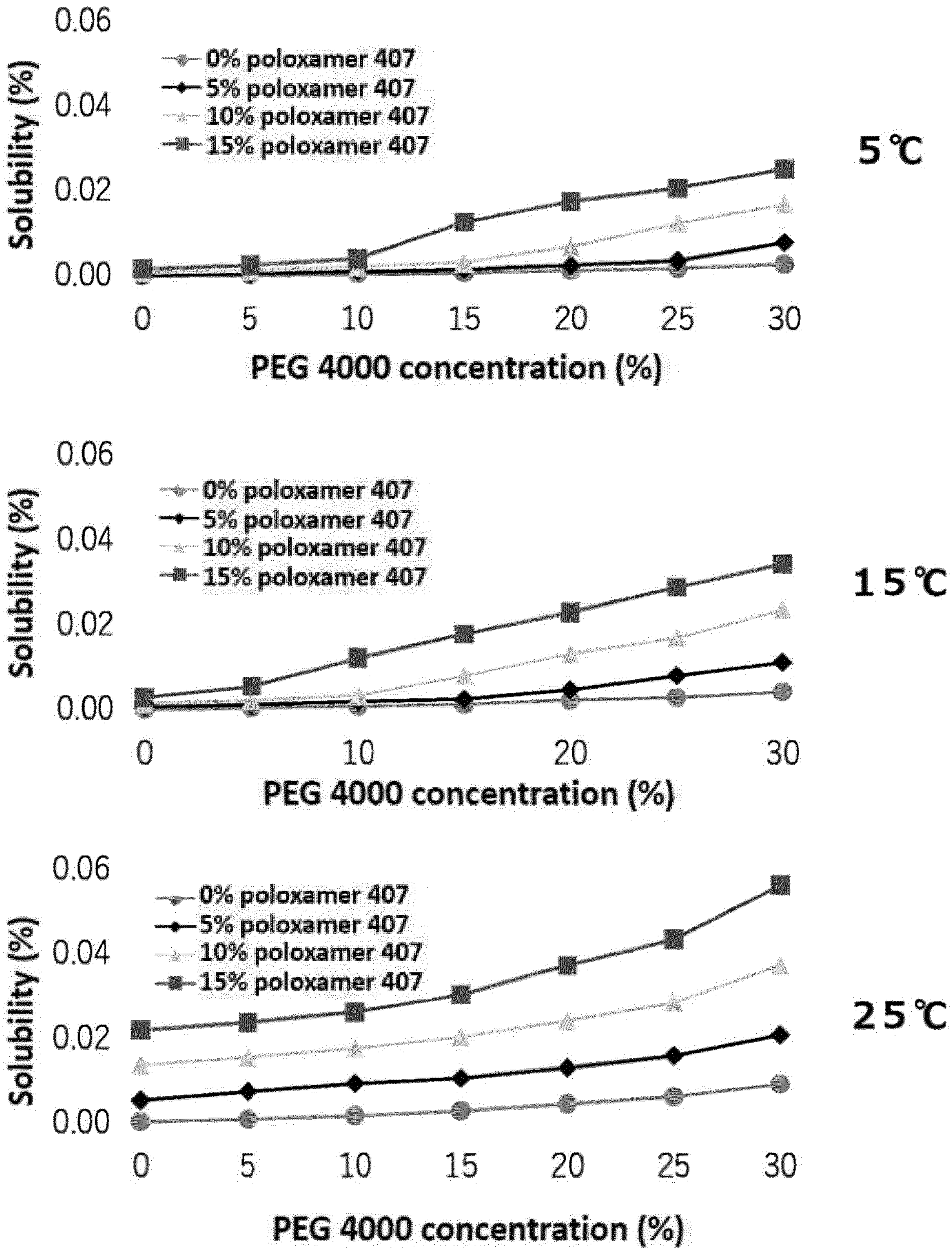

Osthole

Fig 17

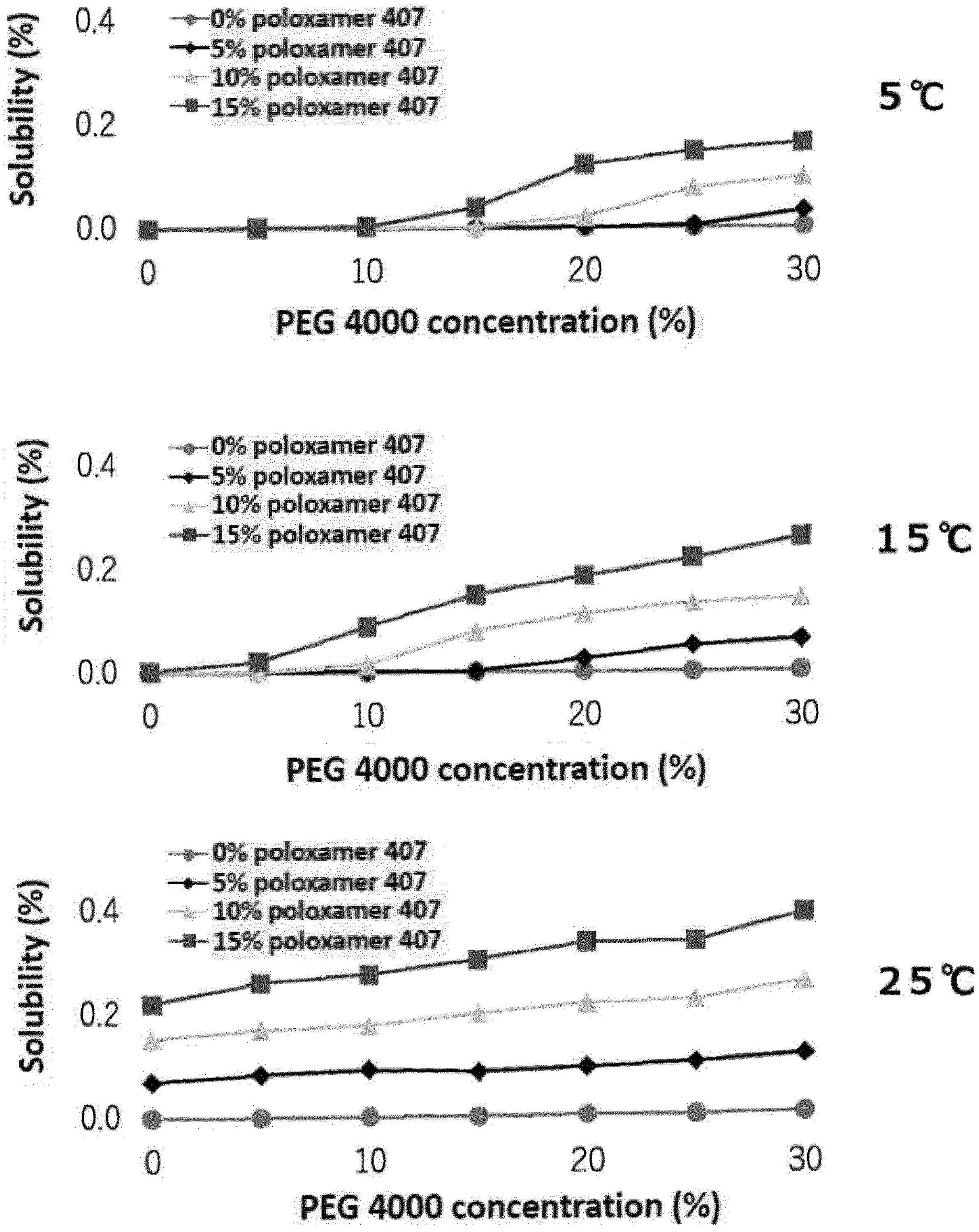

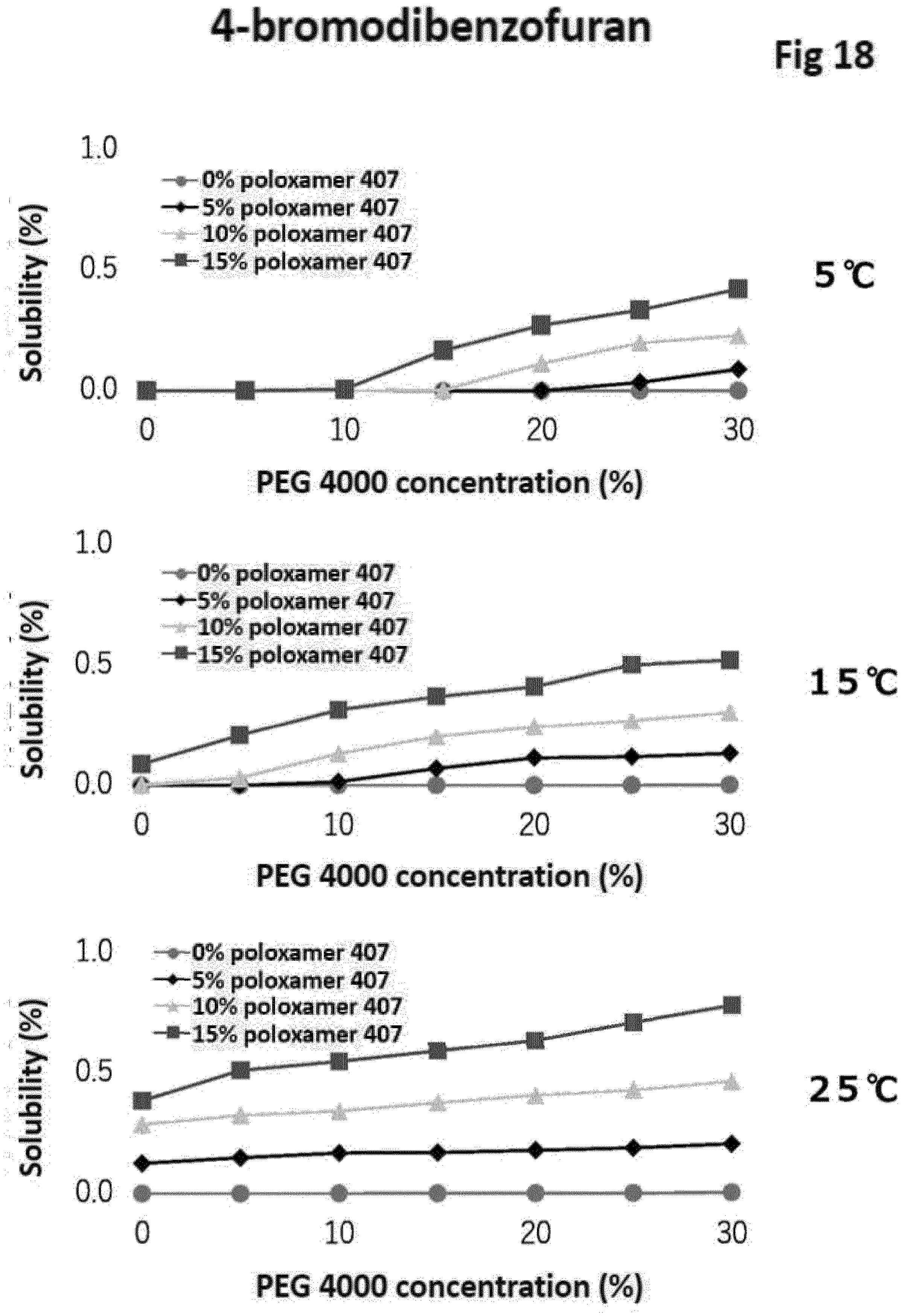
4-bromodibenzofuran
Fig 18
1.0
0.5
0.0
0% poloxamer 407
5% poloxamer 407
10% poloxamer 407
15% poloxamer 407
Solubility (%)
5℃
0
10
20
30
PEG 4000 concentration (%)
1.0
0.5
0.0
0% poloxamer 407
5% poloxamer 407
10% poloxamer 407
15% poloxamer 407
Solubility (%)
15℃
0
10
20
30
PEG 4000 concentration (%)
1.0
0.5
0.0
0% poloxamer 407
5% poloxamer 407
10% poloxamer 407
15% poloxamer 407
Solubility (%)
25℃
0
10
20
30
PEG 4000 concentration (%)

Simvastatin

Fig 19

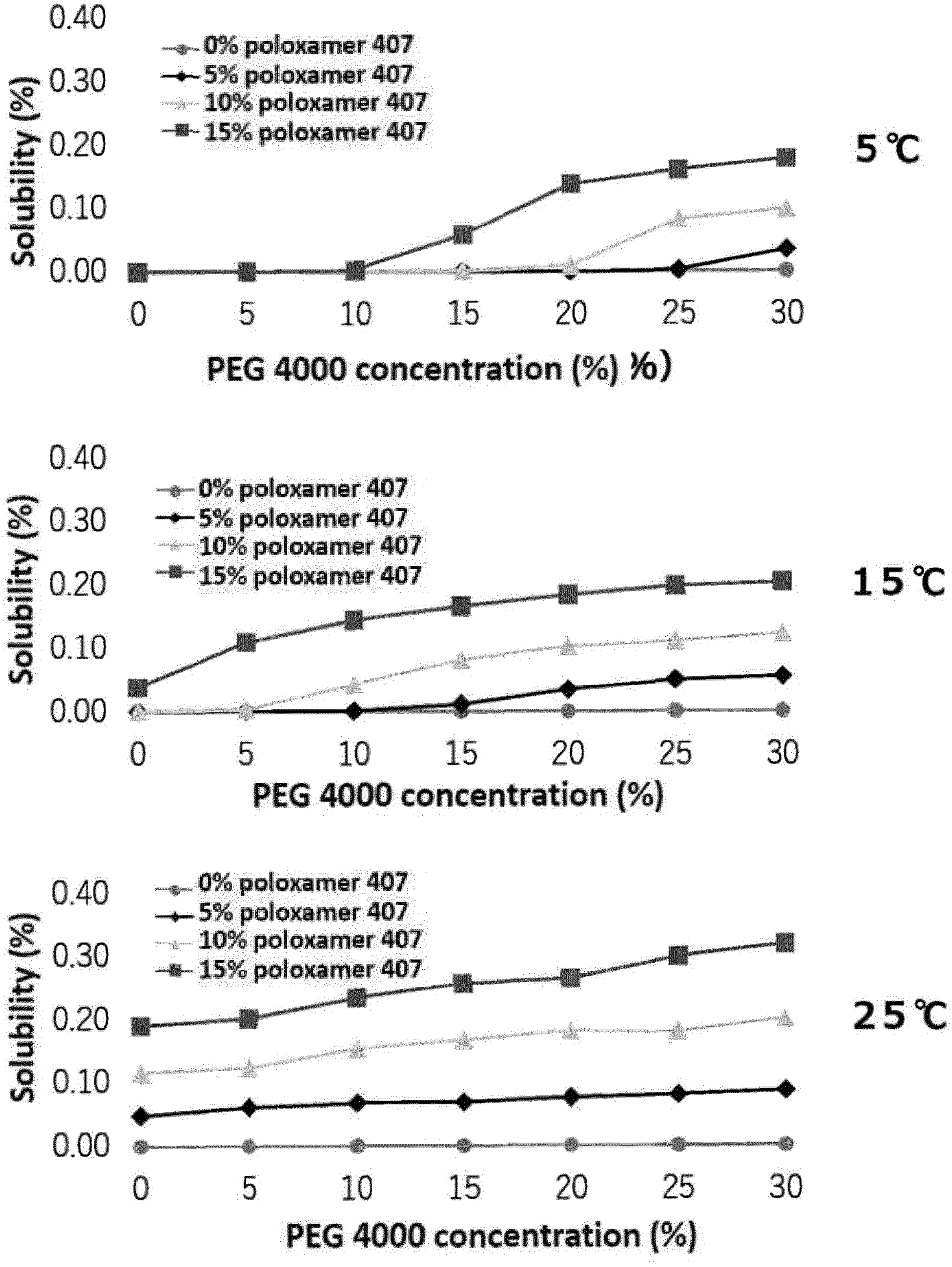

Efavirenz

Fig 20

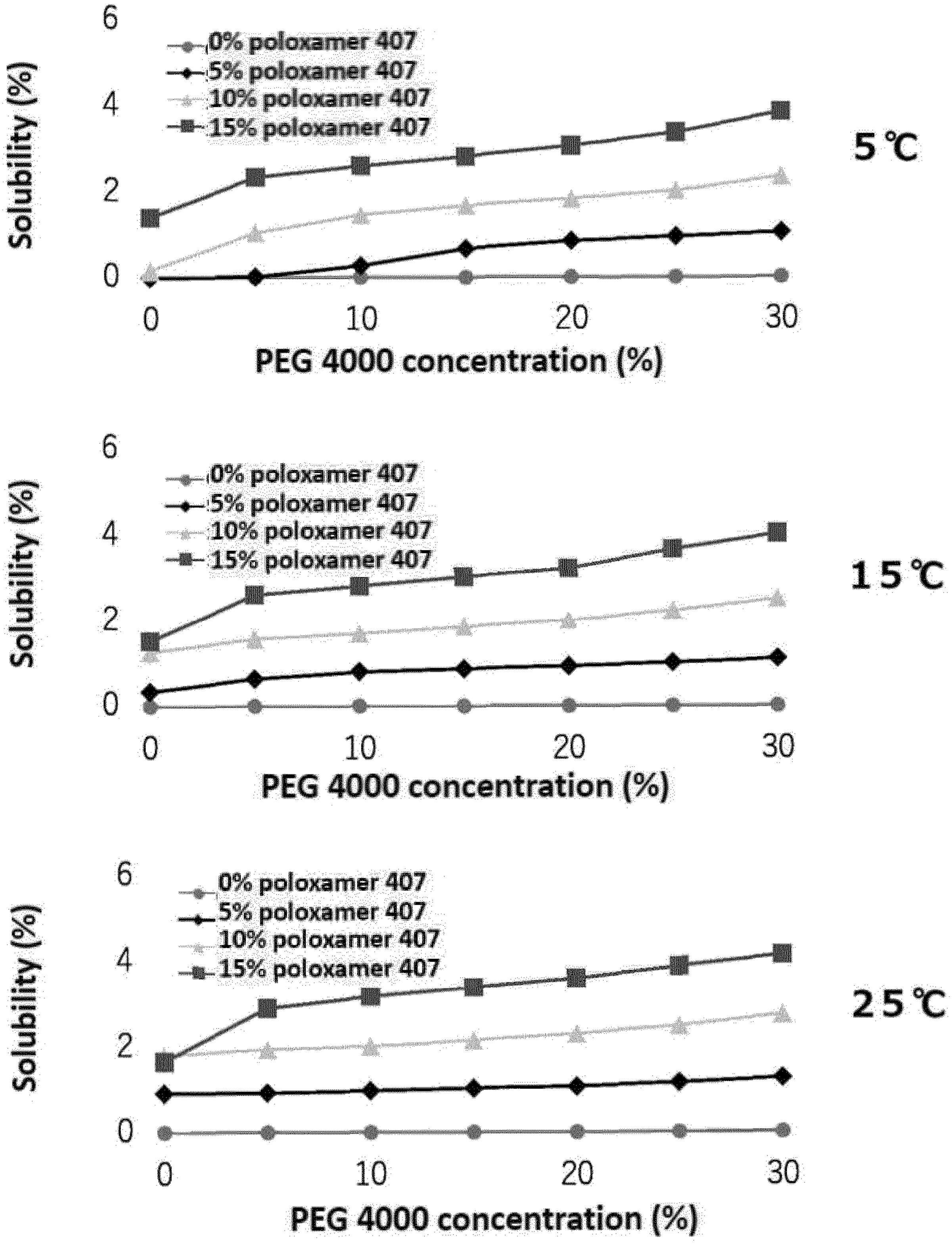

Rebamipide

Fig 21

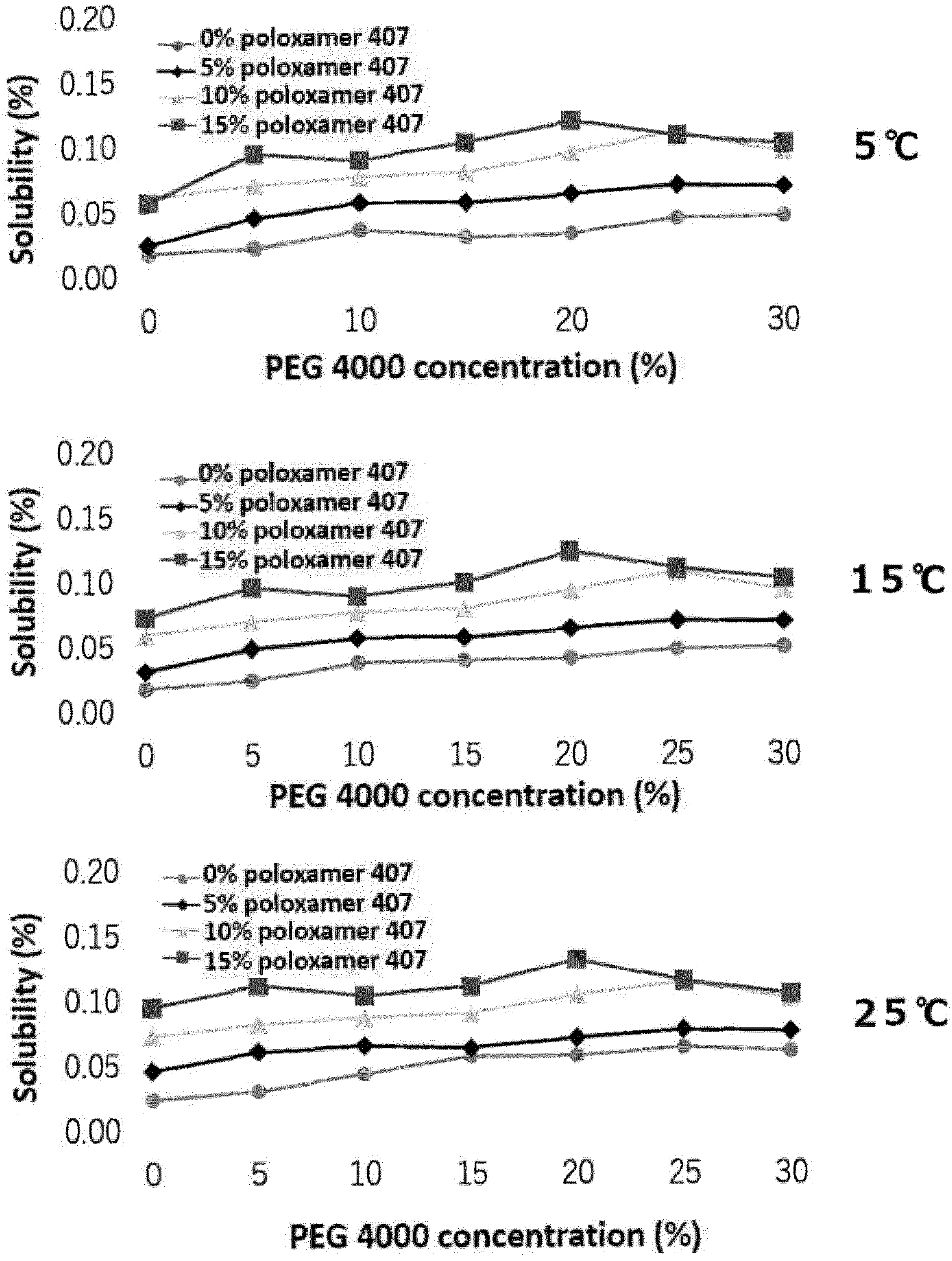

Celecoxib

Fig 22

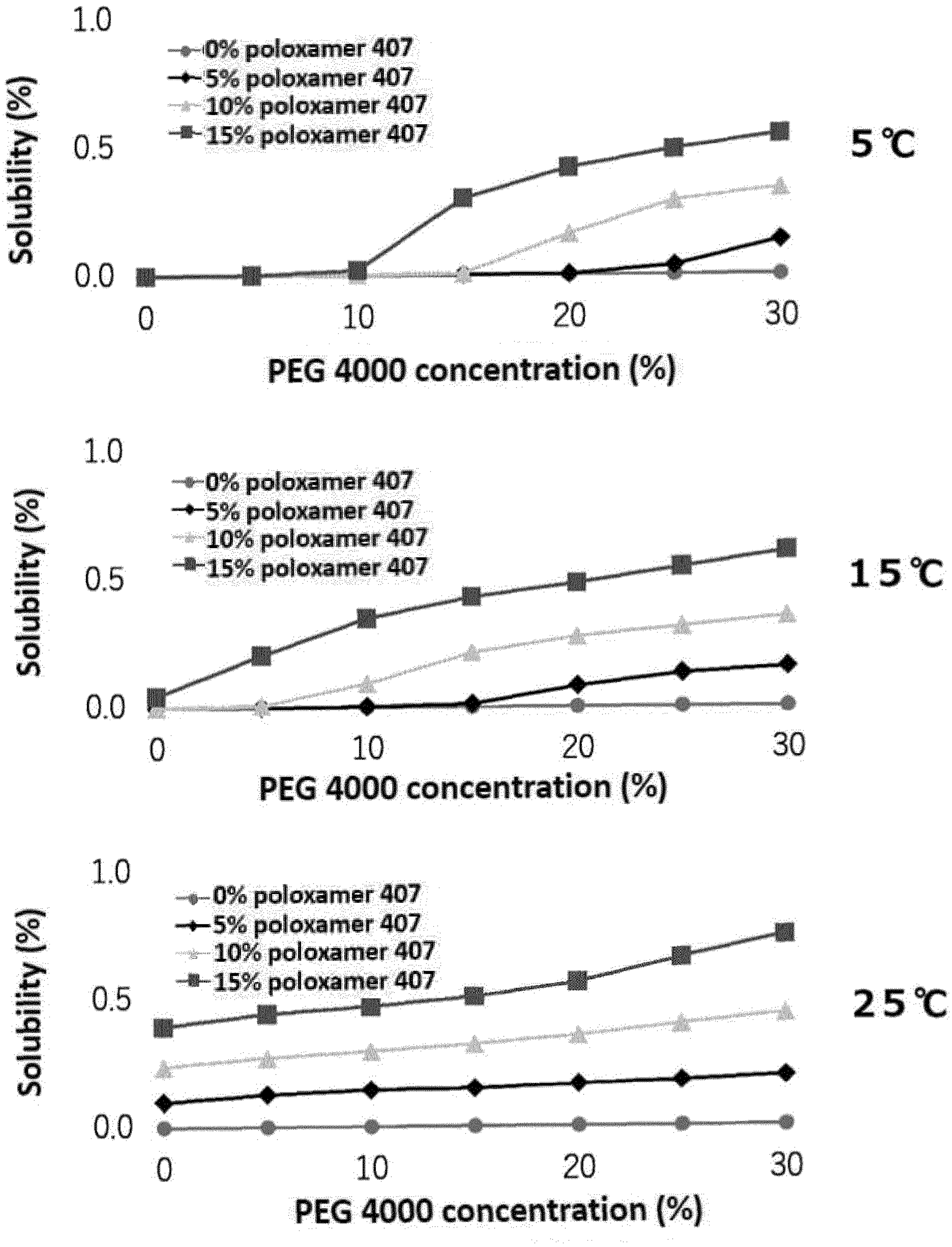

Celecoxib + 0.5% CMC

Fig 23

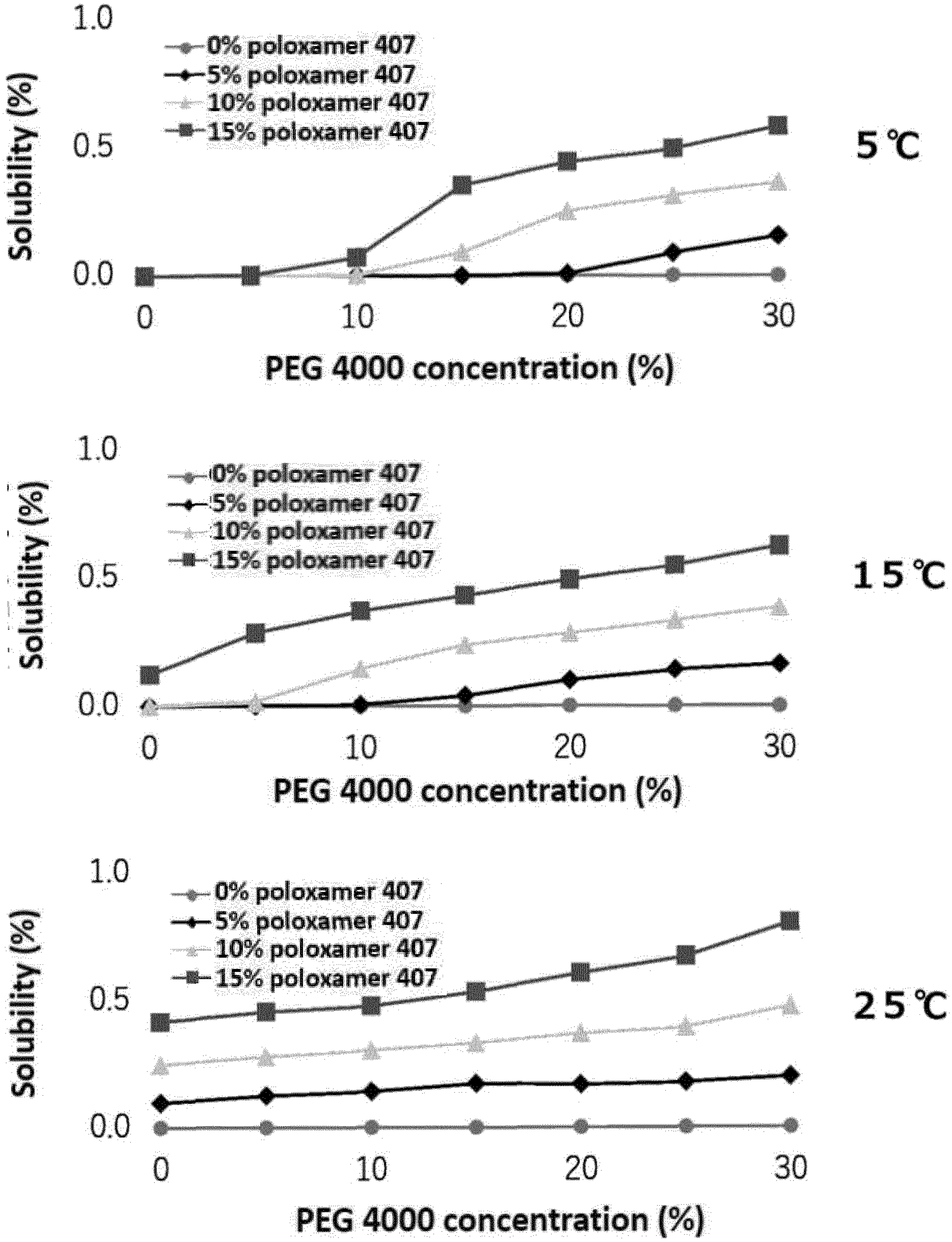

Celecoxib + 5% SBE-β-CD

Fig 24

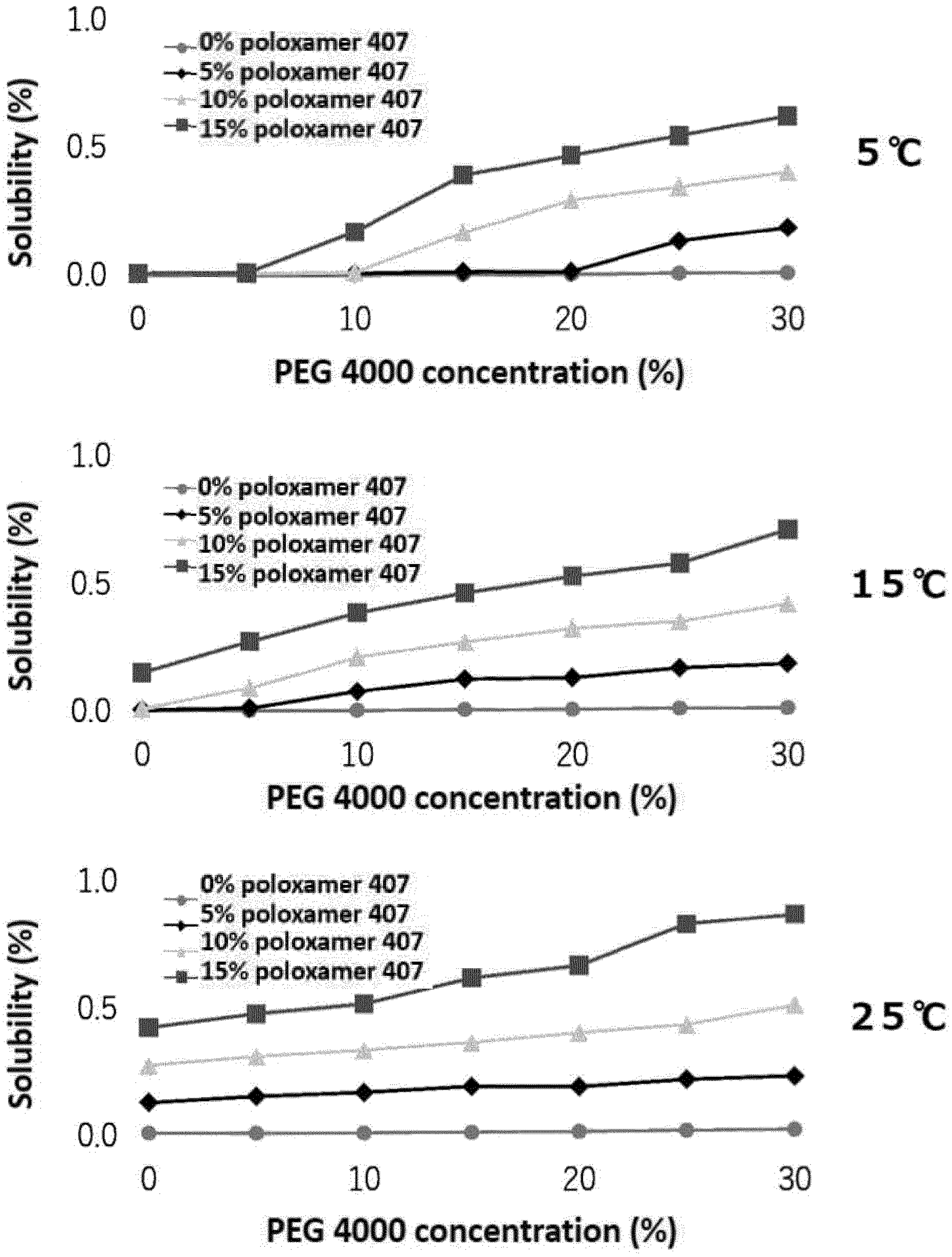

Celecoxib + 5% HP-β-CD

Fig 25

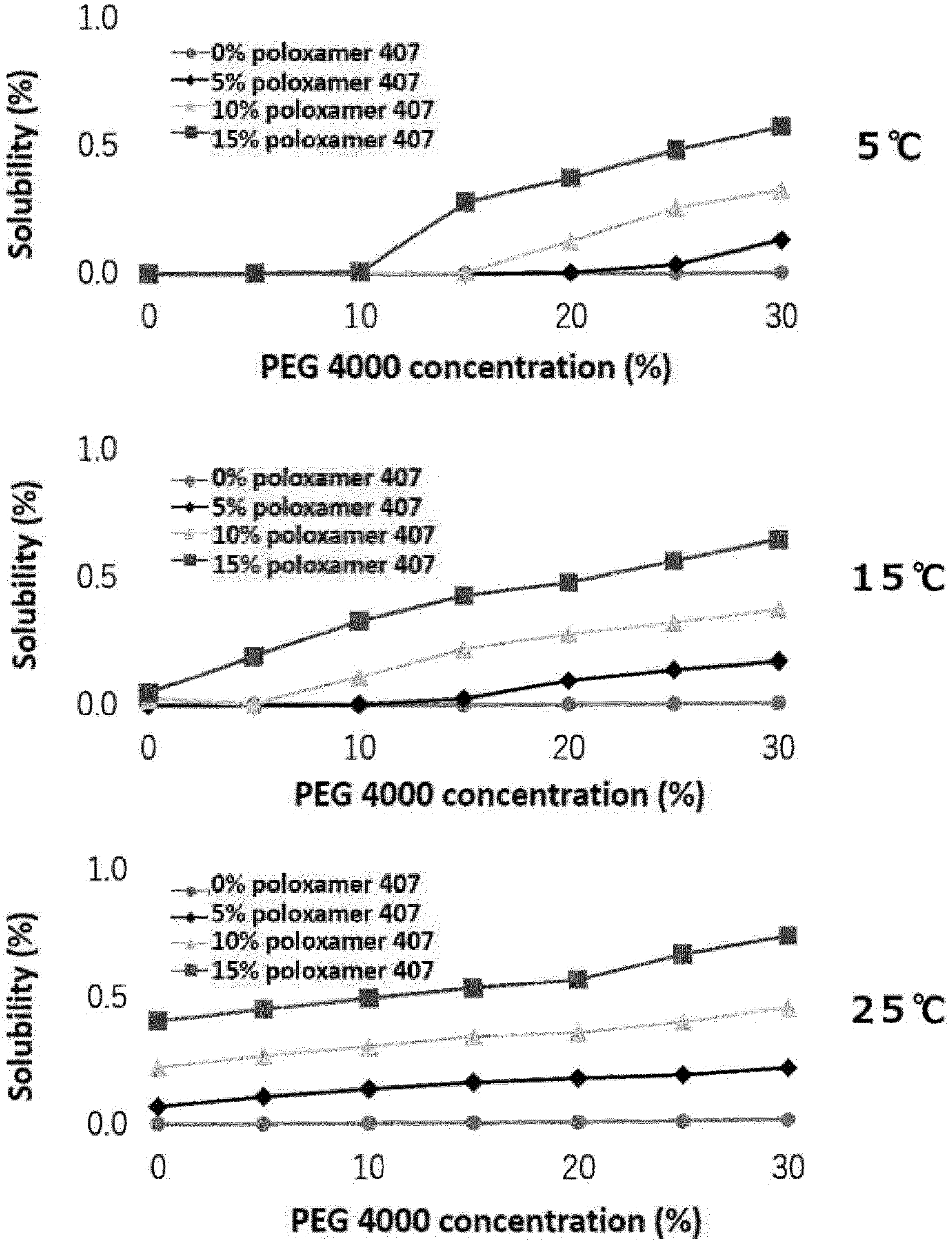

## INTERNATIONAL SEARCH REPORT

International application No. PCT/JP2021/003724

A. CLASSIFICATION OF SUBJECT MATTER

A61K 47/10(2006.01)i; A61K 9/08(2006.01)i; A61K 47/04(2006.01)i; A61K 47/12(2006.01)i; A61K 47/18(2006.01)i; A61K 47/38(2006.01)i; A61K 47/40(2006.01)i; A61K 47/46(2006.01)i

FI: A61K47/10; A61K9/08; A61K47/46; A61K47/04; A61K47/18; A61K47/12; A61K47/40; A61K47/38

According to International Patent Classification (IPC) or to both national classification and IPC

B. FIELDS SEARCHED

Minimum documentation searched (classification system followed by classification symbols)

A61K47/10; A61K9/08; A61K47/04; A61K47/12; A61K47/18; A61K47/38; A61K47/40; A61K47/46

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP 2012-140451 A (CENTOCOR ORTHO BIOTECH INC.) 26 July 2012 (2012-07-26) claims, paragraphs [0039], [0073], [0109], examples | 1-4, 6-25, 27-30 |
| X | CN 1634408 A (ZHANG, Fan) 06 July 2005 (2005-07-06) claims, examples | 1-5, 7-10, 27-29, 31-33 |
| X | CN 102885768 A (JIANGSU JIBEIER PHARMACEUTICAL CO., LTD.) 23 January 2013 (2013-01-23) claims, paragraph [0012], examples | 1-2, 4-10, 27-29, 31-33 |
| X | JP 2008-534586 A (TEVA PHARMACEUTICAL INDUSTRIES LTD.) 28 August 2008 (2008-08-28) claims, paragraph [0022], examples | 1-4, 6-25, 27-29 |

☒ Further documents are listed in the continuation of Box C. ☒ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| 18 March 2021 (18.03.2021) | 30 March 2021 (30.03.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No. PCT/JP2021/003724

C (Continuation). DOCUMENTS CONSIDERED TO BE RELEVANT

| Category* | Citation of document, with indication, where appropriate, of the relevant passages | Relevant to claim No. |
|---|---|---|
| X | JP 2008-513416 A (NYCOMED GMBH) 01 May 2008 (2008-05-01) claims, paragraphs [0013], [0031], examples | 11-24, 27-33 |
| X | JP 2007-504103 A (LIFECYCLE PHARMA A/S) 01 March 2007 (2007-03-01) claims, paragraphs [0061], [0118], examples | 11-29 |
| X | CHAUDHARI, P. et al., "Solubility enhancement of hydrophobic drugs using synergistically interacting cyclodextrins and cosolvent", CURRENT SCIENCE, 2007, vol. 92, no. 11, pp. 1586-1591 abstract, fig. 1 | 11-25, 27-33 |
| Y | abstract, fig. 1 | 26-33 |
| Y | JP 2004-526730 A (CYDEX, INC.) 02 September 2004 (2004-09-02) paragraph [0023] | 26-33 |

Form PCT/ISA/210 (continuation of second sheet) (January 2015)

## INTERNATIONAL SEARCH REPORT

International application No. PCT/JP2021/003724

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
See extra sheet

1. ☐ As all required additional search fees were timely paid by the applicant, this international search report covers all searchable claims.
2. ☒ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

**INTERNATIONAL SEARCH REPORT**
Information on patent family members

International application No.
PCT/JP2021/003724

| Patent Documents referred in the Report | Publication Date | Patent Family | Publication Date |
|---|---|---|---|
| JP 2012-140451 A | 26 Jul. 2012 | US 2004/0265388 A1 claims, paragraphs [0058], [0094], [0131], examples<br>US 2005/0027019 A1<br>US 2004/0220283 A1<br>US 2009/0093549 A1<br>WO 2004/010941 A2<br>WO 2004/039360 A1<br>WO 2005/072343 A2<br>EP 1708719 A2<br>EP 1539122 A2<br>EP 1556022 A1<br>KR 10-2005-0026539 A<br>CN 1671367 A | |
| CN 1634408 A | 06 Jul. 2005 | (Family: none) | |
| CN 102885768 A | 23 Jan. 2013 | (Family: none) | |
| JP 2008-534586 A | 28 Aug. 2008 | US 2006/0222707 A1 claims, paragraph [0024], examples<br>WO 2006/107411 A2<br>EP 1861084 A1<br>KR 10-2007-0119700 A<br>CN 101217951 A | |
| JP 2008-513416 A | 01 May 2008 | US 2007/0259009 A1 claims, paragraphs [0018], [0034], examples<br>WO 2006/032675 A1 | |
| JP 2007-504103 A | 01 Mar. 2007 | US 2010/0008984 A1 claims, paragraphs [0058], [0129], examples<br>WO 2005/020994 A1<br>EP 1663216 A1<br>CN 101869561 A | |
| JP 2004-526730 A | 02 Sep. 2004 | US 2003/0055023 A1 paragraphs [0015]-[0016]<br>WO 2002/074200 A1<br>EP 1383445 A1<br>KR 10-2004-0097181 A | |

Form PCT/ISA/210 (patent family annex) (January 2015)

INTERNATIONAL SEARCH REPORT

International application No. PCT/JP2021/003724

<Continuation of Box No. III>

(Invention 1) Claims 1-10, and claims 27-33 (parts thereof)

Document 1 indicates that propofol, which is a poorly soluble compound, is dissolved in a solution containing Poloxamer 188, PEG-400, and citric acid (paragraph [0109]). Like the present application, in document 1, poloxamer and polyethylene glycol coexist with a chelate compound, and are thus considered to be stabilized by the chelate compound. Accordingly, claims 1-4 lack novelty in light of document 1, and do not have a special technical feature.

However, claim 5, which is dependent on claim 1, has the special technical feature of a "composition which is for stabilizing a polyether-based compound and contains thiosulfate".

Therefore, claims 1-5, and claims 6-10 and 27-33 referring to any of claims 1-5 are classified as invention 1.

(Invention 2) Claims 11-26, and claims 27-33 (parts thereof)

Claims 11-26 cannot be said to share an identical or corresponding technical feature with claim 5 classified as invention 1.

Moreover, claims 11-26 are not substantially identical or equivalent to any of the claims classified as invention 1.

Therefore, claims 11-26 cannot be classified as invention 1.

In addition, claims 11-26 have the special technical feature of a "composition which is for improving the solubility of a poorly soluble compound by a combination of a hydrophobic polyoxy aliphatic moiety-containing polyether-based compound and a hydrated compound, and contains at least one among the hydrophobic polyoxy aliphatic moiety-containing polyether-based compound and the hydrated compound", and are thus classified as invention 2, together with claims 27-33 referring to the invention in claims 11-26.

Document 1: JP 2012-140451 A (CENTOCOR ORTHO BIOTECH INC.) 26 July 2012 (2012-07-26) claims, paragraphs [0039], [0073], [0109], examples & US 2004/0265388 A1 claims, paragraphs [0058], [0094], [0131], examples

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 9510712 W
- JP 2020016656 A


### Non-patent literature cited in the description


- *Colloids and Surfaces A: Physicochemical and Engineering Aspects,* 10 March 1995, vol. 96 (1-2), 1-46